# EUROPEAN PATENT SPECIFICATION

(11) **EP 2 983 647 B1**
(45) Date of publication and mention of the grant of the patent: **09.09.2020**
(21) Application number: 14723122.9
(22) Date of filing: 11.04.2014
(51) Int. Cl.: A61K 9/16, A61K 31/439, A61K 31/395, A61P 1/00

(54) **NSAID ADMINISTRATION AND RELATED COMPOSITIONS, METHODS AND SYSTEMS**
NSAID-VERABREICHUNG SOWIE ZUSAMMENSETZUNGEN, VERFAHREN UND SYSTEME IN ZUSAMMENHANG DAMIT
ADMINISTRATION D'AINS, COMPOSITIONS, PROCÉDÉS ET SYSTÈMES ASSOCIÉS

(30) Priority: 12.04.2013 US 201361811619 P; 11.07.2013 US 201361845240 P
(43) Date of publication of application: 17.02.2016
(73) Proprietor: Alfasigma S.p.A., 40133 Bologna (IT)
(72) Inventor: VISCOMI, Giuseppe, Claudio, I-40133 Bologna (BO) (IT); GRIMALDI, Maria, I-40133 Bologna (BO) (IT); FOGLI, Maria Vittoria, I-40133 Bologna (BO) (IT); MAFFEI, Paola, I-40133 Bologna (BO) (IT); RENZULLI, Cecilia, I-40133 Bologna (BO) (IT); SFORZINI, Annalisa, I-40133 Bologna (BO) (IT); BLANDIZZI, Corrado, I-56126 Pisa (IT); SCARPIGNATO, Carmelo, I-43126 Parma (IT)
(74) Representative: Hiebl, Inge Elisabeth
(86) International application number: PCT/IB2014/060640
(87) International publication number: WO 2014/167533

(56) References cited:
- WO-A1-2009/108814
- WO-A1-2012/038898
- WO-A2-2006/094737
- ZWOLINSKA-WCISLO MALGORZATA M ET AL: "Beneficial Effects of Probiotic Saccharomyces boulardi and Rifaximine in Stress- and NSAID-Induced Exacerbation of Experimental Colitis", GASTROENTEROLOGY, vol. 142, no. 5, Suppl. 1, May 2012 (2012-05), page S46, XP009178452, & DIGESTIVE DISEASE WEEK (DDW); SAN DIEGO, CA, USA; MAY 19 -22, 2012
- BENONI G ET AL: "Indomethacin - induced intestinal lesions and fecal flora", ADVANCES IN INFLAMMATION RESEARCH,, vol. 6, 1 January 1984 (1984-01-01), pages 103-108, XP009178493,
- LANAS ANGEL ET AL: "Microbial flora in NSAID-induced intestinal damage: A role for antibiotics?", DIGESTION, vol. 73, no. Suppl. 1, 8 February 2006 (2006-02-08), pages 136-150, XP009178466, ISSN: 0012-2823 [retrieved on 2006-02-08]
- SCARPIGNATO C ET AL: "Rifaximin, a Poorly Absorbed Antibiotic: Pharmacology and Clinical Potential", CHEMOTHERAPY, S. KARGER AG, CH, vol. 51, no. suppl. 1, 1 January 2005 (2005-01-01), pages 36-66, XP009107325, ISSN: 0009-3157, DOI: 10.1159/000081990
- CARLA J. GARGALLO ET AL: "Is NSAIDs-related gastrointestinal damage preventable?", JOURNAL OF DIGESTIVE DISEASES, vol. 14, no. 2, 24 February 2013 (2013-02-24), pages 55-61, XP055122750, ISSN: 1751-2972, DOI: 10.1111/1751-2980.12019
- L Ciobanu ET AL: "European Review for Medical and Pharmacological Sciences 344 Effects of rifaximin on indomethacin-induced intestinal damage in guinea-pigs", , 1 February 2014 (2014-02-01), XP055122760, Retrieved from the Internet: URL:http://www.europeanreview.org/wp/wp-co ntent/uploads/344-351.pdf [retrieved on 2014-06-11]
- M Colosimob ET AL: "SYNTHESIS OF H LABELED RIFAMYCIN L 105 a C", Journal of Labelled compounds and Radiophamaceuticats -Vot. X X, 1 November 1983 (1983-11-01), XP055174605, Retrieved from the Internet: URL:http://onlinelibrary.wiley.com/store/1 0.1002/jlcr.2580201110/asset/2580201110_ft p.pdf?v=1&t=i6xpswoo&s=1d5945b5815ef7c8a27 bbf136f02229a2712805c [retrieved on 2015-03-06]
- JOHN L WALLACE ET AL: "Proton Pump Inhibitors Exacerbate NSAID-Induced Small Intestinal Injury by Inducing Dysbiosis", GASTROENTEROLOGY : OFFICIAL PUBLICATION OF THE AMERICAN GASTROENTEROLOGICAL ASSOCIATION, WILLIAMS & WILKINS, US, vol. 141, no. 4, 29 June 2011 (2011-06-29) , pages 1314-1322.e5, XP028305248, ISSN: 0016-5085, DOI: 10.1053/J.GASTRO.2011.06.075 [retrieved on 2011-07-13]

## Description

### Field of the invention

This disclosure relates to nonsteroidal anti-inflammatory drugs (NSAIDs) administration.The present disclosure relates to treatment and/or prevention of adverse effects associated with NSAIDs administration.

### Background

NSAIDs are usually indicated for the treatment of acute or chronic conditions wherein pain and inflammation are present. Research continues into their potential for prevention of several diseases also related to cancer, especially colorectal cancer, Alzheimer Dementia, and treatment of other conditions, such as cardiovascular disease.

However, despite the beneficial effects associated with administration of NSAIDs, minimization of adverse drug events in particular at the level of gastrointestinal tract is still challenging.

### Summary

Provided herein are compositions for use which in several embodiments can be used to reduce and possibly minimize adverse effects, such as adverse gastrointestinal effects, associated with NSAIDs administration and in particular, long term administration of NSAID, in an individual.

The invention relates to rifaximin in combination with at least one proton pump inhibitor and optionally a further antibiotic for use in a method for treating or preventing enteropathy in an individual undergoing NSAID administration, wherein the NSAID is diclofenac, ketoprofen, naproxen, ibuprofen, acetylsalicylic acid or mixtures thereof and wherein rifaximin is in gastroresistant form or in form of gastroresistant microgranules and is orally administered in an amount of from 20 mg to 3300 mg per day.

The compositions for use herein described, are used in connection with applications wherein minimization of adverse events and in particular adverse events of the gastrointestinal tract, preferably of the intestinal tract, associated with NSAID administration to the individual is desired.

Suitable exemplary applications comprise medical and in particular clinical applications wherein relief of all grades of pain and/or inflammation in a wide range of conditions is desired, and in particular with reference to conditions such as (i) arthritic conditions: rheumatoid arthritis, osteoarthritis, ankylosing spondylitis, acute gout, (ii) acute musculo-skeletal disorders such as periarthritis, tendinitis, tenosynovitis, bursitis, (iii) other painful conditions resulting from trauma, including fracture, low back pain, sprains, strains, dislocations, orthopedic, dental and other minor surgery neuropathic pain (e.g., diabetic neuropathic pain, trigeminal neuralgia, transverse myelitis, and sciatica) and chronic myofascial pain, (iv) and/or cardiovascular disease. Further conditions where pain and/or inflammation are involved which are treated with NSAIDs comprise various cardiovascular diseases as will be understood by a skilled person.

Additional suitable medical applications will be identifiable by a skilled person with particular reference to applications in which a long-term NSAID therapy of at least one week is desired. The compositions for use according to the invention are useful in treatment and/or prevention of severe enteropathy and/or enteropathy associated with long-term NSAID treatment of at least ten days in the individual.

The details of one or more embodiments of the present disclosure are set forth in the description below. Other features, objects, and advantages will be apparent from the description and from the claims.

### Brief description of drawings

The accompanying drawings, which are incorporated into and constitute a part of this specification, illustrate one or more embodiments of the present disclosure and, together with the description of example embodiments, serve to explain the principles and implementations of the disclosure.

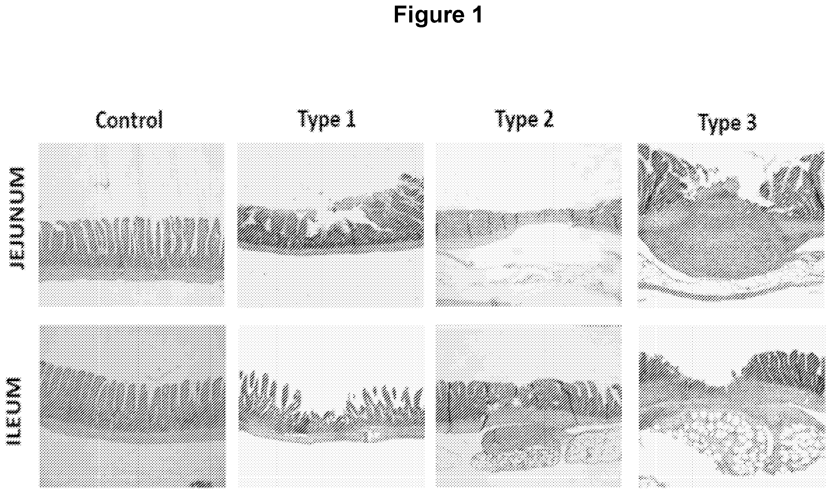

FIG. 1 shows exemplary pictures showing the appearance of Type 1, 2 and 3 lesions in jejunum and ileum of rats treated with indomethacin 1.5 mg/kg BID for 14 days.

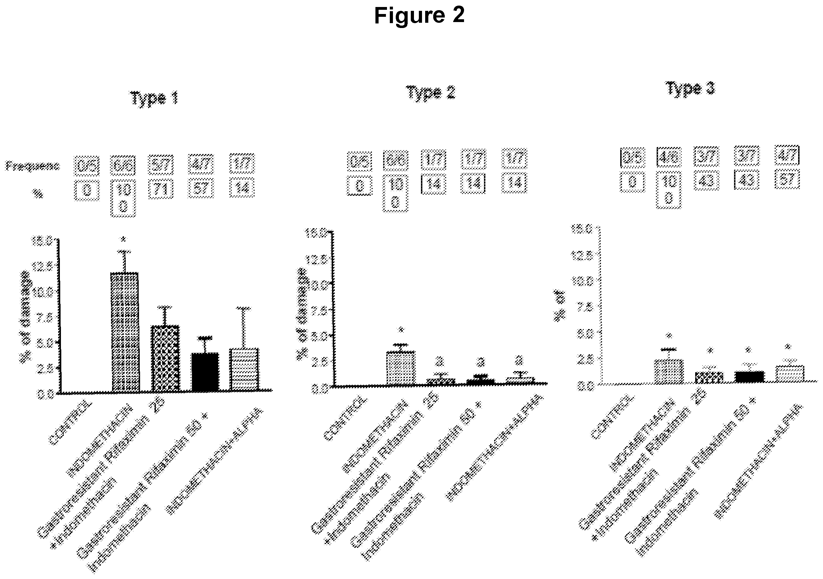

FIG. 2 shows diagrams illustrating the results of microscopic assessment of intestinal damage in samples of jejunum obtained from rats treated with vehicle (control), or indomethacin 1.5 mg/kg BID, either alone or in combination with gastroresistant rifaximin 25 mg/kg BID, 50 mg/kg BID or rifaximin polymorph alpha 50 mg/kg BID for 14 days. Numbers above each column indicate the rate of lesioned rats detected in each group of treatment (frequency) and the respective percentage. Each column represents the mean of 5 to 7 animals ± S.E.M. *P<0.05, significant difference vs. control; a P<0.05, significant difference vs. indomethacin.

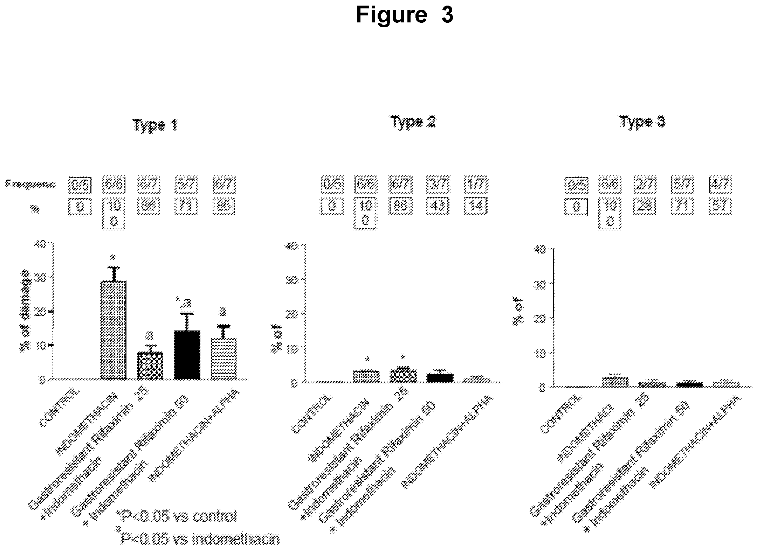

FIG. 3 shows diagrams illustrating the results of microscopical assessment of intestinal damage in samples of ileum obtained from rats treated with vehicle (control), or indomethacin 1.5 mg/kg BID, either alone or in combination with gastroresistant rifaximin 25 mg/kg BID, 50 mg/kg BID or rifaximin polymorph alpha 50 mg/kg BID for 14 days. Numbers above each column indicate the rate of lesioned rats detected in each group of treatment (frequency) and the respective percentage. Each column represents the mean of 5 to 7 animals ± S.E.M. *P<0.05, significant difference vs. control; ^{a}P<0.05, significant difference vs. indomethacin.

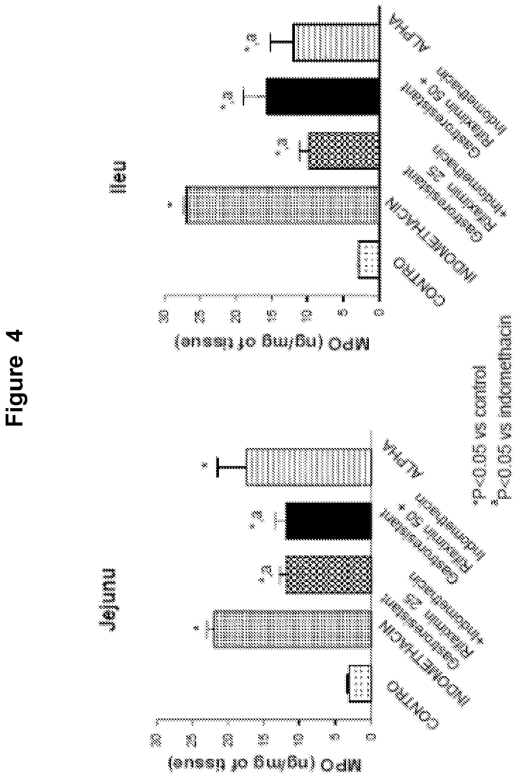

FIG. 4 shows diagrams illustrating myeloperoxidase levels in samples of jejunum and ileum obtained from rats treated with vehicle (control), or indomethacin 1.5 mg/kg BID, either alone or in combination with gastroresistant rifaximin 25 mg/kg BID, gastroresistant rifaximin 50 mg/kg BID or alpha 50 mg/kg BID for 14 days. Each column represents the mean of 5 to 7 animals ± S.E.M. *P<0.05, significant difference vs. control; ^{a}P<0.05, significant difference vs. indomethacin.

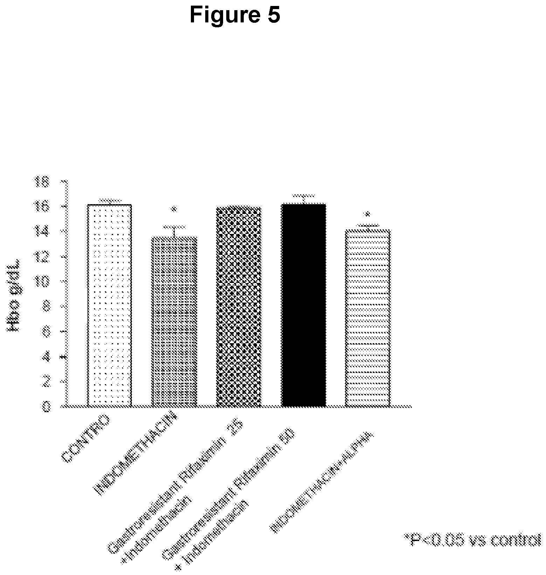

FIG. 5 shows a diagram illustrating the results of blood hemoglobin levels in rats treated with vehicle (control), or indomethacin 1.5 mg/kg BID, either alone or in combination with gastroresistant rifaximin 25 mg/kg BID, 50 mg/kg BID or rifaximin polymorph alpha 50 mg/kg BID for 14 days. Each column represents the mean of 5 to 7 animals ± S.E.M. *P<0.05, significant difference vs. control.

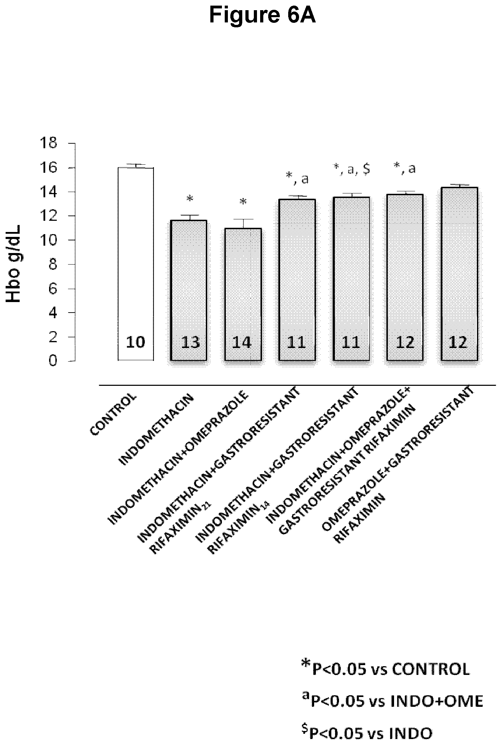

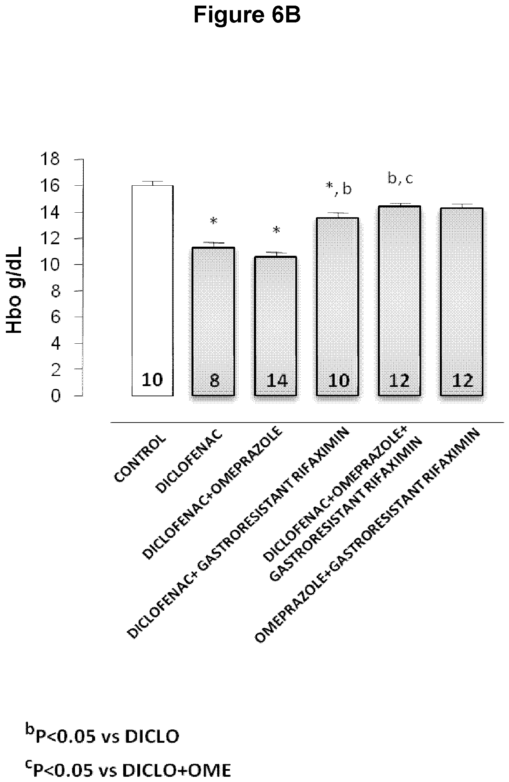

FIG. 6A shows a diagram illustrating the results of blood hemoglobin levels in rats treated with vehicle (control), or indomethacin 1.5 mg/kg BID or indomethacin 1.5 mg/kg BID plus omeprazole 0.7 mg/kg/day or gastroresistant rifaximin (50 mg/kg BID) for 1 week followed by indomethacin 1.5 mg/kg BID plus gastroresistant rifaximin (50 mg/kg BID), or indomethacin plus gastroresistant rifaximin (1.5 mg/kg BID+50 mg/kg BID), or indomethacin plus omeprazole plus gastroresistant rifaximin (1.5 mg/kg BID + 0.7 mg/kg/day+ 50 mg/kg BID). FIG 6B shows a diagram illustrating the results of blood hemoglobin levels in rats with vehicle (control), or diclofenac (4 mg/kg BID), or diclofenac plus omeprazole (4 mg/kg BID+0.7 mg/kg/day), diclofenac plus gastroresistant rifaximin (4 mg/kg BID+50 mg/kg BID), or diclofenac plus omeprazole plus gastroresistant rifaximin (4 mg/kg BID+0.7 mg/kg+ 50 mg/kg BID) or omeprazole plus gastroresistant rifaximin (0.7 mg/kg daily +50 mg/kg BID) for 14 days. Each column represents the mean ± S.E.M. obtained from 8-14 animals. The numbers that appear in each column indicate to the number of animals examined at the end of the treatment period.

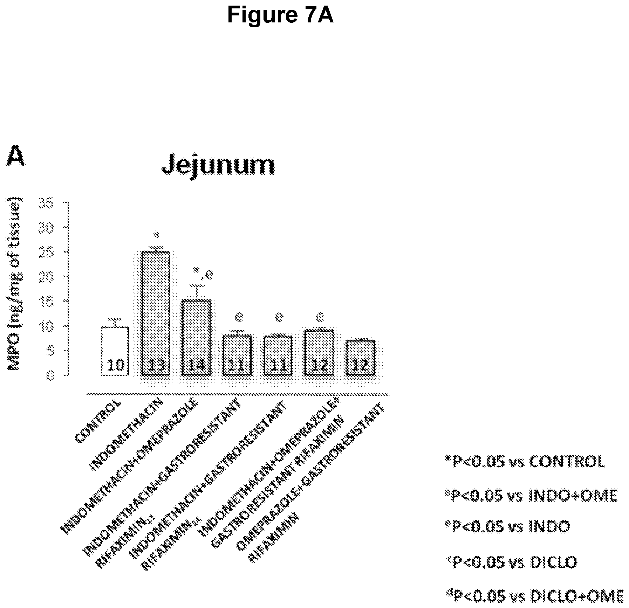

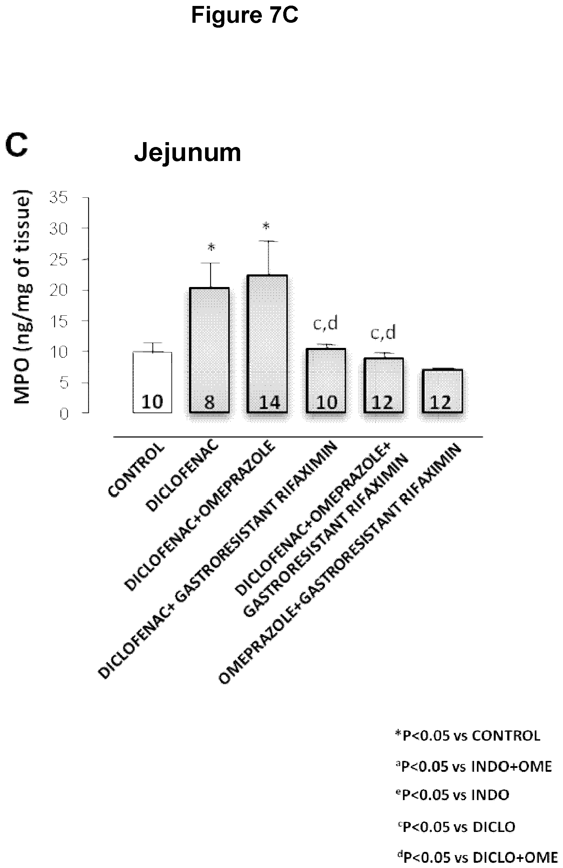

FIG. 7A and FIG 7C show diagrams illustrating the results of intestinal myeloperoxidase (MPO) levels in jejunum in rats treated with vehicle (control), or indomethacin 1.5 mg/kg BID or indomethacin 1.5 mg/kg BID plus omeprazole 0.7 mg/kg/day or gastroresistant rifaximin (50 mg/kg BID) for 1 week followed by indomethacin 1.5 mg/kg BID plus gastroresistant rifaximin (50 mg/kg BID), or indomethacin plus gastroresistant rifaximin (1.5 mg/kg BID+50 mg/kg BID), or indomethacin plus omeprazole plus gastroresistant rifaximin (1.5 mg/kg BID + 0.7 mg/kg/day+ 50 mg/kg BID), or diclofenac (4 mg/kg BID), or diclofenac plus omeprazole (4 mg/kg BID+ 0.7 mg/kg/day), diclofenac plus gastroresistant rifaximin (4 mg/kg BID+50 mg/kg BID), or diclofenac plus omeprazole plus gastroresistant rifaximin (4 mg/kg BID+0.7 mg/kg+ 50 mg/kg BID) or omeprazole plus gastroresistant rifaximin (0.7 mg/kg daily +50 mg/kg BID) for 14 days.

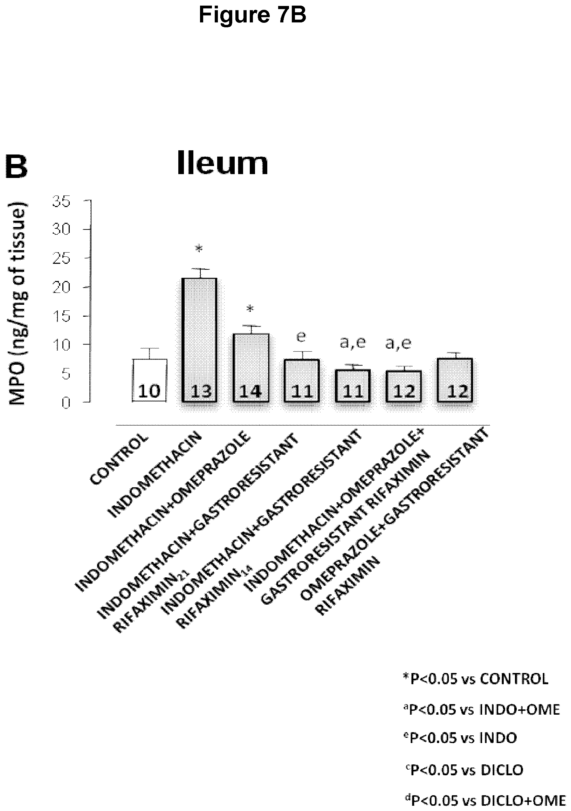

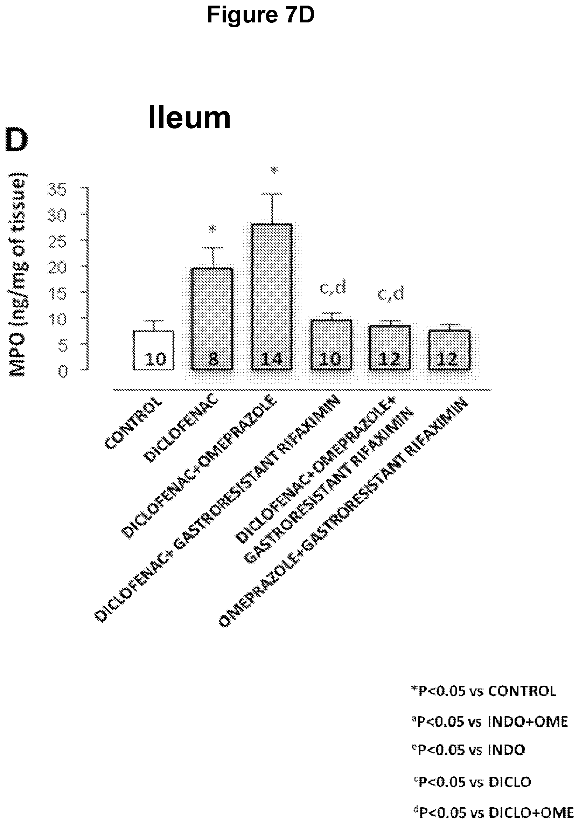

FIG. 7B and FIG 7 D show diagrams illustrating the results of intestinal myeloperoxidase (MPO) levels in or ileum (B, D) in the same treated rats. Each column represents the mean ± S.E.M. obtained from 8-14 animals. The numbers that appear in each column indicate to the number of animals examined at the end of the treatment period.

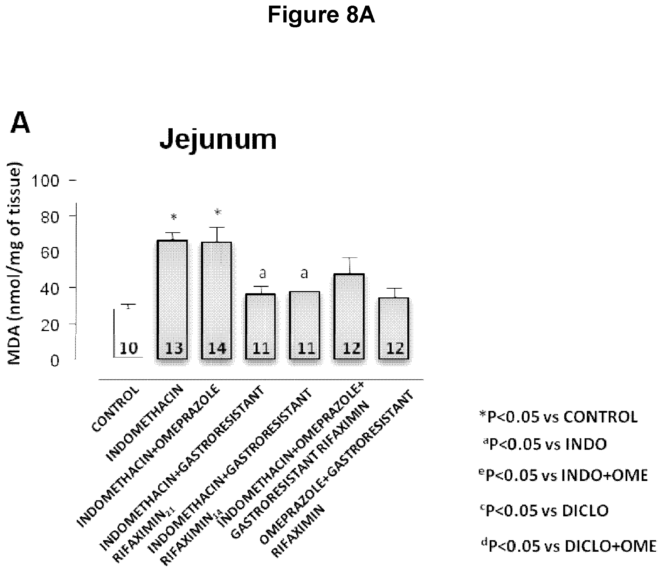

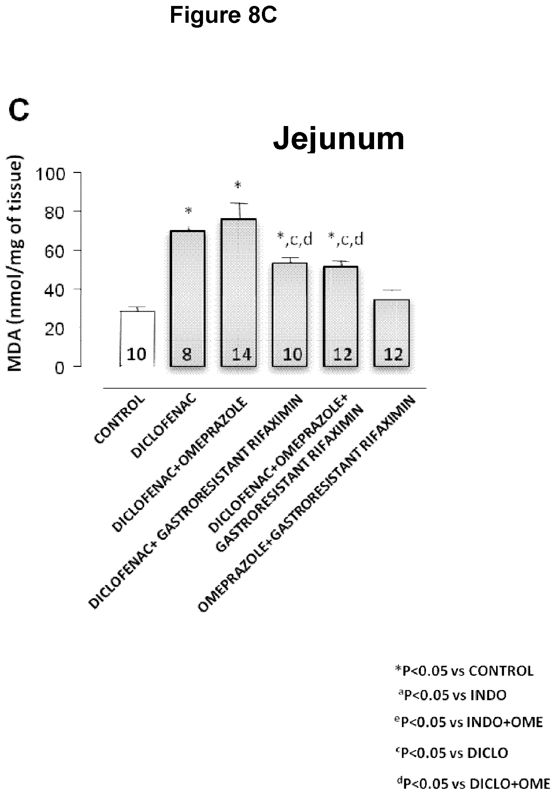

FIG. 8A and FIG 8C show diagrams illustrating the results of Malondialdehyde (MDA) levels in the jejunum of rats treated with vehicle (control), or indomethacin 1.5 mg/kg BID or indomethacin 1.5 mg/kg BID plus omeprazole 0.7 mg/kg/day or gastroresistant rifaximin (50 mg/kg BID) for 1 week followed by indomethacin 1.5 mg/kg BID plus gastroresistant rifaximin (50 mg/kg BID), or indomethacin plus gastroresistant rifaximin (1.5 mg/kg BID+50 mg/kg BID), or indomethacin plus omeprazole plus gastroresistant rifaximin (1.5 mg/kg BID + 0.7 mg/kg/day+ 50 mg/kg BID), or diclofenac (4 mg/kg BID), or diclofenac plus omeprazole (4 mg/kg BID+ 0.7 mg/kg/day), diclofenac plus gastroresistant rifaximin (4 mg/kg BID+50 mg/kg BID), or diclofenac plus omeprazole plus gastroresistant rifaximin (4 mg/kg BID+0.7 mg/kg+ 50 mg/kg BID) or omeprazole plus gastroresistant rifaximin (0.7 mg/kg daily +50 mg/kg BID) for 14 days.

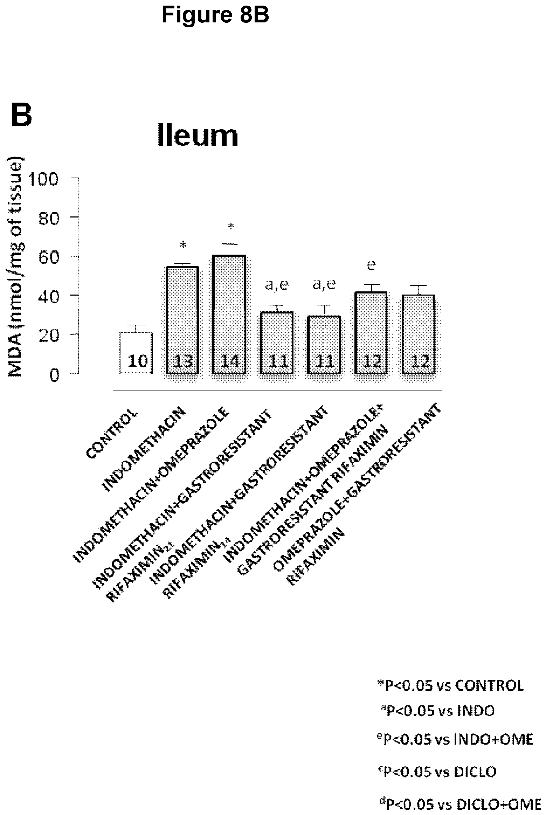

FIG. 8B and FIG 7 D show diagrams illustrating the results of intestinal myeloperoxidase (MPO) levels in or ileum (B, D) in the same treated rats. Each column represents the mean ± S.E.M. obtained from 8-14 animals. The numbers that appear in each column indicate to the number of animals examined at the end of the treatment period.

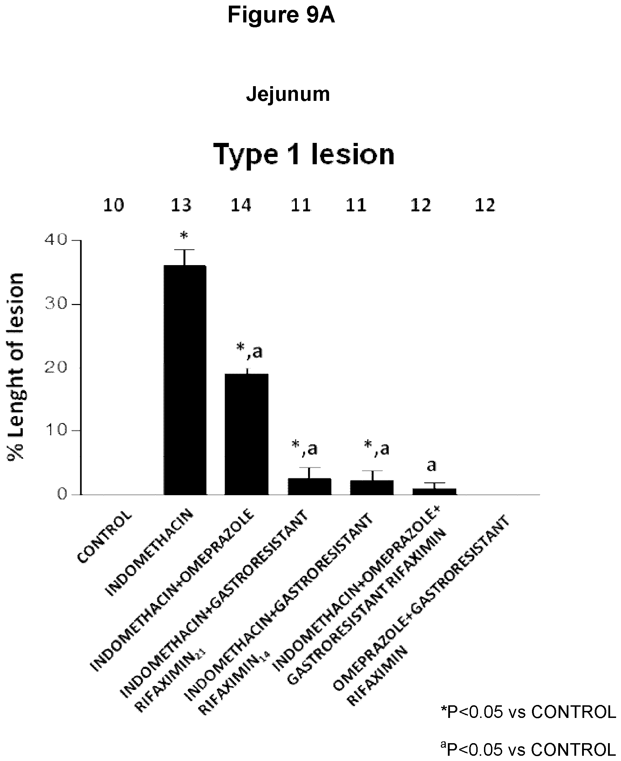

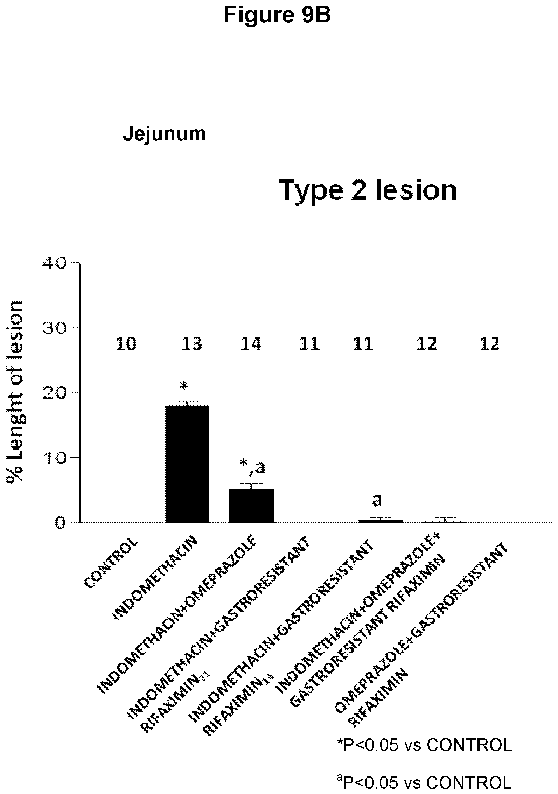

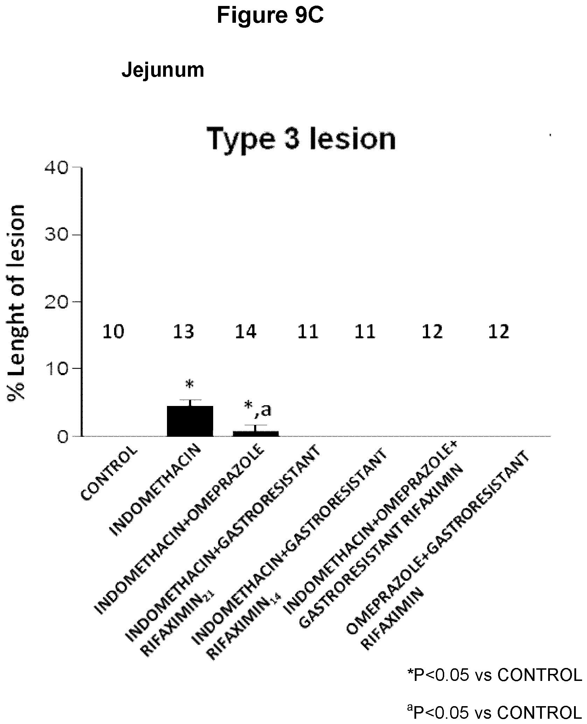

FIG. 9A, FIG.9B and FIG 9C show diagrams illustrating the results of the microscopic analysis of damage in the jejunum of rats treated with vehicle (control), or indomethacin 1.5 mg/kg BID or indomethacin 1.5 mg/kg BID plus omeprazole 0.7 mg/kg/day or gastroresistant rifaximin (50 mg/kg BID) for 1 week followed by indomethacin 1.5 mg/kg BID plus gastroresistant rifaximin (50 mg/kg BID), or indomethacin plus gastroresistant rifaximin (1.5 mg/kg BID+50 mg/kg BID), or indomethacin plus omeprazole plus gastroresistant rifaximin (1.5 mg/kg BID + 0.7 mg/kg/day+ 50 mg/kg BID). Figure 9A refers to Type 1 lesion, Figure 9 B refers to Type 2 lesion and Figure 9 C refers to Type 3 lesion. Each column represents the mean ± S.E.M. obtained from 10-14 animals. The numbers that appear on top of each column indicate to the number of animals examined at the end of the treatment period.

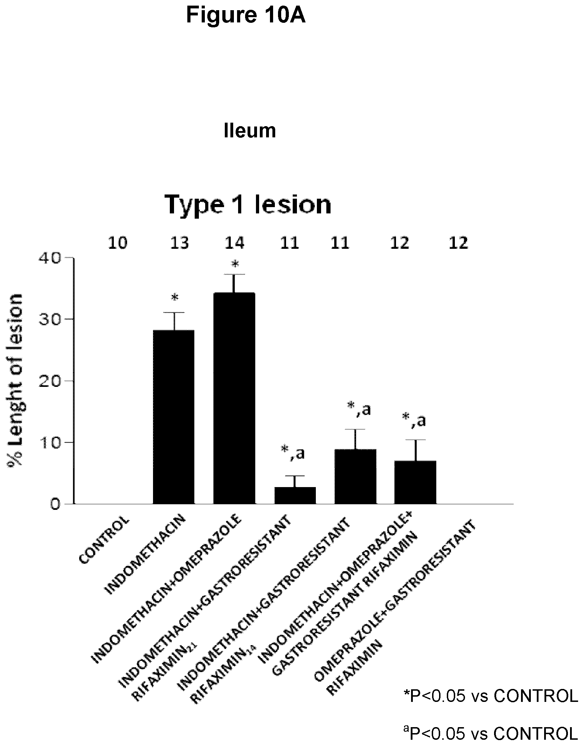

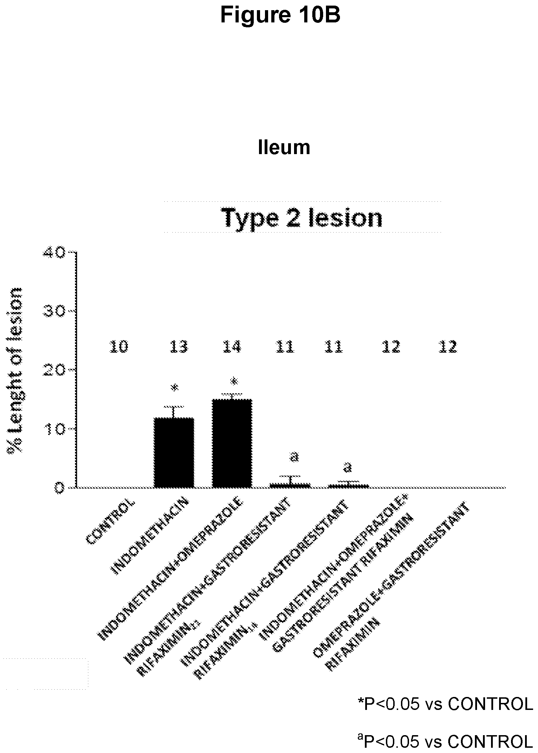

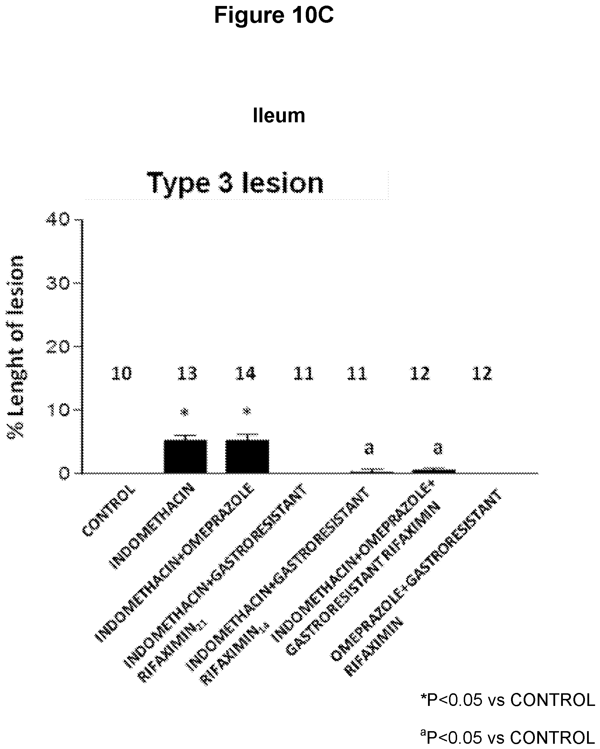

FIG. 10A, FIG.10B and FIG. 10C show diagrams illustrating the results of the microscopic analysis of damage in the ileum of rats treated with vehicle (control), or indomethacin 1.5 mg/kg BID or indomethacin 1.5 mg/kg BID plus omeprazole 0.7 mg/kg/day or gastroresistant rifaximin (50 mg/kg BID) for 1 week followed by indomethacin 1.5 mg/kg BID plus gastroresistant rifaximin (50 mg/kg BID), or indomethacin plus gastroresistant rifaximin (1.5 mg/kg BID+50 mg/kg BID), or indomethacin plus omeprazole plus gastroresistant rifaximin (1.5 mg/kg BID + 0.7 mg/kg/day+ 50 mg/kg BID). Figure 10A refers to Type 1 lesion, Figure 10B refers to Type 2 lesion and Figure 10C refers to Type 3 lesion. Each column represents the mean ± S.E.M. obtained from 10-14 animals. The numbers that appear on top of each column indicate to the number of animals examined at the end of the treatment period.

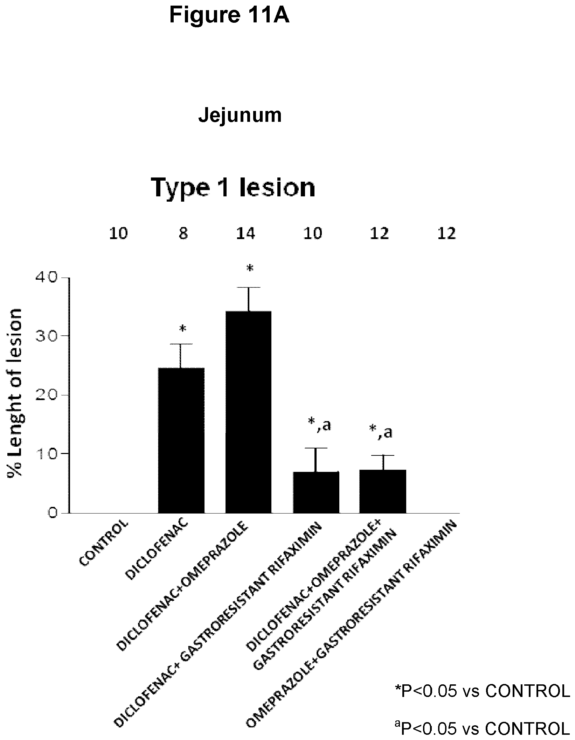

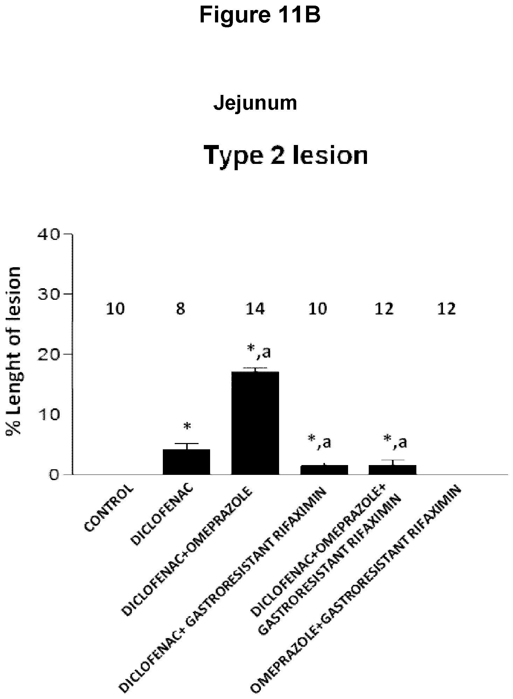

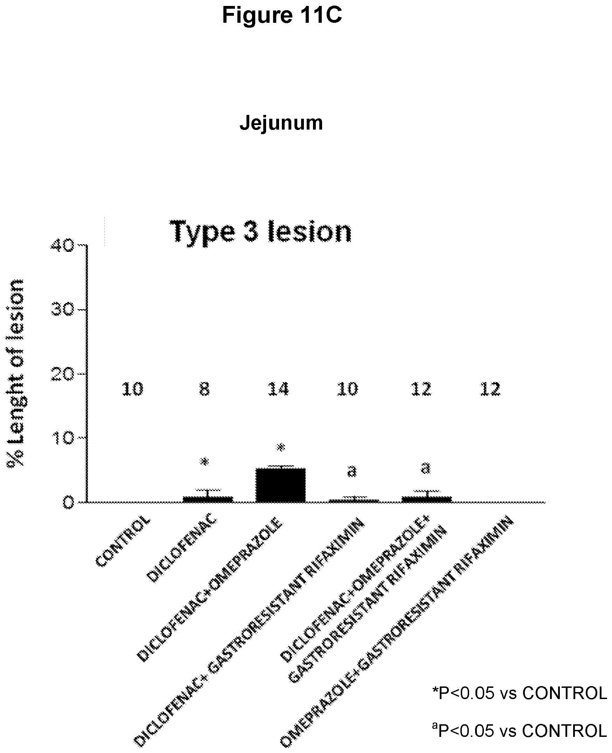

FIG. 11A, FIG. 11B and FIG 11C show diagrams illustrating the results of the microscopic analysis of damage in the jejunum of rats treated with vehicle (Control), or diclofenac (4 mg/kg BID), or diclofenac plus omeprazole (4 mg/kg BID+ 0.7 mg/kg/day), diclofenac plus gastroresistant rifaximin (4 mg/kg BID+50 mg/kg BID), or diclofenac plus omeprazole plus gastroresistant rifaximin (4 mg/kg BID+0.7 mg/kg+ 50 mg/kg BID) or omeprazole plus gastroresistant rifaximin (0.7 mg/kg daily +50 mg/kg BID) for 14 days. Figure 11A refers to Type 1 lesion, Figure 11B refers to Type 2 lesion and Figure 11C refers to Type 3 lesion. Each column represents the mean ± S.E.M. obtained from 8-12 animals. The numbers that appear on top of each column indicate to the number of animals examined at the end of the treatment period.

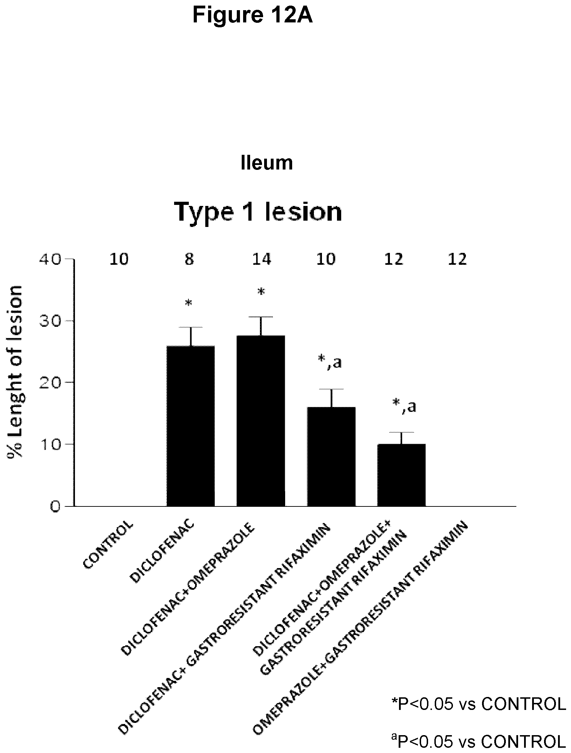

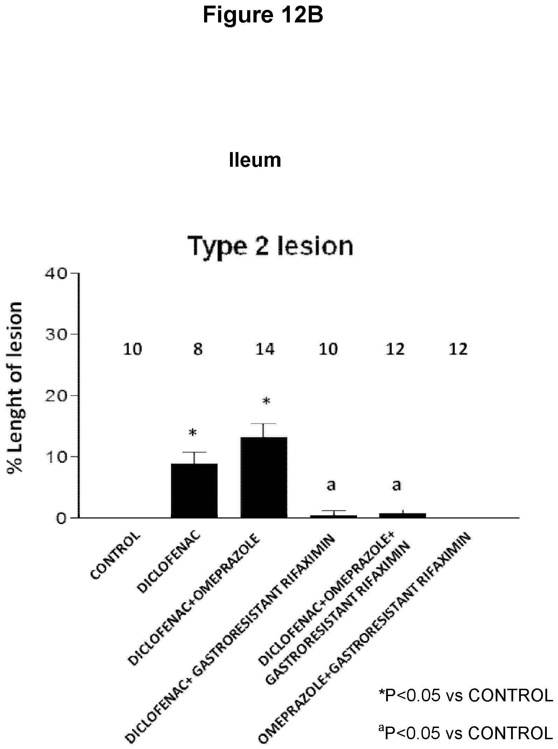

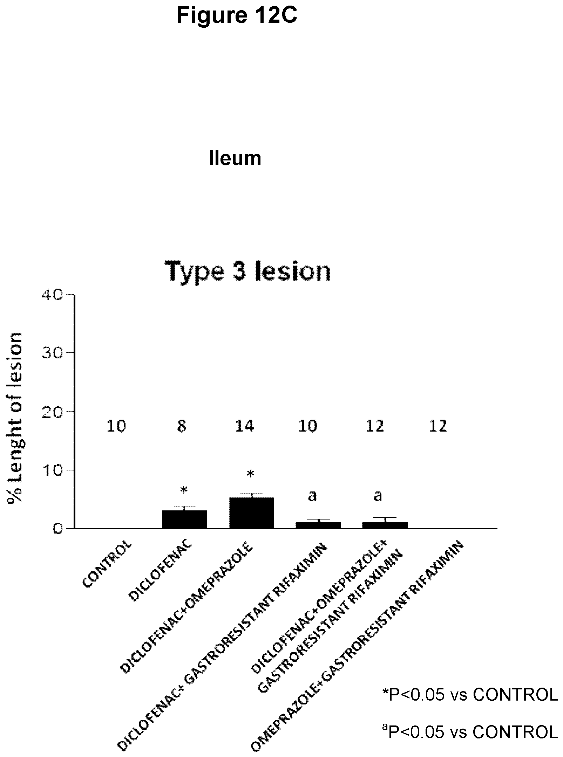

FIG. 12A, FIG.12B and FIG 12C show diagrams illustrating the results of the microscopic analysis of damage in the ileum of rats treated with vehicle (control), or diclofenac (4 mg/kg BID), or diclofenac plus omeprazole (4 mg/kg BID+ 0.7 mg/kg/day), diclofenac plus gastroresistant rifaximin (4 mg/kg BID+50 mg/kg BID), or diclofenac plus omeprazole plus gastroresistant rifaximin (4 mg/kg BID+0.7 mg/kg+ 50 mg/kg BID) or omeprazole plus gastroresistant rifaximin (0.7 mg/kg daily +50 mg/kg BID) for 14 days. Figure 12A refers to Type 1 lesion, Figure 12B refers to Type 2 lesion and Figure 12C refers to Type 3 lesion. Each column represents the mean ± S.E.M. obtained from 8-12 animals. The numbers that appear on top of each column indicate to the number of animals examined at the end of the treatment period.

### Detailed description

The present invention is defined in the claims.

This disclosure relates to compositions for use to treat and/or prevent an adverse effect associated with NSAID administration in an individual.

The term "adverse effect" or "adverse reaction" as used herein with reference to NSAIDs or other drugs indicates an unwanted condition that results from administration of the drug. The term "condition" indicates a physical status of the body of an individual (as a whole or as one or more of its parts e.g., body systems), that does not conform to a standard physical status associated with a state of complete physical, mental and social well-being for the individual. Conditions herein described include but are not limited to disorders and diseases wherein the term "disorder" indicates a condition of the living individual that is associated to a functional abnormality of the body or of any of its parts, and the term "disease" indicates a condition of the living individual that impairs normal functioning of the body or of any of its parts and is typically manifested by distinguishing signs and symptoms in an individual.

The term "individual" or "subject' or "patient" as used herein in the context of treatment includes a single animal and in particular higher animals and in particular vertebrates such as mammals and in particular human beings. In general "individual" according to the present disclosure indicates an animal that has a gastrointestinal (herein also GI) system and that is susceptible to gastric and intestinal ulcerations.

In particular, the compositions for use are described to treat and/or prevent an NSAID enteropathy, wherein NSAID enteropathy indicates an adverse effect of the gastrointestinal system associated with NSAID administration to an individual.

The term "treatment" as used herein indicates any activity that is part of a medical care for, or deals with, a condition, medically or surgically. The terms "treating" and "treatment" refer to reduction in severity and/or frequency of symptoms, elimination of symptoms and/or underlying cause, prevention of the occurrence of symptoms and/or their underlying cause, and improvement or remediation of damage. Thus, for example, "treating" a patient involves prevention of a symptom or adverse physiological event in a susceptible individual, as well as modulation and/or amelioration of the status of a clinically symptomatic individual by inhibiting or causing regression of a disorder or disease.

The term "prevention" as used herein with reference to a condition indicates any activity which reduces the burden of mortality or morbidity from the condition in an individual. This takes place at primary, secondary and tertiary prevention levels, wherein: a) primary prevention avoids the development of a disease; b) secondary prevention activities are aimed at early disease treatment, thereby increasing opportunities for interventions to prevent progression of the disease and emergence of symptoms; and c) tertiary prevention reduces the negative impact of an already established disease by restoring function and reducing disease-related complications.

The term "enteropathy" as used herein relates to a condition of the gastrointestinal system of an individual and can be particularly referred a condition of the intestine. In general, an enteropathy according to the disclosure identifies a disorder or a disease of the GI tract presenting gastrointestinal damage either in the upper and/or lower tract. Exemplary enteropathies comprise bowel lesions, particularly at the small bowel, possibly exacerbated by dysbiosis of the intestine and/or by the use of gastric acid inhibitor, which, when associated with NSAID use, is one of the major contributing factors to the increased susceptibility to small intestine injury induced by NSAID administration. Exemplary enteropathies associated with NSAID administration are conditions described in Allison MC et al. in "Gastrointestinal damage associated with the use of non steroidal anti-inflammatory drugs" N. Engl. J. Med 1992; 327: 749-54; Lengeling et al. in "Ulcerative ileitis encountered at ileo-colonscopy: likely role of nonsteroidal agents" Clin. Gastroenterol. Hepatol. 2003; 1:160-9; and Graham DY et al. in "Visible small-intestinal-mucosal injury in chronic NSAID users" Clin. Gastroenterol. Hepatol. 2005; 3: 5-9, Scarpignato C. et al., in "NSAID-induced intestinal damage: are luminal bacteria the therapeutic target?" Gut February 2008 Vol. 57 No 2, Wallace JL et al., Gastroenterol. 2011, 141; p. 1314 and Thomas J. et al., Clin. Rheumatol. (2006) 25 (Suppl): S22-S29.

Zwolinska - Wcislo M. et al. in Gastroenterology, 142, no. 5, Supp. 1, 2012, S46 describes the use of rifaximin in colitis induced by administration of 2,4,6-trinitrobenzenesulfonic acid (TNBS) in rats, wherein the stress was exacerbated by NSAIN as aspirin. This document refers to the beneficial effect of rifaximin c in the intestinal damage provoked by aspirin, without any mention to the use of rifaximin in combination with anti inflammatory neither to the use of rifaximin in the same time to the anti-inflammatory administration.

Bonomi G. et al., in Advances Inflammation Research 6, 103, 1984 describes the change of fecal flora after antibiotic treatment in the evolution of indomethacine-induced intestinal flora. This document reports that following treatment with antibacterial, in particular clindamycin to protect the intestinal mucousa against indomethacin -induced lesion. This document does not report a preferred antibiotic or a not absorbed antibiotic as rifaximin, as well the dosage and the treatment period and does not give any mention to the use of the gastric acid inhibitor in combination with anti-inflammatory treatment.

Lanas A. et al. in Digestion, 73, 1, 136, 2006 describes the intestinal damage of NSAID and the possible use of poor adsorbed antibiotic, like rifaximin, but it does not give any information if really rifaximin is efficacious in the treatment of NSAID induced damage. This document only suppose of the possibility to use rifaximin and moreover it does not give any information on the effect of the PPI in NSAID concomitant therapy.

Scarpignato et al. in Chemotherapy 51, S1, 26, 2005 gives information that NSAID treatment causes deterioration in ileum and hypnotizes that rifaximin could be useful in the enteropathy, without give any demonstration on the efficacious dosage, the involved disease, the association of the rifaximin and NSDAID with gastric acid inhibitors.

Also Gargallo C et al in Journal Digestive Disease 14, 2, 55, 2013 describes the effect of the use of NSAID including gastrointestinal, cardiovascular, hepatic renal and respiratory system, in particular the intestinal erosions and ulcers provoked by NSAIDs administration.

WO 2006/094737 discloses pharmaceutical formulations containing rifaximin in the shape of microgranules made gastroresistant by an insoluble polymer at pH values between 1.5 and 4.0 and soluble at pH values between 5.0 and 7.

WO 2012/038898 describes the efficacy of tablets containing gastroresistant microganules of rifaximin for the treatment of Crohn's disease.

WO 2009/108814 discloses a methods of treating bowel disease (BD) by the use of rifaximin.

Wallace J.L. et al in Gaastroenterology, vol. n. 141, no. 4, pages 314-1322, 2011 states that PPIs exacerbate NSAID-induced intestinal damage at least in part because of significant shifts in enteric microbial population. The dysbiosis induced by a PPI is a major contributing factor to the increased susceptibility to NSAID-induced small intestinal injury.

The term "NSAID," or "nonsteroidal anti-inflammatory drug" refers to one or more non-steroidal active agents which, when administered to an individual exhibits an analgesic effect, an antipyretic effect, and anti-inflammatory effect or any combinations of the aforementioned effects.

"NSAID administration" refers to treatment of an individual by administering one or more NSAIDs alone or in combination with one another and with an additional active principle wherein the term "administration" refers to routes of introducing a compound or composition into an individual to perform their intended function which comprises systemic administration or locally acting topical administration.

The wording "systemic administration" as used herein indicates a route of administration by which the active principle is brought in contact with the body of the individual so that the desired effect is not necessarily limited to the specific tissue where the inflammation and/or in general the disease (e.g. the cardiovascular disease) occurs. Systemic administration includes enteral and parenteral administration. Enteral administration is a systemic route of administration where the substance is given via the digestive tract, and includes, but is not limited to, oral administration, administration by gastric feeding tube, administration by duodenal feeding tube, gastrostomy, enteral nutrition, and rectal administration. Parenteral administration is a systemic route of administration where the substance is given by route other than the digestive tract and includes but is not limited to intravenous administration, intra-arterial administration, intramuscular administration, subcutaneous administration, intradermal, administration, intraperitoneal administration, and intravesical infusion. The wording "topical administration" as used herein relates to a route of administration wherein the active agent usually included in an appropriate formulation directly where its action is desired. Topical administration includes but is not limited to epicutaneous administration, inhalational administration (e.g., in asthma medications), enema, eye drops (e.g., onto the conjunctiva), ear drops, intranasal route (e.g., decongestant nasal sprays), and vaginal administration, rectal administration and oral administration of non-absorbed agents.

In particular, NSAID administration according to the present disclosure relates to routes of administration in which one or more NSAIDs comes in contact with the gastrointestinal tract of an individual typically after oral administration, wherein a persistent local action is due to the enterohepatic recirculation of the compound, and the systemic effect carried out after its absorption.

In some embodiments, NSAID administration relates to administration of one or more NSAIDs performed to treat and/or prevent a condition of an individual wherein pain and/or inflammations are present, including acute or chronic conditions presenting the above symptoms. The term "pain" as used herein indicates an unpleasant sensory and emotional experience associated with actual or potential tissue damage, or described in terms of such damage. The terms "inflammation" and "inflammatory response" as used herein indicate the complex biological response of tissues, including vascular tissues of an individual to harmful stimuli, such as pathogens, damaged cells, or irritants, and includes secretion of prostaglandins cytokines and more particularly of pro-inflammatory cytokines, i.e., cytokines which are produced predominantly by activated immune cells such as, for example, microglia and are involved in the amplification of inflammatory reactions. Exemplary pro-inflammatory cytokines comprise IL-1, IL-6, TNF-α, IL-17, IL21, IL23, and TGF-β. Exemplary inflammations include acute inflammation and chronic inflammation. The wording "acute inflammation" as used herein indicates a short-term process characterized by the classic signs of inflammation (swelling, redness, pain, heat, and loss of function) due to the infiltration of the tissues by plasma and leukocytes. An acute inflammation typically occurs as long as the injurious stimulus is present and ceases once the stimulus has been removed, broken down, or walled off by scarring (fibrosis). The wording "chronic inflammation" as used herein indicates a condition characterized by concurrent active inflammation, tissue destruction, and attempts at repair. Chronic inflammation is not characterized by the classic signs of acute inflammation listed above. Instead, chronically inflamed tissue is characterized by the infiltration of mononuclear immune cells (monocytes, macrophages, lymphocytes, and plasma cells), tissue destruction, and attempts at healing, which include angiogenesis and fibrosis. An inflammation condition can be controlled according to this disclosure by affecting and in particular inhibiting any one of the events that form the complex biological response associated with an inflammation in an individual. In some of those embodiments, NSAID administration may be performed for a period of time (or duration) from 1 week up to two months or more.

In some embodiments, the condition of an individual wherein pain and/or inflammations are present, is a cardiovascular disease and in particular an acute or chronic cardiovascular disease. The term "cardiovascular disease" as used herein indicates a class of diseases that involve the heart, the blood vessels (arteries, capillaries, and veins) or both. Accordingly, cardiovascular disease refers to any disease that affects the cardiovascular system, principally cardiac disease, vascular diseases of the brain and kidney, and peripheral arterial disease. In particular, inflammation and/or pain are common for heart disease and stroke patients where inflammation in some instance is thought to be a sign or atherogenic response. Exemplary cardiovascular disease treated with NSAID administration comprise coronary artery disease (also known as coronary heart disease and ischaemic heart disease), cardiomyopathy (diseases of cardiac muscle), hypertensive heart disease (diseases of the heart secondary to high blood pressure), heart failure, cardiac dysrhythmias (abnormalities of heart rhythm), inflammatory heart disease such as endocarditis (inflammation of the inner layer of the heart, the endocardium; the structures most commonly involved are the heart valves), inflammatory cardiomegaly, and myocarditis (inflammation of the myocardium, the muscular part of the heart), cerebrovascular disease (disease of blood vessels that supply blood to the brain such as stroke), and peripheral arterial disease (disease of blood vessels that supply blood to the arms and legs). More particularly, NSAID such as acetilsalicylic acid is administered for treatment or prevention of platelet aggregation, unstable angina pectoris, suspected or diagnosed acute myocardial infarction, prophylaxis of repeated myocardial infarction, condition after vascular surgery (e.g. PTCA, CABG), prophylaxis of transient ischemic attacks and stroke in the period of initial symptoms, prophylaxis of coronary thrombosis in patients with multiple risk factors.

The wording "may" as used in the present disclosure is used interchangeably with the word "can" to indicate operability of a referenced item, the ability of a referenced item to perform one or more functions and/or activities, and/or inclusion of a referenced item within the scope of the disclosure, according to the related context as will be understood by a skilled person upon reading of the disclosure.

In some embodiments of the use of the disclosure, the NSAID administration is performed in combination with administering rifaximin and at least one proton pump inhibitor and optionally a further antibiotic and the combined administration is performed to treat and/or prevent the gastrointestinal damage in the upper and/or or lower GI tract associated with the repeated administration of NSAIDs required for treatment of pain and/or inflammation, and in particular for treatment and/or prevention of a cardiovascular disease of the individual.

In some embodiments of the use of the disclosure the NSAID administration is performed in combination with administering rifaximin, in association with NSAID in separate or single composition with at least one proton pump inhibitor and optionally a further antibiotic and the combined administration is performed to treat and/or prevent the gastrointestinal damage in the upper and/or or lower GI tract associated with the repeated administration of NSAIDs.

The term "rifaximin" as used herein indicates a semi-synthetic antibiotic belonging to the class of rifampicins, more precisely it is a pyrido-imidazo rifamycin (INN, see The Merck Index, XIII ed., 8304, CAS No. 80621-81-4), with IUPAC nomenclature (2S, 16Z, 18E, 20S, 21S, 22R, 23R, 24R, 25S, 26S, 27S, 28E)-5, 6, 21, 23, 25 pentahydroxy-27-methoxy-2, 4, 11, 16, 20, 22, 24, 26-octamethyl-2,7-(epoxypentadeca-(1,11,13)trienimino) benzofuro (4,5-e) pyrido (1,2,-a benzimidazole-1,15(2H) dione, 25-acetate). Rifaximin is currently available under the trademarks Normix®, Rifacol® and Xifaxan® (see also IT 1154655, and EP 0161534 the latter in particular describing a process for the production starting from rifamycin O (The Merck Index XIII ed., 8301)).

"Rifaximin" in the sense of the present disclosure includes solvates and polymorphous forms, including for example, Form α, Form β, Form γ, Form δ, Form ε, Form ζ, Form η, Form í, Form β-1, Form β-2, Form ε-dry, mesylate Form, amorphous form and any mixture thereof. IT 1 349 654 discloses Form α, Form β, and Form γ; EP 1 698 630 discloses Form δ, and Form ε; WO 2008/035109, WO 2008/155728, US 7,709,634 disclose amorphous form; WO 2009/108730 discloses Form ζ; Form γ-1; Form η; Form 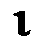 (iota); Form β-1; Form β-2; and amorphous Form; WO 2011/156897 discloses Form APO-I and Form APO-II; WO 2011/153444 discloses Form kappa and Form theta; WO 2011/103120 discloses Form ζ; Form η; Form 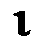 (iota); Form 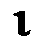 -dry; and Form B; WO 2012/155981 discloses a pseudo-crystalline form; WO2012/156951 discloses Form K; WO 2012/150561 discloses a dimethylformamide solvate. Various polymorphs of rifaximin are disclosed in U.S. patents: 7,045,620 (Form α, Form β, Form γ,), 8,217,054 (stabilized Form β) 8,193,196 (Form δ and Form ε) 8,067,429 (Form ζ, Form η, Form α-dry, Form i and amorphous and 8,227,482 (Form Mu, Form Pi, Form Omicron, Form Xi, Form Zeta, Form Eta and Form lota) .

The terms "polymorph" "polymorphous form" or "polymorphism", as used herein, refers to the occurrence of different crystalline forms of a single compound in distinct hydrate or solvate status, e.g., a property of some compounds and complexes. Thus, polymorphs are distinct solids sharing the same molecular formula, yet each polymorph can have distinct physical properties. Therefore, a single compound can give rise to a variety of polymorphic forms where each form has different and distinct physical properties, such as solubility profiles, melting point temperatures, hygroscopicity, particle shape, density, flowability, compatibility and/or x-ray diffraction peaks. The solubility of each polymorph can vary, thus, identifying the existence of pharmaceutical polymorphs is essential for providing pharmaceuticals with predictable solubility profiles. It is desirable to investigate all solid state forms of a drug, including all polymorphic forms, and to determine the stability, dissolution and flow properties of each polymorphic form. Polymorphic forms of a compound can be distinguished in a laboratory by X-ray diffraction spectroscopy and by other methods such as, infrared spectrometry. For a general review of polymorphs and the pharmaceutical applications of polymorphs see G. M. Wall, Pharm Manuf. 3, 33 (1986); J. K. Haleblian and W. McCrone, J. Pharm. Sci., 58, 911 (1969); and J. K. Haleblian, J. Pharm. Sci., 64, 1269 (1975). As used herein, the term polymorph is occasionally used as a general term in reference to the forms of rifaximin and includes within the context, salt, hydrate, polymorphic and amorphous forms of rifaximin as disclosed herein. This use depends on context and will be clear to one of skill in the art. Additional features of the antibiotic rifaximin which can be used in compositions, methods and systems herein described will be identifiable by a skilled person upon reading of the present disclosure. As used herein, the terms "antibiotic" and "antibacterial" are used interchangeably and refer to one or more active agents that inhibit bacterial growth (bacteriostatic) or kill bacteria (bactericidal).

WO 2006/094737, WO 2012/038898 and WO 2009/108814 describe the use of rifaximin in gastroresistant and not gastroresistant compositions in the Crohn's disease treatment, but these documents do not do any mention to the use of rifaximin in the treatment of intestinal damage produced by NSAID administration.

The term "gastric acid inhibitor" as used herein refers to any compound that would have the effect of reducing the acid content of the stomach or inhibiting acid production in the stomach.

In embodiments herein described, one or more of an NSAID, rifaximin, and optionally at least one antibiotic and/or at least one gastric acid inhibitor are administered in combination to obtain treatment of the conditions presenting pain and/or inflammation and in particular the treatment of cardiovascular disease while treating and/or preventing adverse effect associated with NSAID administration and in particular adverse effects in the gastrointestinal tract, wherein "combined administration" of one therapeutic agent, or administration of a therapeutic agent "in combination with" one or more further therapeutic agents according to the present disclosure comprises simultaneous (concurrent) and consecutive (sequential) administration of the referenced active agents (NSAID, antibiotic, and/or gastric acid inhibitor) performed in any order.

In some embodiments herein described wherein treatment and/or prevention of an NSAID enteropathy are desired, an effective amount of at least one antibiotic may be administered to the individual for a period selected from at least one week, 10 days, two weeks or more than two weeks, e.g., up to two months in combination with at least one NSAID, and at least one proton pump inhibitor. In some of those embodiments, a combination of at least one NSAID, at least one proton pump inhibitor and at least one antibiotic may be administered for two weeks or more.

In some embodiments herein described administration of the at least one antibiotic and the proton pump inhibitor are performed in connection with an NSAID treatment associated with occurrence of a severe enteropathy in the individual.

A severe enteropathy in the sense of the present disclosure is an enteropathy associated with signs and symptoms that can have a severe impact on sufferers' abilities to lead normal everyday lives., such as enteropathies associated with presence of small-bowel lesion of categories (3) and (4) in accordance with the categorization for NSAID enteropathy assigned by Graham DY et al in Clin Gastr. Hepatol. 2005, 3, 55 which assigns small bowel lesion into 4 categories: (1) red spots, defined as demarcated, usually circular, 1-3 mm area of crimson mucosa with preservation of villous patter; (2) ulceration/erosion, defined as a red spot with clear loss of villi across the area of the lesion, <5mm in diameter with a definite edge; (3) ulcers, defined as a penetrating lesion of mucosa with a diameter of >5 mm; and category (4), large erosion ulcers. Reference is made to the Examples section and in particular to. Examples 18 and 19 showing exemplary embodiments wherein the use of rifaximin in accordance with the present disclosure proved particularly effective in connection with the treatment and/or prevention of severe enteropathies. In particular severe enteropathies are typically associated with NSAID long term treatment of at least two weeks, at least two months, two to six months, or one year and more including, chronic administration and life long treatments as will be understood by a skilled person.

In some embodiments herein described, during the NSAID treatment, the NSAID may be administered once a day (OD), twice a day (BID), three times a day (TID), four times a day (QDS) or more often as necessary at dosage from 5 to 1500 mg a day, depending on the NSAID chosen as will be understood by a skilled person in the art.

Timing of administration of rifaximin to treat and/or prevent the enteropathy can vary depending on the individual treated, the effect to be achieved (treatment and/or prevention of enteropathy) and the severity of the enteropathy as will be understood by a skilled person.

The rifaximin may be administered once a day (OD), twice a day (BID), three times a day (TID), four times a day (QDS) or more often as necessary at dosages from 20 to 3300 mg a day, or from 20 to 2400 mg a day depending on the antibiotic chosen as will be understood by a skilled person in the art.

In a particular embodiment, rifaximin may be administered once a day (OD), twice a day (BID), three times a day (TID), four times a day (QDS) or more often, as necessary at dosages from 200 to 3300 mg a day depending on the antibiotic chosen as will be understood by a skilled person in the art.

More particularly, in some embodiments, rifaximin may be administered OD, BID, TID, QDS or more often at a daily dosage in a range comprising 20 mg, 40 mg, 60, mg, 80 mg or more a day within the above dosage range.

In some embodiments, rifaximin may be administered OD, BID, TID, QDS or more often at a daily dosage from 50 mg, 100 mg, 150, mg, to 200 mg or more a day within the above dosage range.

In some embodiments, rifaximin may be administered OD, BID, TID, QDS or more often at a daily dosage from 100 mg, 200 mg, 300 mg, to 400 mg or more a day within the above dosage range.

In some embodiments, rifaximin may be administered OD, BID, TID, QDS or more often at a daily dosage from 200 mg, 400 mg, 600 mg, to 800 mg or more a day within the above dosage range.

In some embodiments, rifaximin may be administered OD, BID, TID, QDS or more often at a daily dosage from 400 mg, 800 mg, 1200 mg, to 1600 mg or more a day within the above dosage range.

In some embodiments, rifaximin may be administered OD, BID, TID, QDS or more often at a daily dosage from 550 mg, 1100 mg, 1650 mg, to 2200 mg or more a day within the above dosage range.

In some embodiments, rifaximin may be administered OD, BID, TID, QDS or more often at a daily dosage from 600 mg, 1200 mg, 1800 mg, to 2400 mg or more a day within the above dosage range.

In some embodiments, rifaximin may be administered OD, BID, TID, QDS or more often at a daily dosage from 800 mg, 1600 mg, 2400 mg, to 3200 mg or more a day within the above dosage range.

In some embodiments, rifaximin may be administered OD, BID, TID, or more often at a daily dosage from 1100 mg, 2200 mg, to 3300 mg.

In some embodiments one or more of a proton pump inhibitor may also be administered once a day (OD), twice a day (BID), three times a day (TID), four times a day (QDS) or more often as necessary at dosages from 5 to 2000 mg a day, depending on the proton pump inhibitor chosen as will be understood by a skilled person.

In some embodiments, the timing and dosage of a combined use and administration of rifaximin and the at least one proton pump inhibitor may be selected to provide the individual with an amount of rifaximin and the proton pump inhibitor that is effective to prevent the NSAID gastrointestinal damage in the individual under NSAID administration. Each of the NSAID, rifaximin and proton pump inhibitor is administered using a dosage and time interval for each class of drug to exert its therapeutic effect, specific for its class of action. In particular, in embodiments herein described, each one of NSAID, rifaximin and proton pump inhibitor may be administered in combination at least OD, at any time of the day. In some of those embodiments, an NSAID, rifaximin and proton pump inhibitor may be administered in combination OD in the evening.

In some embodiments, an NSAID and rifaximin may be administered in combination BID, for example, in a first administration and a second administration performed in a combination in an interval of approximately 12 hours between administrations. In some of those embodiments, NSAID administration and rifaximin administration may be performed OD, BID or TID in combination with a proton pump inhibitor administered OD. In some of those embodiments, a first administration of at least one NSAID and/or at least one antibiotic administered in combination may be performed in the morning and a second administration of at least one NSAID and/or rifaximin administered in combination may be performed in the evening. In some of those embodiments, a proton pump inhibitor may also be administered in the evening in combination with the NSAID and optionally rifaximin.

In some embodiments, the NSAID may be administered to the individual at least BID wherein the administering comprises administering the NSAID and a first one of rifaximin or one or more proton pump inhibitors at least once a day and administering the NSAID and a second one of rifaximin and the one or more proton pump inhibitors at least once a day.

In some embodiments, the antibiotic and NSAID may be administered concurrently, combined in a single dosage form (e.g., rifaximin and NSAID in a single tablet or capsule, or antibiotic and NSAID in a single vehicle such as rifaximin and NSAID granulates dissolved in water).

In some embodiments, rifaximin and NSAID may be administered at the same or at different times in separate dosage forms, wherein antibiotic may be administered before or after the NSAID.

In particular, in some embodiments herein described, rifaximin may be administered one, two, three, four, five, six, seven, eight, nine, ten, eleven or twelve hours or more, either before or after the NSAID is administered. Exemplary dosages and regimens of dosages and regimens of the three active agents according to embodiments herein described are illustrated below.

**TABLE 1**

| **NSAID (e.g., dose range 5-1500 mg)** | **Proton pump inhibitor** | **Rifaximin** |
|---|---|---|
| OD | OD in the morning | OD(×1), dosage, e.g., 20, 50 100, 200. 400, 550, 600, 800, 1100 mg |
| BID | OD in the evening | BID(×2) dosage, e.g., 20, 50, 100, 200, 400, 550, 600, or 800, 1100 mg BID (×2) dosage, e.g., 400 |
| TID | BID morning and evening | |
| OD, BID or TID | None, OD or TID | TID (×3) dosage, e.g., 20, 50, 100, 200, 400, 550, 600, 800, or 1100 mg |
| OD, BID or TID | None, OD or BID | QDS (×4) dosage, e.g., 20, 50, 100, 200, 400, 550 600, or 800 mg |
| OD, BID or TID | None | OD(×1), BID (×2), TID (×3) or QDS (×4) dosage, e.g., 100, 200, 400, 550, 600, 800, or 1100 mg |

Exemplary dosages for the NSAID and for the rifaximin when the antibiotic is rifaximin are reported in Table 1. A skilled person will be able to understand additional dosages in connection with timing of administration upon reading of the present disclosure.

For example in some embodiments, exemplary of NSAID usage for arthritic conditions and/or acute muscular-skeletal disorders and/or other painful conditions and/or cardiovascular disease, are treated or prevented with NSAID administration comprising daily dosages from 20 to 1500 mg for a period of at least one week up to two months or greater.

Additional dosages and timing of administration of the NSAID, a proton pump inhibitor (PPI), and rifaximin, will be identifiable by a skilled person based on the NSAID, proton pump inhibitor and rifaximin selected and the specific one or more conditions treated as will be understood by a skilled person. Similarly, based on the information disclosed herein, specific formulations of the NSAID, proton pump inhibitor, and rifaximin may also be determined by a skilled person based on the NSAID, proton pump inhibitor and rifaximin selected and the specific one or more conditions treated.

In some embodiments, administration of the NSAID, rifaximin and proton pump inhibitor may be performed in separate dosage forms or a single unified dosage form comprising an NSAID and rifaximin or a unified dosage form comprising NSAIDs, antibiotic and proton pump inhibitor. The NSAID may be administered once a day (OD), twice a day (BID), three times a day (TID), four times a day or more often as necessary at dosage comprising from 5 to 1500 mg a day, depending on the NSAID chosen.

In some embodiments, NSAID administered is, for example, selected from the group consisting of: diclofenac, acetylsalicylic acid, ibuprofen, ketoprofen, naproxen and pharmaceutically acceptable salts thereof and mixtures thereof. In some of these embodiments, NSAID is administered once a day (OD), twice a day (BID), three times a day (TID), four times a day or more often as necessary at dosages from 5 to 1500 mg a day, depending on the NSAID chosen. Rifaximin may be administered once a day (OD), twice a day (BID), three times a day (TID), four times a day or more often as necessary at dosages from 20 to 3300 mg a day and or from 20 to 2400 mg a day.

Proton pump inhibitors may be administered once a day (OD), twice a day (BID), three times a day (TID), four times a day or more often as necessary at dosages from 5 to 2000 mg a day, depending on the proton pump inhibitor chosen.

In some embodiments, the proton pump inhibitor can be chosen among PPI such as lansoprazole, ilaprazole, omeprazole, tenatoprazole, rabeprazole,
esomeprazole, pantoprazole, pariprazole, leminoprazole or nepaprazole or a free base, a free acid, a salt, a hydrate, an ester, an amide, an enantiomer, an isomer, a tautomer, a polymorph, a prodrug or any derivative thereof.

In various embodiments, the doses of NSAID, rifaximin and proton pump inhibitor per day may be given at the same time (or times) daily, or at different time (or times) daily.

Additional dosages may be used which provide the individual with a therapeutically effective amount or a prophylactically effective amount in accordance with the related embodiments of the disclosure. In particular, the term "effective amount" of one or more active ingredients refers to a nontoxic but sufficient amount of one or more drugs to provide the desired effect. For example, an "effective amount" associated with the treating and/or preventing (herein also "therapeutically effective amount" or "pharmaceutically effective amount") by at least one NSAID a condition in the individual in which pain and/or inflammation are present, refers to a non-toxic but sufficient amount of the at least one NSAID to provide the treatment and/or prevention of such condition in the individual. As another example, an "effective amount" of at least one antibiotic and proton pump inhibitor associated with the treating and/or preventing an NSAID enteropathy in the individual refers to a non-toxic but sufficient amount of the at least one antibiotic and/or at least one proton pump inhibitor to provide the treatment and/or prevention of the NSAID enteropathy in the individual.

Accordingly, in some embodiments herein described a therapeutically effective amount of at least one antibiotic and at least one proton pump inhibitor indicates amounts able to treat and/or prevent gastrointestinal damage in the individual. In some embodiments, a therapeutically effective amount of at least one NSAID indicates amounts able to treat and/or prevent a condition wherein pain and/or inflammation are present in the individual. Accordingly, a therapeutically effective amount of, at least one antibiotic, at least one NSAID and at least one proton pump inhibitor, comprises for example, dosages and formulations (e.g., in one or more pharmaceutical compositions comprising the active ingredients) necessary to treat and/or prevent the recited condition in the individual, and particularly arthritis or other painful and/or inflammatory conditions while treating and/or preventing gastrointestinal damage associated with NSAID use, at a reasonable benefit/risk ratio applicable to any medical treatment.

In exemplary embodiments, a therapeutically effective amount of the antibiotic can be comprised between 20 to 800 mg OD, BID,TID, QDS until the end of treatment with NSAID. In some embodiments the antibiotic can be administerd for a treatment period following the end of the NSAID treatment, e.g. for at least one week or for at least two weeks.

In some embodiments of the compositions for use herein described, rifaximin can be administered in combination with at least one antibiotic, in combination with the NSAID and at least one proton pump inhibitor. In particular, in some embodiments the antibiotic can be one or more of an antibiotic having effect in the GI tract and/or having low systemic absorption. Low systemic absorption includes, for example, less than 10% absorption, less than 5% absorption, less than 1% absorption and less than 0.5% absorption. Low systemic absorption also includes, for example, from about 0.01 to about 1% absorption, from about 0.05 to about 1% absorption, from about 0.1 to about 1% absorption, from about 1 to about 10% absorption, or from about 5 to about 20% absorption.

More particular, in some embodiments the antibiotic is one or more of a "GI specific antibiotic" and "GI antibiotic" which terms, as used herein, include antibiotic known or determined to have an effect on GI disease. For example, a rifamycin class antibiotic (e.g., rifaximin), neomycin, metronidazole, teicoplanin, ciprofloxacin, doxycycline, tetracycline, augmentin, cephalexin, penicillin, ampicillin, kanamycin, rifamycin, vancomycin, and combinations thereof are useful GI specific antibiotics. In an embodiment the antibiotic can be a GI specific antibiotic having low absorption.

In embodiments herein described, the antibiotic is administered using a dosage and time interval for each class of drug to exert its therapeutic effect, specific for its class of action. In particular, in embodiments herein described, the at least one antibiotic can be administered in combination with each one of NSAID, rifaximin and gastric acid inhibitor, at least OD, at any time of the day. In some of those embodiments, an NSAID, rifaximin, proton pump inhibitor and the at least one antibiotic may be administered in combination OD in the evening.

In some embodiments, the at least one antibiotic may be administered once a day (OD), twice a day (BID), three times a day (TID), four times a day or more often as necessary at dosages from 20 to 3300 mg a day and or from 20 to 2400 mg a day depending on the antibiotic chosen

In some embodiments, the at least one antibiotic can be, for example, selected from the group consisting of: rifamycin class antibiotics aminoglycoside, amphenicol, ansamycin, beta-Lactam, carbapenem, cephamycin, monobactam, oxacephem, lincosamide, macrolide, polypeptide, tetracycline, a 2,4-diaminopyrimidine class antibiotic, penicillin, neomycin, metronidazole, vancomycin, paromomycin, timidazole, clarithromycin, amoxicillin, sulfasalazine; olsalazie; mesalamine; prednisone; azathioprine; mercaptopurine; methotrexate, ampicillin, clindamycin, rifampicin, rifamycin, vancomycin, chloramphenicol, spectinomycin, fluoroquinolones, and cephalosporins. The fluoroquinolone antibiotic can be at least one selected from the group of: balofloxacin, ciprofloxacin, difloxacin, enrofloxacin, fleroxacin, gatifloxacin, grepafloxacin, levofloxacin, lomefloxacin, marbofloxacin, moxifloxicin, nadifloxacin, norfloxacin, ofloxacin, orbifloxacin, pazufloxacin, perfloxacin, rufloxacin, sparfloxacin, temafloxacin, and tosufloxacin. The cephalosporin antibiotic can be at least one selected from the group of: cefacetrile, cefaclomezine, cefaclor, cefadroxil, cefalexin, cefaloglycin, cefalonium, cefaloram, cefaloridine, cefalotin, cefaparole, cefapirin, cefatrizine, cefazaflur, cefazedone, cefazolin, cefbuperazone, cefcanel, cefcapene, cefclidine, cefdaloxime, cefdinir, cefditoren, cefedrolor, cefempidone, cefepime, cefetamet, cefetrizole, cefivitril, cefixime, cefluprenam, cefmatilen, cefmenoxime, cefmepidium, cefmetazole, cefminox, cefodizime, cefonicid, cefoperazone, cefoselis, cefotaxime, cefotetan, cefovecin, cefoxazole, cefoxitin, cefozopran, cefpimizole, cefpirome, cefpodoxime, cefprozil, cefquinome, cefradine, cefrotil, cefroxadine, cefsumide, ceftaroline, ceftazidime, cefteram, ceftezole, ceftibuten, ceftiofur, ceftiolene, ceftioxide, ceftizoxime, ceftriaxone, cefuracetime, cefuroxime, cefuzonam, and loracarbef. In some of those embodiments, the antibiotic can be administered enterally or parenterally, OD or BID with dosages and schedules identifiable by a skilled person upon reading of the present disclosure in connection with the desired effect of treating and/or preventing a condition of the GI tract, particularly of the intestinal tract.

More particularly, in some embodiments, the at least one antibiotic may be administered OD, BID, TID, QDS or more often at a daily dosage in a range comprising 20 mg, 40 mg, 60, mg, 80 mg or more a day.

In some embodiments, the at least one antibiotic may be administered OD, BID, TID, QDS or more often at a daily dosage from 50 mg, 100 mg, 150, mg, to 200 mg or more a day.

In some embodiments, the at least one antibiotic may be administered OD, BID, TID, QDS or more often at a daily dosage from 100 mg, 200 mg, 300 mg, to 400 mg or more a day.

In some embodiments, the at least one antibiotic may be administered OD, BID, TID, QDS or more often at a daily dosage from 200 mg, 400 mg, 600 mg, to 800 mg or more a day.

In some embodiments, the at least one antibiotic may be administered OD, BID, TID, QDS or more often at a daily dosage from 400 mg, 800 mg, 1200 mg, to 1600 mg or more a day.

In some embodiments, the at least one antibiotic may be administered OD, BID, TID, QDS or more often at a daily dosage from 550 mg, 1100 mg, 1650 mg, to 2200 mg or more a day.

In some embodiments, the at least one antibiotic may be administered OD, BID, TID, QDS or more often at a daily dosage from 600 mg, 1200 mg, 1800 mg, to 2400 mg or more a day.

In some embodiments, the at least one antibiotic may be administered OD, BID, TID, QDS or more often at a daily dosage from 800 mg, 1600 mg, 2400 mg, to 3200 mg or more a day.

In some embodiments, the at least one antibiotic may be administered OD, BID, TID, or more often at a daily dosage from 1100 mg, 2200 mg, to 3300 mg or more a day.

In embodiments where more than one antibiotic is administered, the administered antibiotics can be administered at the same time or at different time depending each class of drug and related dosage and time interval identified to exert its therapeutic effect, specific for its class of action In some embodiments the at least one antibiotic may be administered together with rifaximin according to the rifaximin regimens of the various embodiments herein described. For example in some embodiments, exemplary dosages and regimens of dosages and regimens of the at least one antibiotic are the same indicated for rifaximin in the illustration of Table 1 which can be applied to the at least one antibiotic as well.

Enterobacteria and cytokines both play roles in the pathophysiology of NSAID-induced enteropathy. In the small bowel, NSAIDs enhance gut permeability and induce mucosal inflammation. Once the mucosal barrier has been disrupted by NSAIDs, luminal gram negative bacteria can enter the cell and activate Toll-like receptor (TLR) 4, which recognizes lipopolysaccharide (LPS), a major cell wall component of Gram- negative bacteria, resulting in activation of an inflammatory cascade. Nuclear factor-κB (NF-κB) is the final effector molecule of the TLR4 signaling pathway. It promotes the development of many intestinal diseases and it also plays a pivotal role in the translation and transcription of inflammatory mediators.

In the gastrointestinal system, PXR has a role as a modulator of inflammation in the intestinal mucosa barrier. The PXR is a nuclear receptor that regulates genes involved in xenobiotic metabolism and limited antibiotic deposition and detoxication. The mechanisms of the protective effect of PXR activation in intestinal inflammation is in part due to the attenuation of nuclear factor kappa B (NF-κB) signaling that results in lower expression of proinflammatory cytokines. For example, rifaximin, in addition to its anti-bacterial activity, is a gut-specific PXR agonist, that suppresses the expression of NF-Kappa B regulated genes and is a negative regulator of inflammation and immunological responses in human intestine.

Accordingly, in some embodiments of the compositions for use herein described the NSAID administration is performed in combination with administering rifaximin possibly in combination with at least one PXR agonist, at least one proton pump inhibitor and/or at least one antibiotic, wherein the combined administration is performed to treat and/or prevent the gastrointestinal damage in the upper and/or or lower GI tract associated with repeated administrations of NSAIDs required for treatment of pain and/or inflammation and/or related conditions in the individual. In those embodiments, the at least PXR is expected to increase the therapeutic effect with the rifaximin to treat or prevent the enteropathy in the individual. In some embodiments, the PXR agonist is an antibiotic.

As used herein, the term "PXR agonist" refers to one or more active agents that can activate the pregnane X receptor ("PXR"). Activation of PXR can result in inhibiting, reducing or preventing inflammation of the bowel and related tissues and organs. Exemplary PXR agonists comprise PCN, rifampicin, RU486, SR12813, taxol, hyperforin, 5β-pregnane-3,20-dione, lithocholic acid, metyrapone, clotrimazole, phenobarbital, spironolactone, trans-nonachlor, nifedipine, ritonavir, tamoxifen, 4-hydroxytamoxifen, troglitazone, lovastatin, glutethimide, bisphenol A, diethylhexylphthalate, nonyl-phenol, pregnenolone, 17α-hydroxylated derivative of prenenolone, progesterone, 17a-hydroxylated derivative of progesterone, estradiol, and corticosterone. Other PXR agonists will be identifiable by those of skill in the art.

In embodiments herein described, an NSAID, rifaximin, at least one PXR agonist, a proton pump inhibitor and/or at least one antibiotic are administered in combination to obtain treatment of the conditions presenting pain and/or inflammation, (e.g. arthritic conditions, acute musculo-skeletal disorders, conditions resulting from trauma, chronic myofascial pain and cardiovascular diseases) while treating and/or preventing adverse effect associated with NSAIDs administration and in particular adverse effect in the gastrointestinal tract, wherein "combined administration" of one therapeutic agent, or administration of a therapeutic agent "in combination with" one or more further therapeutic agents according to the present disclosure comprises simultaneous (concurrent) and consecutive administration of the referenced principles performed in any order. The various therapeutic agents for use with the disclosure may therefore be administered in any order to achieve treatment or prevention of the underlying condition for which one or more NSAIDs are administered and minimizing the development of or treating enteropathies caused by the NSAIDs by administering an appropriate combination of antibiotic and gastric acid inhibitor, separately, or together with each other, and the NSAIDs.

In some embodiments herein described wherein treatment and/or prevention of an NSAID enteropathy and/or of a condition presenting pain and/or inflammation are desired, an effective amount of rifaximin in combination with at least one PXR agonist may be administered to the individual for a period selected from at least one week, 10 days, two weeks or more than two weeks up to two months or later in combination with at least one NSAID, and at least one proton pump inhibitor. In some of those embodiments, a combination of at least one NSAID, at least one proton pump inhibitor and at least one PXR agonist may be administered for two weeks or more.

In some embodiments herein described, the NSAID may be administered once a day (OD), twice a day (BID), three times a day (TID), four times a day or more often as necessary at dosage comprising from 5 to 1500 mg a day, depending on the NSAID chosen as will be understood by a skilled person.

In some embodiments the at least one PXR agonist may be administered once a day (OD), twice a day (BID), three times a day (TID), four times a day (QDS) or more often as necessary at dosages sufficient to activate PXR activity. The dosage sufficient to activate PXR activity will vary according to the selected PXR agonist and will be understood by a skilled person. For example, a sufficient dosage may comprise from 20 mg to 5000 mg per day, or from 100 to 2500 mg per day.

In some embodiments one or more of a proton pump inhibitor may be administered once a day (OD), twice a day (BID), three times a day (TID), four times a day (QDS) or more often as necessary at dosages comprising from 5 to 2000 mg a day, depending on the proton pump inhibitor chosen as will be understood by a skilled person.

In particular, in embodiments herein described, each one of NSAID, rifaximin, PXR agonist, proton pump inhibitor and antibiotic may be administered in combination at least OD, at any time of the day. In some of those embodiments, an NSAID, rifaximin, PXR agonist, proton pump inhibitor and antibiotic may be administered in combination OD in the evening.

In some embodiments, an NSAID, rifaximin and PXR agonist may be administered in combination BID, for example, in a first administration and a second administration performed in combination in an interval of approximately 12 hours between administrations. In some of those embodiments, NSAID administration and rifaximin and PXR agonist administration may be performed OD, BID or TID in combination with a proton pump inhibitor administered OD. In some of those embodiments, a first administration of at least one NSAID, rifaximin and/or at least one PXR agonist administered in combination may be performed in the morning and a second administration of at least one NSAID, rifaximin and/or at least one PXR agonist administered in combination may be performed in the evening. In some of those embodiments, a proton pump inhibitor may also be administered in the evening in combination with the NSAID and optionally rifaximin and/or the PXR agonist.

In some embodiments, the NSAID may be administered to the individual at least BID wherein the administering comprises administering the NSAID and a first one of rifaximin in combination with one or more PXR agonists or one or more proton pump inhibitors at least once a day and administering the NSAID and a second one of rifaximin in combination with the one or more PXR agonists and the one or more proton pump inhibitors at least once a day.

In some embodiments, rifaximin, the PXR agonist and NSAID may be administered concurrently, combined in a single dosage form (e.g., rifaximin, PXR agonist and NSAID in a single tablet or capsule, or rifaximin, PXR agonist and NSAID in a single vehicle such as PXR agonist and NSAID granulates dissolved in water).

In some embodiments, rifaximin the PXR agonist and NSAID may be administered at the same or at different times in separate dosage forms, wherein the PXR agonist may be administered before or after the NSAID.

In particular, in some embodiments herein described, the PXR agonist may be administered one, two, three, four, five, six, seven, eight, nine, ten, eleven or twelve hours or more, either before or after the NSAID is administered.

As will be readily apparent to one skilled in the art, the useful *in vivo* dosage to be administered and the particular mode of administration will vary depending upon the age, weight and mammalian species treated, the particular compounds employed, and/or the specific use for which these compounds are employed. The determination of effective dosage levels, that is the dosage levels necessary to achieve the desired result, may be accomplished by one skilled in the art using routine pharmacological methods. Typically, human clinical applications of products are commenced at lower dosage levels, with dosage level being increased until the desired effect and durability of response is achieved (e.g. in clinical practice where a therapeutic effect is sought or dose ranging finding clinical study where selection of a dose associated to a set effect is sought).

As used herein, "durability of response" includes for example, adequate relief of symptoms after removal of treatment, continuous adequate relief of symptoms after removal of treatment, or response that is greater than or superior to placebo response. The response can be measured, for example using one or more of the methods outlined below, including, for example, a subject's subjective assessment of their symptoms or a healthcare provider's or caretaker's assessment of a subject's symptoms.

In certain embodiments, one or more NSAID(s) may be cyclically administered with one or more of rifaximin and optionally an antibiotic, PXR agonist, and/or proton pump inhibitors. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, e.g., the cycle in order to reduce the development of resistance to one of the therapies, to avoid or reduce the side effects of one of the therapies, and/or to improve the efficacy of the therapies. In particular in some embodiments, the first prophylactic or therapeutic agent may comprise one or more NSAIDs and the second prophylactic or therapeutic agent may comprise one or more antibiotics, and in particular rifaximin. In some embodiments, the first prophylactic or therapeutic agent may comprise rifaximin optionally in combination with one or more antibiotics and the second prophylactic or therapeutic agent may be one or more NSAID(s). In some embodiments, the first prophylactic or therapeutic agent may comprise one or more NSAIDs and the second prophylactic or therapeutic agent may comprise rifaximin optionally in combination with one or more PXR agonists. In some embodiments, the first prophylactic or therapeutic agent may comprise rifaximin optionally in combination with one or more PXR agonists, and the second prophylactic or therapeutic agent may be one or more NSAID(s). In some of those embodiments, the third prophylactic or therapeutic agent may comprise a proton pump inhibitor. In some of those embodiments, the first prophylactic or therapeutic agent and the second prophylactic or therapeutic agent may be administered concurrently or at different times. In some embodiments the first and second prophylactic or therapeutic agent may be administered in a single unified dosage form. In some embodiments the first and second prophylactic or therapeutic agent may be administered in separate dosage forms. In some embodiments, the administration of the same compounds may be repeated and the administrations may be separated by at least about 1 week, 2 weeks, 3 weeks, 4 weeks, 5 weeks, 6 weeks, 8 weeks, 10 weeks, 12 weeks, 14 weeks, 16 weeks, 18 weeks, 20 weeks, 22 weeks, or at least about 24 weeks.

As with other pharmaceuticals, it will be understood that the total daily usage of one or more pharmaceutical compositions of the present disclosure will be decided by a patient's attending physician within the scope of sound medical judgment. The specific therapeutically effective or prophylactically effective dose level for any particular patient will depend upon a variety of factors including the disorder being treated and the severity of the disorder; activity of the specific compound employed; the specific composition employed; the age, body weight, general health, sex and diet of the patient; the time administration, route of administration, and rate of excretion of the specific compound employed; the duration of the treatment; drugs used in combination or coincidental with the specific compound employed; and other factors known to those of ordinary skill in the medical arts.

In some embodiments of the compositions for use of the disclosure the NSAID administration is performed in combination with administering rifaximin, in association with NSAID in separate or single composition optionally with at least one proton pump inhibitor and/or at least one antibiotic and the combined administration is performed to treat and/or prevent the gastrointestinal damage in the upper and/or or lower GI tract associated with the repeated administration of NSAIDs in elderly patients.

Accordingly, the amount of drug that is "effective" will vary from subject to subject, depending on the age and general condition of the individual, the particular drug or drugs, and the like and an appropriate "therapeutically effective amount" or "prophylactically effective amount" in any individual case can be determined by one skilled in the art.

In some embodiments of the compositions for use herein described NSAID administration in combination with rifaximin, and/or an antibiotic, and/or an PXR agonist and/or a proton pump inhibitor may be performed preferably by oral and parenteral administration of the related active ingredients optionally included in suitable formulations and kits as will be understood by a skilled person.

In embodiments, herein described rifaximin administration in combination with an NSAID and/or a proton pump inhibitor may be performed in particular by oral and parenteral administration of the related active ingredients optionally included in suitable formulations as will be understood by a skilled person.

In embodiments, herein described administration of rifaximin, proton pump inhibitor in combination with an NSAID and/or antibiotic may be performed in particular by oral and parenteral administration of the related active ingredients optionally included in suitable formulations as will be understood by a skilled person.

In some embodiments, bowel lesions induced by different NSAIDs exacerbated by dysbiosis induced by PPI, can be the effective target of the antimicrobial therapy in order to reduce and heal the lower gastrointestinal lesions associated with NSAID use.

In some embodiments, rifaximin can prevent, reduce and heal the lower gastrointestinal lesions, meanwhile the administration of proton pump inhibitor protect the mucosa of the upper gastrointestinal tract from the well acknowledged damage NSAID-induced.

In some embodiments, the combined administration of rifaximin, at least one NSAID, at least one antibiotic and at least one proton pump inhibitor is expected to result in a successful treatment for subject suffering from a condition presenting pain and/or inflammation such as rheumatological diseases for which need a long term therapy with NSAIDs.

In embodiments herein described, administration of rifaximin, in combination with an NSAID and optionally a proton pump inhibitor and/or an a PXR agonist may be performed by oral and parenteral administration of the related active ingredients optionally included in suitable formulations as will be understood by a skilled person.

In embodiments herein described, administration of proton pump inhibitor in combination with an NSAID and optionally the PXR agonist may be performed by oral and parenteral administration of the related active ingredients optionally included in suitable formulations as will be understood by a skilled person.

In some embodiments, bowel lesions induced by different NSAIDs exacerbated by dysbiosis induced by PPI, can be the effective target of PXR activation in order to reduce and heal the lower gastrointestinal lesions associated with NSAID use.

In some embodiments, the PXR agonist can prevent, reduce and heal the lower gastrointestinal lesions, meanwhile the administration of proton pump inhibitor protect the mucosa of the upper gastrointestinal tract from the well acknowledged damage NSAID-induced.

In some embodiments, the combined administration of at least one NSAID, rifaximin, at least one PXR agonist and at least one proton pump inhibitor can result in a successful treatment for subject suffering from a condition presenting pain and/or inflammation such as rheumatological diseases or cardiovascular disease for which a long term therapy with NSAIDs is needed. In particular, in several embodiments, the combined administration can be used in connection with an NSAID therapy of at least ten days and in particular of at least one months, of one to six months, of at least one year and/or a longer treatment up to a life long treatment.

In some embodiments, the combined administration of at least one NSAID, rifaximin, at least one PXR agonist and at least one proton pump inhibitor can performed in an individual who is an elderly subject suffering from a condition presenting pain and/or inflammation and in particular for treatment of cardiovascular disease.

Accordingly, in some embodiments methods herein described to treat and/or prevent an NSAID enteropathy in an individual under NSAID administration comprise administering rifaximin to the individual under NSAID administration, in combination with at least one proton pump inhibitor and/or an antibiotic and/or PXR agent, wherein the rifaximin is coated with one or more polymeric materials preferably arranged in a multilayer composition, to confer or increase one or more of bioadhesivity, gastroresistance and/or controlled release of the administered rifaximin.

In some embodiments, the rifaximin can be coated by a bioadhesive polymeric material to confer or increase bioadhesivity to the rifaximin formulation. In some of those embodiments, the bioadhesive polymeric material can be hydroxyethyl cellulose, hydroxypropyl cellulose (KLUCEL®, Hercules Corp.), hydroxypropyl methylcellulose (METHOCEL®, Dow Chemical Corp.), polyvinylpyrrolidone (AVICEL®), hydroxypropyl methyl cellulose and additional polymers identifiable by a skilled person.

In particular, in some embodiments the pharmaceutical compositions can have bioadhesive or mucoadhesive properties in order to adhere to intestinal mucosa.

Examples of polymers, oligomers or their mixtures which can confer bioadhesive properties are chosen in the group comprising: pectins, zeins, casein, gelatin, albumin, collagen, kitosan, oligosaccharides and polysaccharides such as, for instance, cellulose, dextran, polysaccharides from tamarind seeds, xanthan gum, arabic gum, hyaluronic acid, alginic acid, sodium alginate.

In some embodiments, the bioadhesive polymer is a synthetic polymer, the polymer is chosen among polyamides, polycarbonates, polyalkylenes, polyalkylene glycols, polyalkylene oxides, polyalkylene terephthalates, polyvinyl alcohols, polyvinyl ethers, polyvinyl esters, polyvinylpyrrolidone, polysiloxanes, polyurethanes, polystyrenes, polymers of acrylic acid and methacrylic esters, copolymer of methacrylic acid-ethyl acrylate, polylactides, barbituric polyacids, polyanhydrides, polyorthoesters and their mixtures.

Other polymers suitable for use in the invention include, but are not limited to, methylcellulose, ethylcellulose, hydroxypropyl cellulose, hydroxybutyl methylcellulose, cellulose acetate, cellulose propionate, cellulose acetate butyrate, cellulose acetate phthalate, carboxy methyl cellulose, cellulose triacetate, cellulose sulfate sodium salt, polymethyl methacrylate, poly isobutyl acrylate, poly octadecyl acrylate, polypropylene, polyethylene glycol, polyethylene oxide, polyethylene terephthalate, polyvinyl acetate, polyvinyl chloride, polystyrene, polyvinylpyrrolidone, polyvinyl phenol and their mixtures.

Another group of polymers useful in the obtainment of bioadhesivity are polymers having a branch with at least one bonded hydrophobic group, wherein hydrophobic groups generally are non-polar groups. Examples of said hydrophobic groups comprise alkyls, alkenyls and alkyl groups. Preferably, hydrophobic groups are chosen to increase polymers bioadhesivity. Other polymers are characterized by hydrophobic branches with at least one hydrophilic group, such as carboxylic acids, sulphonic acids and phosphonic acids, neutral and positively charged amines, amides and imines, wherein the hydrophilic groups are such to increase the polymer bioadhesivity.

In some embodiments, the rifaximin can be coated by an enteric polymeric material insoluble at pH values between 1.5 and 4.0 and soluble at pH values between 5.0 and 7.5 to confer gastroresistance to the rifaximin formulation. In particular in some embodiments, the enteric polymeric material is selected from acrylic polymers, methacrylic acid copolymers, methacrylic acid copolymers with an acrylic or methacrylic ester, cellulose acetate phthalate, hydroxypropyl cellulose acetate phthalate, hydroxypropyl methylcellulose phthalate, polyvinyl acetate phthalate, methacrylic acid ethylacrylate copolymer, and methacrylic acid methylmethacrylate copolymer, polyvinyl acetate phthalate, methacrylic acid ethylacrylate copolymer, copolymers of acrylic acid, such as the copolymer methacrylic acid-ethyl acrylate 1:1, copolymer of methacrylic acid with an acrylic or methacrylic ester such as the copolymer methacrylic acid-ethyl acrylate 1:1 and the copolymer methacrylic acid-methyl methacrylate 1:2, polyvinyl acetate phtalate, hydroxy propyl methyl cellulose phtalate and cellulose acetate phtalate, commercially available products, for instance with the trademarks KOLLICOAT®, EUDRAGIT®, AQUATERIC®, AQOAT®; natural polymers like shellac, commercially available with the trademark AQUAGOLD® (shellac 25%) and ethyl cellulose.

In some embodiments, the rifaximin can be coated by a water semipermeable polymer possibly over the enteric polymeric material to control release of rifaximin in the rifaximin formulation. In some of this embodiments, the water semipermeable polymer is selected from one or more of cellulose acetate, cellulose acetate butyrate, cellulose acetate propionate, ethyl cellulose, fatty acids and their esters, waxes, zein, EUDRAGIT RS and RL 30D, EUDRAGIT® NE 30D, EUDRAGIT® 40, AQUACOAT®, SURELEASE® and cellulose acetate latex.

In some embodiments, the water semi permeable polymer can be in combination with one or more hydrophilic polymers. In some of those embodiments, the one or more hydrophilic polymers comprise hydroxyethyl cellulose, hydroxypropyl cellulose hydroxypropyl methylcellulose, polyvinylpyrrolidone. In some of those embodiments, the one or more hydrophilic polymers comprise hydroxypropyl cellulose hydroxypropyl methylcellulose, polyvinylpyrrolidone.

In some embodiments, the bioadhesive polymeric material, enteric polymer material and water semipermeable material can be arranged in a multilayer composition wherein the coated rifaximin is coated by the enteric polymeric material, the enteric polymeric material is coated by the water semipermeable polymer and the water semipermeable material is coated by the bioadhesive polymeric material.

In some embodiments the coated rifaximin is a rifaximin based mixture which comprises one or more of a diluent, a plasticizer, an anti-agglomerative agent, an anti- sticking agent, a glidant, an anti-foam agent and a coloring substance, In some emboddiments, the plasticizer can be selected from the group consisting of acetylated monoglycerides, butyl phthalyl butyl glycolate, dibutyl tartrate, diethyl phthalate, dimethyl phthalate, ethyl phthalyl ethyl glycolate, glycerin, ethylene glycol, propylene glycol, triacetin citrate, triacetin, tripropinoin, diacetin, dibutyl phthalate, acetyl monoglyceride, polyethylene glycols, castor oil, triethyl citrate, polyhydric alcohols, acetate esters, gylcerol triacetate, acetyl triethyl citrate, dibenzyl phthalate, dihexyl phthalate, butyl octyl phthalate, diisononyl phthalate, butyl octyl phthalate, dioctyl azelate, epoxydised tallate, triisoctyl trimellitate, diethylhexyl phthalate, di-n-octyl phthalate, di-1-octyl phthalate, di-1-decyl phthalate, di-n-undecyl phthalate, di-n-tridecyl phthalate, tri-2-ethylhexyl trimellitate, di-2-ethylhexyl adipate, di-2-ethylhexyl sebacate, di-2-ethylhexyl azelate, dibutyl sebacate, glyceryl monocaprylate, glyceryl monocaprate, and mixtures thereof.

In some embodiments, the coated rifaximin comprises one or more of polymorphous forms of rifaximin, a raw form of rifaximin or a combination thereof in combination with at least one excipient.

In some of embodiments, wherein the coated rifaximin is in a multilayer composition, the plurality of layers further comprises a film coating layer over the barrier coating layer, the film coating layer comprising one or more of cellulose and its substitutes such as hydropropylcellulose hydromethylcellulose, hydropropyl-ethylcellulose.

In some embodiments, uses herein described to treat and/or prevent an NSAID enteropathy in an individual under NSAID administration comprise comprises rifaximin and in particular, coated rifaximin, and at least one of i.) at least one NSAID and ii.) at least one proton pump inhibitor and/or at least one antibiotic, for simultaneous, combined or sequential use in the use herein described. In some embodiments, the enteropathy is a condition of the intestinal tract. In some embodiments, the NSAID treatment has a duration of at least one week, at least 10 days or at least two weeks. In some embodiments, the rifaximin is in gastroresistant form.

In some embodiments, rifaximin and in particular coated rifaximin can be formulated in coated gastroresistant microgranules, in solid compositions containing gastroresistant microgranules (e.g. the extended intestinal release (EIR) rifaximin as described in US patent 8,568,782) (see also Examples 2 and 12).

In some embodiment granules, microgranules, tablets or multilayer tablets can be coated with film coating which can comprise coating agents, opacifer, stabilizer plasticizer, dye, sweetener, hydrophobic agents and taste- masking agents. In some embodiments, the rifaximin can be the commercialized product marked with trade name NORMIX®, FLONORM®, XIFAXAN®, and RIFACOL®.

In an embodiment, coated gastroresistant rifaximin is administered at a daily dosage from 20 to 3300 mg. In an embodiment, coated gastroresistant rifaximin is administered at daily dosage from 20 to 2400 mg.

More particularly, in some embodiments, coated gastroresistant rifaximin may be administered OD, BID, TID, QDS or more often at a daily dosage in a range comprising 20 mg, 40 mg, 60, mg, 80 mg, 100 mg, 200mg, 400 mg, 550 mg, 600, 800 mg, 1100mg, 1200 mg, 1600 mg, 2400 mg, 3300 mg a day.

In some embodiments coated gastroresistant rifaximin may be administered OD, BID, TID, QDS or more often at a dosage form of 20 mg, 40 mg, 60 mg, 80 mg, 100 mg, 200 mg, 400 mg, 550 mg, 600 mg, 800 mg. In some gastroresistant rifaximin embodiments, the rifaximin may be administered OD, BID, TID, QDS or more often at a daily dosage from 20 mg to 3300 mg a day.

In some embodiments the coated rifaximin and in particular gastroresistant rifaximin may be administered OD, BID, TID, QDS or more often at a daily dosage from 400 mg, 800 mg, 1200 mg, to 1600 mg or more a day.

In some embodiments the coated gastroresistant rifaximin may be administered OD, BID, TID, QDS or more often at a daily dosage from 550 mg, 1100 mg, 1650 mg, to 2200 mg or more a day within the above dosage range.

In some embodiments the coated gastroresistant rifaximin may be administered OD, BID, TID, QDS or more often at a daily dosage from 600 mg, 1200 mg, 1800 mg, 2400 mg to 3300 mg a day.

In some embodiments the coated gastroresistant rifaximin may be administered OD, BID, TID, QDS or more often at a daily dosage from 800 mg, 1600 mg, 2400 mg, to 3300 mg a day.

In some embodiments the coated gastroresistant rifaximin may be administered OD, BID, TID, or more often at a daily dosage from 1100 mg, 2200 mg, 3300 mg a day.

In some embodiments, the coated gastroresistant rifaximin can be administered in association with other antibiotics.

As used herein the term "proton-pump inhibitor" or PPI, which are used interchangeably herein, refers to any acid labile active agents possessing pharmacological activity as an inhibitor of H/K-ATPase.

A PPI can, if desired, be in the form of a free base, free acid, salt, ester, hydrate, anhydrate, amide, enantiomer, isomer, tautomer, prodrug, polymorph, derivative or the like, provided that the free base, salt, ester, hydrate, amide, enantiomer, isomer, tautomer, prodrug or any other pharmacologically suitable derivative is therapeutically active or undergoes conversion within or outside the body to a therapeutically active form.

In some embodiments of the disclosure, the PPI that can be used in the present disclosure is one or more selected from the group consisting of: lansoprazole, ilaprazole, omeprazole, tenatoprazole, rabeprazole, esomeprazole, pantoprazole, pariprazole, leminoprazole or nepaprazole or a free base, a free acid, a salt, a hydrate, an ester, an amide, an enantiomer, an isomer, a tautomer, a polymorph, a prodrug or any derivative thereof.

"Pharmaceutically acceptable salts," or "salts," of a proton pump inhibitor include, but are not limited to, the salt of a proton pump inhibitor prepared from formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic, methanesulfonic, ethanesulfonic, benzenesulfonic, pantothenic, toluenesulfonic, toluenesulfonic, 2-hydroxyethanesulfonic, sulfanilic, cyclohexyl-aminosulfonic, algenic, B-hydroxybutyric, galactaric and galacturonic acids.

Acid addition salts of proton pump inhibitors can be prepared from the free base forms using conventional a methodology, e.g., involving the reaction of a free base with a suitable acid. Suitable acids for preparing acid addition salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, e.g., hydrochloric acid, hydrobromic acid, sulfuric acid, nitric acid, phosphoric acid, and the like. An acid addition salt can be reconverted to the free base by treatment with a suitable base. Thereupon, also contemplated herein are acid addition salts of the proton pump inhibitors that are halide salt and which can be prepared using hydrochloric or hydrobromic acids. Additionally, the basic salts can be alkali metal salts, e.g., sodium salt.

Salt forms of proton pump inhibitors include, but are not limited to, a sodium salt form such as esomeprazole sodium, omeprazole sodium, rabeprazole sodium, pantoprazole sodium; or a magnesium salt form such as esomeprazole magnesium or omeprazole magnesium; a calcium salt form; or a potassium salt form such as the potassium salt of esomeprazole.

Preparation of esters of proton pump inhibitors involves functionalizing hydroxyl and/or carboxyl groups that can be present within the molecular structure of the drug. Alternatively, the esters are acyl-substituted derivatives of free alcohol groups, e.g., moieties derived from carboxylic, acids of the formula RCOOR₁ where R₁ is a lower alkyl group. A lower alkyl group may be less than 20 carbons, preferably less than 10 or 5 carbons. Esters can be reconverted to the free acids, if desired, by using conventional procedures such as hydrogenolysis or hydrolysis.

"Amides" or proton pump inhibitors can be prepared using techniques known to those skilled in the art or described in the pertinent literature. For example, amides can be prepared from esters, using suitable amine reactants, or they can be prepared from an anhydride or an acid chloride by reaction with an amine group such as ammonia or a lower alkyl amine.

"Tautomers" of substituted bicyclic aryl-imidazoles include, e.g., tautomers of omeprazole such as those. An example of an "isomer" of a substituted bicyclic aryl-imidazole is the isomer of omeprazole.

As used herein "misoprostol" is a synthetic prostaglandin E1 (PGE1) analog that is used for the prevention of NSAID induced gastric ulcers. It acts upon gastric parietal cells, inhibiting the secretion of gastric acid via G-protein coupled receptor mediated inhibition of adenylate cyclase, which leads to decreased intracellular cyclic AMP levels and decreased proton pump activity at the apical surface of the parietal cell.

In some embodiments, administering NSAID, antibiotic and proton pump inhibitor can be performed on individuals which are suffering, capable of, or at risk of suffering from a bowel disease or other disorder treatable by a rifamycin class antibiotic (e.g., rifaximin) or who could otherwise benefit from the administration of a rifamycin class antibiotic (e.g., rifaximin) as described herein, such as human and non-human animals. In particular human animals include human subjects such as "an individual", "a person" or "a patient." The term "non-human animals" of the disclosure includes all other animals including vertebrates, e.g., mammals such as rodents (e.g., mice) non-human primates, sheep, dogs, cattle, and non-mammals including birds (e.g., chickens) amphibians, reptiles.

The phrase "at risk for a bacterial infection" is meant to include a subject at risk of developing an infection or a person who is in remission from an infection or a person who can relapse, e.g., a subject suffering from immune suppression, a subject that has been exposed to a bacterial infection, physicians, nurses, a subject traveling to remote areas known to harbor bacteria that cause travelers' diarrhea, an aging person, an individual with liver damage, and additional individual identifiable by a skilled person.

In some embodiments, the NSAID and rifaximin and optionally the antibiotic and/or PXR inhibitor can be administered parenterally, enterally and preferably orally. The PPI, can be administered orally or parenterally for example orally TID or orally OD and parenterally BID. In embodiments rifaximin can be administered orally or rectally, PPI can be administered orally and NSAID can be administered parenterally.

Rifaximin is administered orally, PPI can be administered orally or parenterally and NSAID can be administered orally or parenterally or rectally.

In some embodiments, the NSAID administered is one or more of diclofenac, ketoprofen, naproxen and ibuprofen. In some embodiments, the NSAID can be formulated in granules (Example 7) and also in fast release granules (Example 5-6).

In some embodiments, the proton pump inhibitor is omeprazole. In some embodiments, the proton pump inhibitor, and in particular omeprazole can be formulated in gastroresistant granules (Examples 3 and 4)

In some embodiments, rifaximin is in the extended intestinal release rifaximin herein also indicated as EIR, which can be any of the compositions described for example in accordance with the disclosure US application publication no. 2009/011020. In some embodiments of the compositions for use herein described, EIR rifaximin is administered once a day (OD), twice a day (BID), three times a day (TID), four times a day (QDS) or more often as necessary at dosages from 20 to 3300 mg a day and in particular from 20 to 1200 mg a day and from 20 to 2400 mg a day. More preferably in some embodiments a dosage from 100, 200, 400, 550, 600, 800 up to 1100 mg, in particular from 100, 200, 400, 550, 600, 800 mg or more EIR rifaximin can be administered OD, BID, TID, QDS or, 1100 mg EIR rifaximin may be administered OD, BID, TID or more often as necessary.

In some of those embodiments an NSAID may be administered once a day (OD), twice a day (BID), three times a day (TID), four times a day (QDS) or more often as necessary at dosages comprising from 5 to 1500 mg a day, depending on the NSAID chosen.

In some those embodiments the proton pump inhibitor may be administered once a day (OD), twice a day (BID), three times a day (TID), four times a day (QDS) or more often as necessary at dosages comprising from 5 to 2000 mg a day, depending on the proton pump inhibitor chosen.

In some of those embodiments, compositions for use in treating a condition presenting pain or inflammation comprise performing a first administering of gastroresistant rifaximin (EIR rifaximin), and at least one NSAID and; performing a second administering of rifaximin, at least one NSAID and at least one PPI.

In embodiments, herein described wherein rifaximin is the administered, gastroresistant rifaximin may be administered in an amount from 20 to 1200 mg/day and NSAIDs are administered in a quantity depending on the chosen NSAID. In embodiments, when EIR rifaximin and NSAID are administered once a day, they can be administered at any time and when the administration is two times a day, morning and evening administration can be chosen.

In some embodiments uses herein described comprise administering for a period of at least one week up to two months effective amount of gastroresistant rifaximin in association with at least one NSAID, at least one PPI, wherein the administration can be done in separate dosage forms or a single unified dosage form comprising NSAIDs and gastroresistant rifaximin or a unified dosage form comprising NSAIDs, gastroresistant rifaximin and PPI.

In some embodiments, uses herein described comprise treating a subject suffering of a condition wherein inflammation and/or pain are present (e.g., arthritis or cardiovascular disease) with the administration of rifaximin and NSAIDs performed for at least one time a day.

In some embodiments, one or more proton pump inhibitors are associated with the administration of rifaximin and NSAIDs and they are administered one time a day. In an embodiment, rifaximin, NSAID and PPI are administered in the evening.

In some embodiments, active ingredients, rifaximin, NSAIDs and proton pump inhibitors can be administered in separate doses, e.g., in three separate tablets or capsules or sachets, or in one unified dosage form comprising NSAID, rifaximin and PPI. In a further embodiment, at least two active ingredients are administered in a unified dosage form and the remaining to be administered in a separate dose.

Rifaximin can be administered also in the form of tablets or granules for suspension. Gastroresistant rifaximin can be used in embodiments in which a quantitative release of rifaximin in intestine is desired.

In one embodiment the disclosure provides uses for treatment of rheumatic diseases in a subject, by administering rifaximin and at least one NSAID.

In an embodiment NSAID is selected in the group comprising diclofenac, naproxen, aspirin and ibuprofen.

In one embodiment the disclosure provides uses for treatment of a rheumatic disease in subjects, by administering rifaximin and at least one NSAID and at least one PPI.

In an embodiment PPI is selected in from group comprising omeprazole, lansoprazole, esomeprazole and pantoprazole.

One embodiment is a use comprising administering effective amounts of an NSAID selected in the group of diclofenac, naproxen, aspirin and ibuprofen with an effective amount of rifaximin.

One embodiment is a use comprising administering effective amounts of an NSAID selected in the group of diclofenac, naproxen, aspirin and ibuprofen with an effective amount of rifaximin and an effective amount of a PPI.

The PPI is preferably selected from the group of consisting of omeprazole, lansoprazole, esomeprazole and pantoprazole, and the administering of one among diclofenac, naproxen, aspirin and ibuprofen and at least one among rifaximin or omeprazole, lansoprazole, esomeprazole and pantoprazole.

In some embodiments rifaximin can be a raw rifaximin or a polymorphic rifaximin, or amorphous rifaximin or their mixture. Rifaximin is gastroresistant rifaximin, and in particular the gastroresistant rifaximin (EIR rifaximin).

In some embodiments uses described herein comprise administering diclofenac, rifaximin and omeprazole; and administering diclofenac and at least one among rifaximin or omeprazole.

Further embodiments of the disclosure are uses comprising administering diclofenac, rifaximin and omeprazole; and administering diclofenac and at least rifaximin, wherein rifaximin is gastroresistant rifaximin, and in particular rifaximin in gastroresistant granules as described in US 8,568,782 (see also Example 2) and any additional form of gastroresistant rifaximin (e.g. EIR rifaximin).

In some embodiments the uses herein described comprise administering NSAID, at least one antibiotic and at least one proton pump inhibitor; and the administering of one NSAID and at least one among antibiotic and PPI to a subject suffering from all grades of pain and inflammation in a wide range of conditions, including: (i) arthritic conditions: rheumatoid arthritis, osteoarthritis, ankylosing spondylitis, acute gout, (ii) acute musculo-skeletal disorders such as periarthritis (for example frozen shoulder), tendinitis, tenosynovitis, bursitis, (iii) other painful conditions resulting from trauma, including fracture, low back pain, sprains, strains, dislocations, orthopedic, dental and other minor surgery, any cardiovascular disease, neuropathic pain (diabetic neuropathic pain, trigeminal neuralgia, transverse myelitis, sciatica) chronic myofascial pain, muscle pain wherein the subject is in needed of a long-term therapy, e.g., of at least one week. Additional conditions comprise various cardiovascular diseases wherein an NSAID such as acetylsalicylic acid is administered for treatment or prevention of the disease for a long-term therapy and in particular for a treatment period of at least two weeks (e.g. platelet aggregation, unstable angina pectoris, suspected or diagnosed acute myocardial infarction, prophylaxis of repeated myocardial infarction, condition after vascular surgery (e.g. PTCA, CABG), prophylaxis of transient ischemic attacks and stroke in the period of initial symptoms, prophylaxis of coronary thrombosis in patients with multiple risk factors). Uses described herein are particularly useful for preventing or treating enteropathy associated with NSAID administration wherein the NSAID is administered for a length of time for treating disease which causes the specific enteropathy. Uses herein described are useful for treatment or prevention of enteropathy typically associated with a long term NSAID administration.

In a particular embodiment, wherein treatment or prevention of intestinal damage is desired, uses herein described can comprise administering of EIR rifaximin in combination with the NSAID and the proton pump inhibitor.

In some embodiments the uses herein described comprise administering NSAID, rifaximin at least one PXR agonist and at least one proton pump inhibitor; and the administering of one NSAID, rifaximin and at least one among PXR agonist and PPI to a subject suffering from all grades of pain and inflammation in a wide range of conditions, including: (i) arthritic conditions: rheumatoid arthritis, osteoarthritis, ankylosing spondylitis, acute gout, (ii) acute musculo-skeletal disorders such as periarthritis (for example frozen shoulder), tendinitis, tenosynovitis, bursitis, (iii) other painful conditions resulting from trauma, including fracture, low back pain, sprains, strains, dislocations, orthopedic, dental and other minor surgery, neuropathic pain (diabetic neuropathic pain, trigeminal neuralgia, transverse myelitis, sciatica) chronic myofascial pain, muscle pain wherein the subject is in needed of a long-term therapy, e.g., of at least one week. Additional conditions comprise various cardiovascular diseases wherein an NSAID such as acetylsalicylic acid is administered for treatment or prevention of the disease for a long-term therapy and in particular for a treatment period of at least two weeks (e.g. platelet aggregation, unstable angina pectoris, suspected or diagnosed acute myocardial infarction, prophylaxis of repeated myocardial infarction, condition after vascular surgery (e.g. PTCA, CABG), prophylaxis of transient ischemic attacks and stroke in the period of initial symptoms, prophylaxis of coronary thrombosis in patients with multiple risk factors.

In particular, in some embodiments of of the uses for treating arthritis or another condition presenting pain and/or inflammation comprise administering diclofenac at daily dosages from 5 to 300 mg; or naproxen at daily dosages from 100 mg to 1000 mg, or ibuprofen at daily dosages from 200 to 2400 mg, or aspirin at daily dosages from 25 to 3000 mg, with rifaximin in a daily dosages from 20 to 3300 mg or from 20 to 2400 mg; and omeprazole in a daily dosage from 5 to 100 mg, lansoprazole at daily dosages from 5 to 100 mg or esomeprazole at daily dosages from 5 to 100 mg, or pantoprazole at daily dosages from 5 to 100 mg for a period of time from at least one week to one month, two months or for all period wherein treatment with NSAID is required. The dosage per day maybe the same or different between any two days treatment is administered.

In particular, in some of these embodiments uses for treating rheumatic diseases, or another condition presenting pain and/or inflammation comprise administering: diclofenac in a daily dosage from 5 to 200 mg; rifaximin in a daily dosage from 20 to 3300 mg, optionally from 20 to 2000; and omeprazole in a daily dosage from 5 to 100 mg for a period of time from at least one week to one months, two months or for all period wherein treatment with NSAID is required. In particular, in some of these embodiments uses for treating rheumatic diseases or another condition presenting pain and/or inflammation can comprise administering in an amount selected from 100, 200, 400, 550, 600, 800 and 1100 mg of rifaximin, and more particularly, 200, 400, 550, 600, 800 mg, once a day, twice a day, three times a day or four times a day and 1100 mg rifaximin once a day, twice a day, three times a day or more often as necessary. In some of those embodiments the rifaximin and diclofenac or other NSAID can be administered concurrently combined in a single dosage form or at the same or different time in separate dosage forms. The dosage per day maybe the same or different between any two days treatment is administered.

In a particular embodiment, rifaximin, and in particular EIR rifaximin, diclofenac and omeprazole are cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, e.g., the cycle in order to reduce the development of resistance to one of the therapies, to avoid or reduce the side effects of one of the therapies, and/or to improve the efficacy of the therapies.

In certain embodiments, the administration of the same compounds may be repeated and the administrations may be separated by at least about 1 week, 2 weeks, 3 weeks, 4 weeks, 5 weeks, 6 weeks, 8 weeks, 10 weeks, 12 weeks, 14 weeks, 16 weeks, 18 weeks, 20 weeks, 22 weeks, or at least 24 weeks. In particular, in some of these embodiments methods of treating arthritis or another condition presenting pain and/or inflammation and/or cardiovascular disease comprise administering diclofenac twice a day, omeprazole once a day, and rifaximin twice a day for a period of time from one week to two months.

In particular, in some of these embodiments of uses for treating rheumatic diseases or another condition presenting pain and/or inflammation, and in particular cardiovascular disease comprise rifaximin in a daily dosage from 20 to 3300 mg simultaneously or before administering: NSAID and proton pump inhibitor, wherein rifaximin is administered for a period corresponding to the period of time of NSAID treatment and also for a time from 1 to 10 days after the end of NSAID treatment. In an embodiment the composition comprises rifaximin in a gastroresistant composition (e.g. EIR rifaximin), NSAID is diclofenac and proton pump inhibitor is omeprazole. In a particular embodiment, diclofenac can be administered in a daily dosage from 5 to 200 mg; omeprazole in a daily dosage from 5 to 100 mg; and rifaximin in a daily dosage from 20 to 2000 mg for a period of time from one week to two months.

In an embodiment is the use of composition comprising gastroresistant rifaximin in a solid composition comprising an amount of rifaximin from 20 to 1200 mg and diclofenac in an amount from 5 to 75 mg in form of tablets, capsules or microgranules in sachets.

In another embodiment is the use of composition comprising gastroresistant rifaximin in a solid composition comprising an amount of rifaximin from 20 to 1200 mg, diclofenac in an amount from 5 to 75 mg and omeprazole in an amount from 5 to 25 mg in form of tablets, capsules or microgranules in sachets. Another embodiment is a use for treating rheumatoid arthritis, spondiloarthritis, osteoarthritis, gout or other conditions presenting pain and/or inflammation, such as cardiovascular disease, which can comprise in some embodiments administering 1 dosage of NSAID diclofenac 75 mg twice a day (every 12 hours); 1 dosage of omeprazole 20 mg once a day (i.e., 24 hours); and 2 dosage of rifaximin 400 mg twice a day (every 12 hours) for a period of time from one week up to two months.

Alternatively, the dosages of the drug combinations stated to be administered in the morning and the evening may be administered twice daily, about 12 hours apart from each other.

In another embodiment is the use of composition comprising rifaximin and NSAID in a unique composition for treating rheumatoid arthritis, spondiloarthritis, osteoarthritis, gout or other conditions presenting pain and/or inflammation and/or cardiovascular disease. In some of those embodiments gastroresistant rifaximin is in a gastroresistant composition as EIR rifaximin.

In another embodiment gastroresistant rifaximin is administered at a dosage from 20 to 400 mg twice a day (every 12 hours), or three time a day (every 8 hours) with a dosage of NSAID diclofenac 75 mg twice a day (every 12 hours) or three times a day (every 8 hours), wherein the rifaximin and diclofenac can be administered in a unique solid form or in a separate forms together with an amount of omeprazole of 20 mg once a day (i.e., 24 hours). Another embodiments is the composition for the claimed use comprising rifaximin or gastroresistant rifaximin in association with a dosage of aspirins and a proton pump inhibitor, wherein rifaximin is administered for all the time of the aspirins administration.

Another embodiment is a use comprising administering 1 dosage of diclofenac from 5 to 75 mg, 1 dosage of omeprazole from 5 to 20 mg and 2 dosage of gastroresistant rifaximin from 20 to 600 mg (e.g. 400 mg) in the morning wherein rifaximin and diclofenac can be administered together in a single sachet, tablet or capsules or in separate forms; and 1 dosage of diclofenac from 5 to 75 mg and 2 dosages of gastroresistant rifaximin from 20 to 600 mg (e.g. 400 mg) wherein rifaximin and diclofenac can be administered together in a single sachet, tablet or capsules or in separate forms in the evening for a period of time from one week to two months.

A particular embodiment comprising administering 1 dosage of diclofenac 75 mg, 1 dosage of omeprazole 20 mg and 2 dosage of rifaximin 400 mg in the morning, wherein rifaximin omeprazole and diclofenac are administered in a single sachet, tablet or capsule; 1 dosage of diclofenac 75 mg and 2 dosages of rifaximin 400 mg wherein rifaximin and diclofenac can be administered together in a single sachet, tablet, capsule or in a separate forms in the evening, for a period of time from one week to two months.

In a particular embodiment the composition comprising rifaximin in gastroresistant granules, omeprazole in gastroresistant granules and diclofenac in granules, wherein diclofenac can be at controlled release, as fast release.

The administration of rifaximin NSAID and PPI can be administered in association with other concomitant therapies.

In an another embodiment pharmaceutical composition comprising gastroresistant rifaximin at dosage from 20 to 600 mg, diclofenac from 5 to 75 mg and omeprazole can be used in connection with treatment of pathologies wherein the prolonged administration of NSAID is required.

In one embodiment gastroresistant rifaximin can be useful in the treatment of enteropathies caused by NSAID administration. The rifaximin and gastroresistant rifaximin prevent lesion, ulceration and reduce the bleeding which cause emoglobine decrease and anaemia.

The efficacy of rifaximin (referential) or gastroresistant rifaximin (invention) administration in individuals undergoing NSAID administration and in particularly wherein the therapy with NSAID is concomitant with the PPI therapy has been demonstrated in preclinical study in animals (Examples 16 and 17) and clinical study in humans (see Examples 18 and 19).

In particular, the results of preclinical study in animals shown in Examples 16 and 17 demonstrate the efficacy of rifaximin (referential) and gastroresistant rifaximin (invention) in enteropathy induced by NSAID administration

The results expressed as nanograms of MPO per milligram of intestinal tissue and the levels of MPO in animals which received rifaximin polymorph α were reduced in a percentage of about 22%in respect to the animals which nor received rifaximin (referential) and in animal which received gastroresistant rifaximin (invention) administration reduced MPO in a percentage of about 50%.

Rats treated with only indomethacin displayed a percentage of 40% mortality rate, while the rats which received rifaximin (referential) or gastroresistant rifaximin (invention) do not displayed death.

Hemoglobin analysis was performed on rat blood samples collected at the end of the treatment period (14 days), and the resulting data have showed that the rats which received only indomethacin showed a reduction in hemoglobin values of about 20%; the rats which received rifaximin (referential) showed a deduction of hemoglobin of 12%, while the rats which received gastroresistant rifaximin (invention) do not change the hemoglobin values in respect to the control.

Also Figures 2 and 3 demonstrate that the microscopic assessment of intestinal damage are reported when rifaximin (referential) and gastroresistant rifaximin (invention) is administrated with NSAID. In particular gastroresistant rifaximin significantly reduced type 2 lesions, in comparison with indomethacin alone.

The disclosure demonstrates that treatment with gastroresistant rifaximin 25 mg/kg, gastroresistant rifaximin 50 mg/kg or rifaximin polymorph alpha 50 mg/kg BID are effective in the reduction of lesions in ileum e in jejunum, in particular in lesion of type 1 and 2.

The above data indicate that Rifaximin is more effective in the enteropathy caused by NSAID administration, in particular embodiment of gastroresistant rifaximin(EIR rifaximin) is more efficacious than rifaximin not gastroresistant in the treatment or prevention of NSAID intestinal damage.

The effective doses of rifaximin used in the animal model (16 mg/kg) in terms of human equivalent doses are less than the dose used in the clinical trial (27 mg/kg) at demonstration that similar results can be obtained in humans in terms of histological results in ileum and jejunum and hemoglobin levels and MPO results, as measure of damage and inflammation and/or cardiovascular disease.

Accordingly, the above data in rats show efficacy of rifaximin in particular in connection with severe enteropathies which in the study described in Examples 16 and 17 are associated with detection of type 3 lesions in rats, with a detection of MPO in an amount equal to or higher than 20 ng per mg of tissue in rats, and/or detection of HBO in an amount equal to or lower than 13.5 g/dl.

Efficacy of rifaximin and in particular gastroresistant rifaximin (invention) has been shown in the clinical trial of Examples 19 and 20. According to the procedure reported therein, in a phase 2b, double blind study, randomised, healthy volunteers received diclofenac SR 75 mg BID plus omeprazole 20 mg once a day with or without rifaximin 800 mg BID for 14 days and the intestinal analyzed by the use of multicenter video capsule endoscopy (VCE).

This study had a duration of approximately 5 weeks and consisted of Screening period which included a screening visit for potential subjects, to perform clinical investigations and laboratory tests, followed by a baseline VCE at visit 2.

The final visit was performed within 36 hours after the last drug administration and including VCE, clinical assessments and laboratory test. The primary efficacy endpoint criteria was the percentage of subjects developing at least one mucosal break at final visit evaluated by VCE and assessed according to a validated scoring system reported from 0 to 4.

The secondary efficacy endpoints were the change from baseline to final visit in the number of mucosal lesions in the small bowel and the change from baseline to final visit in the number of mucosal lesions in the small bowel with/without hemorrhage (visible blood).

The safety parameters considered were adverse events (AEs), clinical laboratory parameters such as hematology, clinical chemistry and urinalysis, and vital signs. At the end of 2-week treatment with diclofenac plus omeprazole, the rate of subjects who developed at least one mucosal lesion in the small bowel was double in the placebo group, 13 subjects corresponding to 43.3% as compared to 6 subjects of the rifaximin group corresponding to a 20%. In particular, the proportion of subjects developing the lesions as compared to the proportion of subjects not developing the lesions was much lower in the rifaximin group 20% vs. 80% than in the placebo group 43.3% vs. 56.7%, strongly suggesting a protective action of rifaximin on mucosal damage caused by diclofenac during the 14 -day treatment.

The change from baseline in the total number of lesions was higher in the placebo group than in the rifaximin group. All the mucosal lesions detected at final assessment were lesions without haemorrhage. No lesions with haemorrhage were observed (Table 30). Results of the statistical analysis by negative binomial regression clearly indicated a protective effect of rifaximin on mean changes from baseline in total number of lesions and lesions without haemorrhage. The effect was statistically significant for total lesions and lesions without haemorrhage, respectively).

At the end of the 2-week treatment at the Final visit, large erosions/ulcers (category 4) were only detected in the placebo group in the subjects corresponding to a value higher than 20%. No large erosions/lesions were observed in the gastroresistant rifaximin group.

In the placebo group erosions can be categorized as ulcers.

By the clinical study, a protective effect of rifaximin on diclofenac-induced mucosal lesions is clearly observed. Primary efficacy results showed that fewer subjects in the rifaximin group than in the placebo group developed at least one mucosal lesion in the small bowel during the study. Results of the secondary analysis on the changes from baseline, on the other hand, clearly showed a statistically significant difference in total number of lesions and lesions without hemorrhage, thus confirming a protective action of rifaximin on diclofenac-induced gastrointestinal damage.

In particular, in some embodiments, the use of rifaximin or gastroresistant rifaximin (invention) is useful to treat enteropathies exacerbated by the PPI concomitant therapy with NSAID.

When rifaximin or gastroresistant rifaximin (invention) are administered in concomitant therapy with NSAID and/or NSAID plus PPI, with respect to placebo without rifaximin intestinal lesions were reduced such as bleeding, anaemia. The administration of rifaximin in a concomitant NSAID therapy reduce the inflammation status. The inflammation is also reduced when PPI are NSAID concomitant administered. (see e.g. Examples 16 to 19).

In one embodiment the compositions for the use of the present disclosure are useful for the prevention of mucosal breaks in individuals under chronic NSAID administration. The compositions of the present disclosure is useful for the treatment of mucosal breaks in subjects under chronic NSAID administration. In one embodiment the mucosal breaks are petichiae spot. In another embodiment the erosions are comprised between 1 and 4. In another embodiment the erosions are higher than 4.

A further embodiment is a use comprising administering 1 tablets of diclofenac 75 mg, 1 capsule of omeprazole 20 mg and 2 tablets of gastroresistant rifaximin 400 mg in the morning and 1 tablet of diclofenac 75 mg and 2 tablets of gastroresistant rifaximin 400 mg in the evening.

Another embodiments is a use for treating cardiovascular disease comprise administering 1 dosage of aspirins; 1 dosage of proton pump inhibitor and rifaximin which in some embodiments can comprise EIR rifaximin, for the entire life of the individual. In some embodiments the aspirin dosage can be within the range of 30-325 mg /day.

In certain embodiments, one or more of the rifaximin (which can be in some embodiments EIR rifaximin), NSAID and PPI are cyclically administered. Cycling therapy involves the administration for a first therapy for a period of time, followed by the administration of a second therapy for a period of time, optionally, followed by the administration of a third therapy for a period of time and repeating this sequential administration, i.e., the cycle in order to reduce the arthritis.

The disclosure relates also to a pharmaceutical compositions for the claimed use comprising antibiotic, NSAID and PPI with a pharmaceutically acceptable vehicle. In particular, a composition including the antibiotic, NSAID and PPI can be prepared in thermo welded bags (Example 8 and 11), tablets (Example 9, 13 and 14), and capsules (Examples 10 and 15).

The disclosure relates also to a pharmaceutical compositions for the claimed use comprising antibiotic, NSAID with a pharmaceutically acceptable vehicle For example, gastroresistant tablets can be made of a composition of comprising the NSAID and antibiotic (see Example 14, wherein rifaximin and sodium salt diclofenac are described).The disclosure relates also to a pharmaceutical compositions comprising a PXR agonist, NSAID and PPI with a pharmaceutically acceptable vehicle.

In some embodiments, the pharmaceutical composition can comprise at least one antibiotic and/or PXR agonist and at least one proton pump inhibitor in a pharmaceutically effective amount to treat and/or prevent an NSAID enteropathy according to uses herein described. In some embodiments, the enteropathy is a condition of the intestinal tract. In some embodiments, the pharmaceutical composition is formulated for oral administration.

In some variants, the pharmaceutical composition can comprise a pharmaceutically effective amount of i) at least one NSAID and of ii) at least one antibiotic and/or PXR agonist to treat and/or prevent a condition wherein pain and/or inflammation are present in an individual while treating and/or preventing enteropathy in the individual. In some embodiments, the pharmaceutical composition is formulated for oral administration.

In some embodiments, the pharmaceutical composition can comprise a pharmaceutically effective amount of at least one NSAID, at least one antibiotic and/or PXR agonist and at least one proton pump inhibitor in an effective amount to treat and/or prevent a condition wherein pain and/or inflammation while treating and/or preventing an enteropathy and in particular a severe enteropathy in the individual. In some embodiments, the pharmaceutical composition is formulated for oral administration.

In some embodiments, the pharmaceutical composition for the claimed use can comprise a pharmaceutically effective amount of at least one of i) at least one NSAID and ii) at least one proton pump inhibitor in an effective amount to treat and/or prevent a condition wherein pain and/or inflammation are present while treating and/or preventing an enteropathy and in particular a severe enteropathy in the individual. The rifaximin is in a gastroresistant form

In some embodiments, the pharmaceutical composition for the claimed use can comprise a pharmaceutically effective amount of EIR rifaximin and a pharmaceutically effective amount of at least one of i) at least one NSAID and ii) at least one proton pump inhibitor in form of granules or microgranules for aqueous suspensions or multilayer solid compositions in an effective amount to treat and/or prevent a condition wherein pain and/or inflammation. As used herein, the term "pharmaceutically acceptable" includes moieties or compounds that are, within the scope of sound medical judgment, suitable for use in contact with the tissues of humans and lower animals without undue toxicity, irritation, allergic response, and the like, and are commensurate with a reasonable benefit/risk ratio.

The pharmaceutical preparations can be given by forms suitable for each administration route. For example, these preparations are administered in tablets or capsule form, by injection, inhalation, eye lotion, eye drops, ointment, suppository, and additional forms identifiable by a skilled person, administration by injection, infusion or inhalation; topical by lotion or ointment; and rectal by suppositories. The injection can be bolus or can be continuous infusion. Depending on the route of administration, a GI specific antibiotic can be coated with or disposed in a selected material to protect it from natural conditions that can detrimentally affect its ability to perform its intended function. A GI specific antibiotic can be administered alone, or in conjunction with either another agent or agents as described above or with a pharmaceutically-acceptable carrier, or both. A GI specific antibiotic can be administered prior to the administration of the other agent, simultaneously with the agent, or after the administration of the agent. Furthermore, a GI specific antibiotic can also be administered in a prodrug which is converted into its active metabolite, or more active metabolite *in vivo.*

In some embodiments, pharmaceutical compositions are described that comprise one or more antibiotics, and one or more NSAIDs. The compositions can further comprise one or more proton pump inhibitors.

In some embodiments, pharmaceutical compositions are described that comprise one or more PXR agonists and one or more NSAIDs. The compositions can further comprise one or more proton pump inhibitors.

The compositions can be as a single pharmaceutical formulation or dosage form, also herein called multi dosage compositions. These compositions are prepared so as to avoid the circumstance in which the active ingredients interact each other, thereby avoiding degradation.

The compositions according to the disclosure may be an oral composition, and in particular, oral compositions releasing the active ingredient in the intestine are also included in the scope of the present disclosure. In one embodiment the active ingredient of the disclosure are formulated as multi-layer tablets, effervescent tablets, powder, pellets, granules, hard and soft gelatin capsules comprising multiple beads, capsules within a capsule (in which rifaximin, NSAID sand PPI are physically separated). Liquid dosage forms such as solutions, emulsions, foams and suspension are also suitable for use with the presently disclosed compositions, methods and systems.

According to one embodiment of the present disclosure, the solid dosage form comprises rifaximin in a dosage from 20 to 1200 mg, and one or more NSAID.

In one embodiment of the present disclosure, the solid dosage form comprises rifaximin in a dosage from 20 to 1200 mg, one or more NSAID and a PPI. Rifaximin is gastroresistant rifaximin. In an embodiment of the present disclosure, the solid dosage form comprises rifaximin in a dosage from 20 to 1200 mg, and the NSAID is selected from the group consisting of diclofenac, naproxen, ibuprofen and aspirin. In some embodiments, the NSAID is diclofenac.

In some embodiments, the solid dosage form comprises rifaximin in a dosage from 20 to 1200 mg, an NSAID and a PPI selected from the group consisting of omeprazole, pantoprazole, lansoprazole and esomeprazole. In some embodiments PPI is omeprazole.

In some embodiments, the solid composition comprises 400 mg rifaximin, 75 mg of diclofenac. In some embodiments, the solid composition comprises 400 mg rifaximin, 75 mg of diclofenac and 20 mg of omeprazole.

In some embodiments, the solid composition is in thermo-welded bags.

In several embodiments the solid composition may comprise pharmaceutically acceptable excipients as disintegrants, e.g., such as sodium carboxymethylcellulose (carmellose sodium), cross-linked sodium carboxymethylcellulose (o croscarmelose sodium), polyvinylpyrrolidone (povidone), cross-linked polyvinylpolypyrrolidone, (crospovidone), starch, pre-gelatinized starch, and silica; lubricants e.g., such as magnesium or calcium stearate, sodium stearyl fumarate, vegetable hydrogenated oils, mineral oils, polyethylene glycols, sodium lauryl sulfate, glycerides, sodium benzoate talc, calcium stearate, magnesium stearate, solid polyethylene glycols, sodium lauryl sulfate and mixtures thereof; diluents, e.g., such as cellulose, microcrystalline cellulose, calcium phosphate, starch, kaolin, di-hydrated calcium sulfate, calcium carbonate, lactose, saccharose, glucose, sorbitol and mannitol; binders such as cellulose, cellulose derivatives, starches, potatoes starch, corn starch, gums, synthetic gum, carboxymethyl cellulose, polyvinylpyrrolidone, sodium carboxymethyl cellulose, cellulose microcrystalline, hydroxypropylcellulose, hydroxyethylcellulose, hydroxypropyl methylcellulose, ethylcellulose, polyethylene glycol, gelatin, polypropylene glycol, alginic acid, alginate salts, sugars; glidants such as colloidal silicon dioxide, tal; fluidizer e.g, such as silica or fumed silica. Colorants, opacifing agents, flavoring agents, anti-oxidants, and sweeteners may also optionally be added to the formulations.

Suspensions, in addition to the active compounds (e.g., at least one antibiotic, at least one PXR agonist, at least one NSAID, at least one PPI or any combination thereof), may contain suspending agents, for example, ethoxylated isostearyl alcohols, polyoxyethylene sorbitol and sorbitan esters, microcrystalline cellulose, aluminum metal hydroxides, bentonite, agar-agar and tragacanth, or mixtures of these substances, and the like.

Described are the processes for obtaining the composition comprising rifaximin or gastroresistant rifaximin, NSAID and optionally gastric inhibitor agent.

Another variant are the process for obtaining composition comprising effective dosage of rifaximin or gastroresistant rifaximin, effective dosage of NSAID, in particular diclofenac in form of tablets or thermo- welded bags.

Another variant are the process for obtaining composition comprising effective dosage of rifaximin or rifaximin gastroresistant, effective dosage of NSAID, in particular diclofenac and gastro inhibitor agent, in particular omeprazole in form or thermo-welded bags, wherein the agent are in granule or microgranule or gastroresistant granule or microgranule.

Rifaximin, diclofenac and omeprazole can be formulated in microgranules, granules and/or gastroresistant granules or microgranules wherein the active ingredient is together with pharmaceutical acceptable excipients. The gastroresistant microgranules of rifaximin can be prepared according to the US patent 8,568,782.

Omeprazole can be formulated in gastroresistant granules wherein the active ingredient is loaded on a core of inert excipient and then is coated with a gastroresistant material. The gastroresistant granules of omeprazole can comprise an amount of omeprazole between 1% and 20% (w/w) together but not limited with a diluent, a binder, a glidant and a fluidizer.

Diclofenac can be formulated in fast release granules comprising the diclofenac together with excipients wherein the amount of diclofenac granules are comprised between 50% and 90% (w/w).

In one embodiment the composition for the claimed use is in form of thermo-welded bags wherein the gastroresistant microgranules of rifaximin, wherein rifaximin is in an amount from 20 mg to 800 mg in association with the diclofenac fast release granules, wherein diclofenac is in an amount from 5 to 75 mg and optionally omeprazole gastroresistant granules, wherein omeprazole is in amount from 5 to 20 mg together with pharmaceutical acceptable excipients.

In one embodiment the composition for the claimed use is in form of thermo-welded bags wherein the gastroresistant microgranules of rifaximin, wherein rifaximin is in an amount from 10% to 30% in association with the diclofenac fast release granules in an amount from 54% to 60% (w/w), wherein and optionally omeprazole gastroresistant granules in an amount from 5% to 12% (w/w) mg together with pharmaceutical acceptable excipients.

In another embodiment the composition for the claimed use is in form of thermo-welded bags wherein rifaximin in amount from 20 to 1200 mg is in form of gastroresistant microgranules in association with the diclofenac in an amount from 5 to 75 mg and optionally omeprazole gastroresistant granules wherein omeprazole is in amount from 5 to 20 mg together with pharmaceutical acceptable excipients.

In another embodiment the composition for the claimed use is in form of thermo-welded bags wherein the rifaximin is in gastroresistant granules is in amount from 20 to 800 mg in association with the diclofenac powder in an amount from 5 to 75 mg and optionally omeprazole gastroresistant granules from 5 to 20 mg together with pharmaceutical acceptable excipients.

In one embodiment the composition for the claimed use is in form of tablets wherein the rifaximin granules in an amount from 50% to 60% (w/w) are in association the diclofenac powder in an amount from 2.5% to 5% (w/w) and optionally omeprazole powder in an amount from 0.1% to 2% (w/w) together with pharmaceutical acceptable excipients wherein the resultant tablets are coated with gastroresistant material.

In one embodiment the composition for the claimed use is in form of tablets wherein the rifaximin granules in an amount from 50% to 60% (w/w) are in association the diclofenac powder in an amount from 2.5 to 5% (w/w) and optionally omeprazole powder in an amount from 0.1% to 2% (w/w). together with pharmaceutical acceptable excipients comprising a disintegrant in amount from 3 to 6%, a lubricant is an amount from 0.5% to 1.5% (w/w) a binder in an amount from 1.5% to 3% (w/w) and a diluents in an amount from 45% to 60% (w/w), wherein the resultant tablets are coated with gastroresistant material in an amount from 5% to 7% (w/w).

In some embodiments, tablets can be formed by a core of diclofenac with a layer of rifaximin and the resulting tablets can be coated with a gastroresistant layer.

In some embodiments, tablet can be formed by a layer of diclofenac and a layer of rifaximin. In one embodiment the composition is in form of capsules wherein the rifaximin powder in an amount from 50% to 65% (w/w) is in association with diclofenac powder in an amount from 6% to 12% (w/w) and optionally gastroresistant granules of omeprazole in an amount from 15% to 25% (w/w) together with pharmaceutical acceptable excipients, in respect to the weight of the granules.

In another embodiment the composition for the claimed use is in form of capsules wherein the rifaximin gastroresistant microgranules in an amount from 60% to 75% (w/w) are in association with diclofenac powder in an amount from 6% to 12% (w/w) and optionally gastroresistant granules of omeprazole in an amount from 15% to 25% (w/w) together with pharmaceutical acceptable excipients.

In one embodiment rifaximin gastroresistant microgranules are prepared as described in Example 2. The coating of the microgranules comprises polymeric materials which are preferably insoluble at pH values between about 1.5 and 4.0 and which are soluble at pH values between 5.0 and 7.5. In general, the gastroresistant coating is made of any material which is insoluble in a pH range between 1.5 and 4.0 and which is soluble at higher pH values, preferably at pH values between 5.0 and 7.5. Examples of particular polymers which are suitable for use with methods systems and compositions herein described are chosen among the copolymers of acrylic acid, such as the methacrylic acid-ethyl acrylate copolymer 1:1 and the methacrylic acid-methyl methacrylate copolymer 1:2, polyvinyl acetate phtalate, hydroxypropylmethylcellulose phtalate, cellulose acetate phtalate, commercially available, for instance, with the trademarks Kollicoat®, Eudragit®, Aquateric®, Aqoat®.

The amount of gastroresistant material used to make the microgranules for use with the use and compositions for use herein described is about between 10% and 60% (w/w), preferably between 20% and 40% (w/w), if compared to the total weight of the gastroresistant granule. The gastroresistant coating which is applied on the active principle in microgranules can optionally also contain plasticizers, diluents, antiadherents, antiagglomerants, glidants, defoamers, colorants and antioxidants.

The components which are used for coating microgranules, can be solubilized using organic solvents or kept in an aqueous suspension. The solutions or suspensions of coating material are applied by nebulization on powders or granules or microgranules which are kept moving inside a coating pan or in air suspension in fluid bed apparatuses during the application process.

Non-limiting examples of organic solvents that may be used to solubilize the coating material are methylene chloride, methyl acid, isopropyl alcohol, triethyl acetate and ethyl alcohol.

As an alternative, the gastroresistant polymeric material can be applied through aqueous suspensions, which is the preferable technique because it does not require the use of solvents with the relating toxicological and safety concerns.

The gastroresistant microgranules can also be prepared with other processes known to those in the pharmaceutical arts. Such techniques may include, for example, granulating the active principle rifaximin together with diluents, glidants and ligands, and by submitting the dried and sieved microgranules to the successive coating process with a gastroresistant coating.

Another system which can be used for the preparation of the microgranules involves the application of rifaximin by means of a ligand compounds selected from the group consisting of cellulose, cellulose derivatives, starches, potatoes starch, corn starch, gums, synthetic gum, polyvinylpyrrolidone, sodium carboxymethylcellulose, cellulose microcrystalline, hydroxypropylcellulose, hydroxyethylcellulose, hydroxypropyl methylcellulose, ethylcellulose, polyethylene glycol, gelatin, polypropylene glycol, alginic acid, alginate salts, sugars, and combinations thereof to microcrystalline cellulose granules having a diameter between about 100 and 200 microns and submitting the resulting microgranules to the successive coating process with the gastroresistant film.

Omeprazole gastroresistant granule comprise omeprazole in an amount from 5% to 20% (w/w), fluidizer in an amount from 0.01% to 1% (w/w), glidant in an amount from 1% to 10% (w/w), binder in an amount 0.5%-5% (w/w) on an inert core in an amount from 64% to 97.5% (w/w) in respect to the weight of the granule. The omeprazole granule are coated with a gastroresistant coating in an amount from 20% to 50% in respect to the weight of the granule. In a particular embodiment binder is hydroxypropyl methyl cellulose, fluidizer is fumed silica, glidant is talc, inert core are microcrystalline cores. Gastroresistant polymer useful for preparing omepraziole granule are chosen among the copolymers of acrylic acid, such as the methacrylic acid-ethyl acrylate copolymer 1:1 and the methacrylic acid-methyl methacrylate copolymer 1:2, polyvinyl acetate phtalate, hydroxypropylmethylcellulose phtalate, cellulose acetate phtalate, commercially available, for instance, with the trademarks Kollicoat®, Eudragit®, Aquateric®, Aqoat® and natular polymers.

NSAID such as diclofenac can be in form of granule or fast release granules. Diclofenac fast release granules can comprise 25 mg or 75 mf of diclofenac corresponding to a percentage amount from 1% to 5% (w/w), disgregants in an amount from 0.5 to 5% (w/w) and diluents in an amount form 90% to 98.5% (w/w) in respect to the weight of the granules. Diclofenac granules can comprise diclofenac in an amount from 10% to 80% (w/w), diluents in an amount from 10% to 70% (w/w), disgregants from 1% to 10% (w/w), in respect to the total weight of the granule. Pharmaceutical composition comprising rifaximin in gastroresistant granule, diclofenac granule or diclofenac in fast release granules and optionally omeprazole gastroresistant granules can be in form of thermo welded bags with pharmaceutically acceptable excipients.

In a particular embodiment of the claimed use, thermowelded bags comprise gastroresistant rifaximin (EIR rifaximin), wherein rifaximin is in an amount of 400 mg and diclofenac in fast release granules is in an amount of 75 mg. Thermowelded bags can also comprise sweetening, flavouring, preservatives and antioxidant agent. Thermowelded bags can be suspended in water.

Solid composition can be in form of tablets, wherein said tablet can be in form of multilayer tablets can comprise rifaximin granules or rifaximin gastroresistant granules, diclofenac granules or diclofenac fast release granules, optionally omeprazole in gastroresistant granules and pharmaceutically excipients, and said tablets can be coated with film coated or gastroresistant coating. In particular embodiment tablets can comprise rifaximin in an amount from 20 to 600 mg and diclofenac from 5 mg to 75 mg.

Solid composition can be in form of gelatin capsules, wherein said capsules comprise rifaximin, diclofenac granules or diclofenac fast release granules and pharmaceutically acceptable excipients. Said capsules can comprise rifaximin in an amount from 20 to 600 mg and diclofenac from 5 mg to 75 mg.

According to another variant the processes for preparing omeprazole gastroresistant granules comprises a step wherein an aqueous solution of binder in an amount from 5% to 25% (w/w) is added to a an aqueous suspension comprising an amount of omeprazole from 10% to 40% (w/w), a binder from 2% to 50% (w/w) and a glidant from 5% to 20% (w/w). The resultant mixture on inert core with flow air 10-60 m³/h is sprayed in a fluid bed at temperature from 30°C to 80°C. The omeprazole granules dried are successively coated with an aqueous suspension of HPMC in an amount from 10% to 30% (w/w) in a fluid bed apparatus with an air flow from 30 to 70 m³/h at temperature from 50°C to 80°C. The dried granules were successfully coated with a gastroresistant polymer in the same apparatus and the gastroresistant granules dried. The yield of the process is higher than 85%.

According to another variant is the process for obtaining diclofenac fast release granules is described, wherein an aqueous solution comprising diclofenac from 10% to 50% (w/v), disgregants from 5% to 10% (w/v) and diluents from 40% to 85% (w/v) is sprayed in a fluid bed apparatus and the granule dried until constant weight.

According to another variant the process for obtaining diclofenac granules is described, wherein diclofenac powder from 10% to 80% (w/w), diluents in an amount from 10% to 70%, disgregants from 5% to 10% (w/w) are mixed and the mixture granulated. The granule are dried and sieved at about 1-1.5 mm of sieve.

According to another variant the process for obtaining thermo welded bags, is described wherein diclofenac granule or fast release granule comprising an unitary amount from 5 to 75 mg are mixed with rifaximin granule or gastroresistant rifaximin granule wherein the rifaximin is from 20 mg to 600 mg and excipients such as diluents, lubricant and optionally sweetening, flavouring, preservatives. Omeprazole in gastroresistant granule can be added to the mixture and the weight of diluents reduced proportionally to the amount of omeprazole. According to another embodiment a process for obtaining tablet is described, comprising rifaximin granules or rifaximin gastroresistant granules, diclofenac granules or diclofenac fast release granules, optionally omeprazole in gastroresistant granule and pharmaceutically excipients, wherein the component are mixed in a V mixer for a time from 10 to 30 minutes and the mixture compressed in a tableting machine. The tablets can be coated with film coating or gastroresistant film coating and the resulted tabled are dried to costant weight.

According to another variant the process for obtaining gelatin capsules is described, wherein rifaximin granules and diclofenac with excipients are mixed and the mixture introduced in prefilled gelatin capsules. Optionally omeprazole can be added to the mixture and the diluents amount proportionally reduced.

The active ingredients of the present disclosure for the claimed use may be also formulated in separate dosage forms which are provided as a system which can be in the form of a kit of parts comprising the any of the active ingredients and any of the formulations described herein.

In some embodiments, the system for the claimed use comprises an antibiotic and a proton pump inhibitor, for simultaneous, combined or sequential use in a related method herein described to treat and/or prevent an NSAID enteropathy in an individual under NSAID administration. In some embodiments, the enteropathy is a condition of the intestinal tract. In some embodiments, the duration of NSAID administration is at least one week, at least 10 days or at least two weeks. In some embodiments, the system herein described for the claimed use comprises at least one antibiotic and at least one proton pump inhibitor in dosages providing, upon timely administration to the individual, prevention of the NSAID gastrointestinal damage or enteropathy in the individual.

In some embodiments, the system for the claimed use comprises an NSAID, at least one antibiotic and optionally at least one proton pump inhibitor for simultaneous, combined or sequential use in a related use herein described to treat and/or prevent a condition of an individual wherein pain and/or inflammation are present. In some embodiments an effective amount of at least one NSAID can be performed for at least one week, at least ten days, or at least two weeks. In particular the NSAID administration can be performed for at least two weeks. In some embodiments, systems herein described for the claimed use comprise the NSAID and at least one of the antibiotic and the proton pump inhibitor in dosages allowing upon timely administration treatment and/or prevention of a rheumatic condition and/or other painful and/or inflammatory conditions in the individual.

In a particular embodiment, kits for the claimed use may include single or multiple doses of one or more active ingredients, each packaged or formulated individually, or single or multiple doses of two or more active ingredients packaged or formulated in combination. Thus, one or more active ingredients may be present in a first container, and the kit may optionally include one or more active ingredients in a second container. The container or containers are placed within a package, and the package may optionally include administration or dosage instructions in the form of a label on the package or in the form of an insert included in the packaging of the kit.

In some embodiments, the kit of the present disclosure for the claimed use comprises as active ingredients therapeutically effective amounts of i) rifaximin, ii) at least one NSAID(s).

In some embodiments, the kit of the present disclosure for the claimed use comprises as active ingredients therapeutically effective amounts of i) rifaximin, ii) at least one NSAID(s).

In some embodiments, the kit of the present disclosure for the claimed use comprises as active ingredients therapeutically effective amounts of i) a PXR agonist, ii) at least one NSAID(s); and iii) at least one PPI.

In an exemplary kit embodiment of the present disclosure for the claimed use, the antibiotic, the at least one NSAID and PPI are be provided as separate, independent dosage forms, such as, but not limited to, at least three dosage forms. Alternatively, rifaximin, NSAIDs and PPI are combined in a single, unified dosage form. Alternatively, gastroresistant rifaximin (and in particular EIR rifaximin), NSAIDs and PPI are combined in a single, unified dosage form.

The solid dosage forms selected from the group consisting of capsules, tablets, multilayer tablets, powders, granules, and sachets.

In some embodiments, the antibiotic and NSAIDs are combined in a single, unified dosage form and the at least one PPI are provided as a separate, independent dosage form. A kit may include a container or packaging for containing the pharmaceutical compositions and may also include divided containers such as a divided bottle or a divided foil packet.

The container may be, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. It is feasible that more than one container may be used together in a single package to market a single dosage form. For example, tablets may be contained in a bottle which is in turn contained within a box.

An example of a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules may be removed from the blister pack by manually applying pressure on the recesses whereby an opening is formed in the sheet at the place of the recess. The tablet or capsule may then be removed via said opening.

In some embodiments, a written memory aid containing information and/or instructions for the physician, pharmacist or subject may be comprised regarding when the medication is to be taken. A "daily dose" may be a single tablet or capsule or several tablets or capsules to be taken on a given day.

A kit may take the form of a dispenser designed to dispense the daily doses one at a time in the order of their intended use. The dispenser may be equipped with a memory-aid, so as to further facilitate compliance with the regimen. An example of such a memory-aid is a mechanical counter which indicates the number of daily doses that have been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The kit of the present disclosure may be for use in the methods described herein in the treatment of, for example, arthritis.

In some embodiments, uses and systems for use herein described and related compositions are expected to modulate and/or ameliorate the treated conditions.

The term "ameliorate," "amelioration," "improvement" or the like refers to, for example, a detectable improvement or a detectable change consistent with improvement that occurs in a subject or in at least a minority of subjects, e.g., in at least about 2%, 5%, 10%, 15%, 20%, 25%, 30%, 40%, 50%, 60%, 70%, 75%, 80%, 85%, 90%, 95%, 98%, 100% or in a range between any two of these values. Such improvement or change can be observed in treated subjects as compared to subjects not treated with rifaximin, where the untreated subjects have, or are subject to developing, the same or similar disease, condition, symptom or the like. Amelioration of a disease, condition, symptom or assay parameter can be determined subjectively or objectively, e.g., self-assessment by an individual, by a clinician's assessment or by conducting an appropriate assay or measurement, including, e.g., a quality of life assessment, a slowed progression of a condition, a reduced severity of a condition, or a suitable assay for the level or activity of a biomolecule, cell or by detection of BD episodes in a subject. Amelioration may be transient, prolonged or permanent or it may be variable at relevant times during or after a GI specific antibiotic is administered to a subject or is used in an assay or other method described herein or a cited reference, e.g., within timeframes described infra, or about 1 hour after the administration or use of a GI specific antibiotic to about 7 days, 2 weeks, 28 days, or 1, 3, 6, 9 months or more after an individual has received such treatment.

The "modulation" of, e.g., a symptom, level or biological activity of a molecule, or the like, refers, for example, that the symptom or activity, or the like is detectably increased or decreased. Such increase or decrease can be observed in treated subjects as compared to subjects not treated with a GI specific antibiotic, where the untreated subjects have, or are subject to developing, the same or similar disease, condition, symptom or the like. Such increases or decreases may be at least about 2%, 5%, 10%, 15%, 20%, 25%, 30%, 40%, 50%, 60%, 70%, 75%, 80%, 85%, 90%, 95%, 98%, 100%, 150%, 200%, 250%, 300%, 400%, 500%, 1000% or more or within any range between any two of these values. Modulation can be determined subjectively or objectively, e.g., by the individual's self-assessment, by a clinician's assessment or by conducting an appropriate assay or measurement, including, e.g., quality of life assessments or suitable assays for the level or activity of molecules within a subject. Modulation may be transient, prolonged or permanent or it may be variable at relevant times during or after a GI specific antibiotic is administered to a subject or is used in an assay or other method described herein or a cited reference, e.g., within times descried infra, or about 1 hour of the administration or use of a GI specific antibiotic to about 2 weeks, 28 days, 3, 6, 9 months or more after a subject(s) has received a GI specific antibiotic.

The term "modulate" may also refer to increases or decreases in the activity of a cell in response to exposure to a GI specific antibiotic, e.g., the inhibition of proliferation and/or induction of differentiation of at least a sub-population of cells in an animal such that a desired end result is achieved, e.g., a therapeutic result of GI specific antibiotic used for treatment may increase or decrease over the course of a particular treatment.

Further advantages and characteristics of the present disclosure will become more apparent hereinafter from the following detailed disclosure by way of illustration only with reference to an experimental section.

### EXAMPLES

The compositions, methods and systems herein described are further illustrated in the following examples, which are provided by way of illustration and are not intended to be limiting.

Example 1 describes a clinical, randomised, multicenter clinical trial. The study is designed to evaluate the efficacy and safety of rifaximin in gastroresistant microgranules 800 mg tablets twice a day in preventing small bowel lesions due to diclofenac SR 75 mg BID plus Omeprazole 20 mg once a day in healthy volunteers.

### Example 1: Double blind clinical study

A clinical randomised study, wherein 60 healthy volunteers are enrolled, will be performed in accordance with the description below. The study is a multicenter video capsule endoscopy (VCE) study comparing small bowel findings after treatment with diclofenac SR 75 mg BID plus Omeprazole 20 mg once a day with or without rifaximin 800 mg BID.

The main inclusion criteria for enrolling volunteers in the study comprises:
- male or non-pregnant, non-lactating female subjects from 18 years to 65 years old;
- normal findings on the physical examination and laboratory evaluations;
- mo more than 1 mucosal break at the baseline VCE;
- subjects' signed written informed consent.

The main exclusion criteria comprise:
- use of oral and systemic steroids, other NSAIDs (including low-dose aspirin and aminosalicylates), biological, sulphasalazine, misoprostol and other mucosal protective compounds, bisphosphonates, within 4 weeks prior to and during screening;
- use of probiotics, prebiotics, rifaximin and other antimicrobials, within 8 weeks prior to and during screening;
- use of prokinetic drugs within 2 weeks prior to and during screening;
- alcohol abuse;
- risk of pregnancy without adequate contraception;
- evidence of active duodenal and/or gastric ulcer, diverticulitis, infectious gastroenteritis, intestinal stricture or obstruction;
- a significant medical condition, which in the opinion of investigator precludes study participation.

Up to 60 volunteers randomised in two treatment groups as follows:
Group 1: one tablet of diclofenac SR 75 mg taken every 12 hours plus one capsule of omeprazole 20 mg once a day plus two placebo rifaximin 400 mg tablets will be administered every 12 hours, by oral route.
Group 2: one tablet of diclofenac SR 75 mg taken every 12 hours plus one capsule omeprazole 20 mg once a day plus two rifaximin gastroresistant microgranules 400 mg tablets (rifaximin EIR) every 12 hours, by oral route.

The total period to complete study per subject is expected to be 1 month, consisting of:
Screening period: Potential subjects undergo a screening visit (V1) to perform investigation on clinical data and laboratory tests, followed by a baseline VCE at visit 2 (V2).

Treatment period: Eligible subjects were randomised in two treatment groups at randomisation visit (V3). Each day during the 14 day-treatment period each subject received one capsule of diclofenac SR 75 mg every 12 hours plus one omeprazole 20 mg tablet once a day plus two tablets of investigational drug (rifaximin-EIR or matching placebo) every 12 hours. The final visit (V17) was performed no more than 36 hours after last drug administration and included second VCE and clinical and laboratory test assessments.

The primary efficacy endpoint criteria is the percentage of subjects developing at least one mucosal break at final visit evaluated by VCE and assessed according to a validated scoring system.

The secondary efficacy endpoints are the change from baseline to final visit in the number of mucosal lesions in the small bowel and the change from baseline to final visit in the number of mucosal lesions in the small bowel with/without hemorrhage (visible blood).

The safety parameters considered are: adverse events (AEs), clinical laboratory parameters (hematology, clinical chemistry and urinalysis), vital signs.

The expected result of this study is that the 60 randomised healthy volunteers (30 subjects per group) provide 80% power to detect a reduction of approximately 55% in the rate of subjects with mucosal breaks in the small bowel from baseline to final visit on the diclofenac plus omeprazole plus rifaximin group respect to diclofenac plus omeprazole group at a one-sided significance level of 0.05, assuming a rate of 58% in group treated with diclofenac plus omeprazole and a 10% rate of non-evaluable subjects.

### Example 2: Preparation of rifaximin in aastroresistant granules: Extended Intestinal Release (EIR) rifaximin (referential)

Rifaximin microgranules were prepared as described in U.S. patent application no. 11/814,628. In a fluid bed apparatus, Glatt GPC 30, with a Wurster system of 18 inches with a 1.8 mm spray jet, 25000 g of rifaximin powder and 125 g of Aerosil as fluidiser were loaded. Contemporaneously in a mixer under agitation a suspension was prepared using 48107 g of demineralised water, 9281 g of methacrylic acid ethylacrylate copolymer marketed under the trademark Kollicoat® MAE 100 P, 1392 g 1,2 propanediol, 2475 g of talc, 557 g of titanium dioxide FU and 62 g of iron oxide E 172. The solid components of the suspension were homogeneously mixed in demineralised water with a high speed homogeniser (Ultra Turrax). The prepared suspension was loaded in the Wurster type apparatus with a peristaltic pump and nebulised, at a pressure from 1.0 to 1.5 bar, through the 1.8 mm nozzle on the mixture of rifaximin powder and Aerosil® 200 maintained in suspension in the fluid bed by a warm air flow.

The applied conditions are described in Table 2:

**TABLE 2**

| **Process parameters** | **Pre-warm phase** | **Application of coating solution** | **Drying** |
|---|---|---|---|
| Air flow in entrance (m³/hour) | 400 ± 100 | 550 ± 100 | 350 ± 50 |
| Air temperature in entrance (°C) | 60 ± 2 | 60 ± 10 | 70 ± 2 |
| Product temperature (°C) | 32 | 25 - 27 | 30 ± 2 |
| Jet pressure (bar) (initial phase) | | 1-1.5 ± 0.1 | |
| Jet speed (g/min) | | 150 - 200 | |

The obtained microgranules were submitted to granulometry analysis by Light Scattering technology using a Malvern Mastersizer 2000 apparatus which result in greater than 91% of the microgranules having a dimension lower than 300 micron.

The microgranules composition comprising rifaximin is described in Table 3.

**TABLE 3**

| Composition | Amount (grams) | Amount (%) |
|---|---|---|
| Rifaximin | 25000 | 64.3 |
| Silica (Aerosil ® 200) | 125 | 0.3 |
| Methacrylic acid- methyl methacrylate copolymer 1:1 (Kollicoat® MAE 100P) | 9281 | 23.9 |
| 1,2 Propanediol | 1392 | 3.6 |
| Talc | 2475 | 6.4 |
| Titanium dioxide FU | 557 | 1.4 |
| Iron oxide E172 | 62 | 0.2 |

The microgranules obtained were analyzed by X-ray diffraction. The diffractograms show that the rifaximin has peaks characteristic of the β form, as identified in Cryst. Eng. Comm. 10, 1074-1081 (2008).

### Example 3: Preparation of Omeprazole in gastroresistant granules (referential)

The process for the preparation of omeprazole gastroresistant granules comprised three steps:
- spraying in a fluid-bed apparatus on a suspension containing omeprazole on microcrystalline cellulose cores;
- coating of the omeprazole loaded cores with hydroxypropyl methylcellulose film coating;
- spraying in a fluid-bed apparatus an aqueous suspension of gastroresistant film-coating on omeprazole granules.

An aqueous suspension was prepared by mixing 600 g omeprazole, 1.20 g of fumed silica and 60.0 g talc in 700 ml water and the solution was kept at 60°C under stirring until solubilization of the components and then 1312 g of hydroxypropyl methylcellulose were added; the resultant suspension was sprayed in a fluid bed.

1192.64 g of microcrystalline cellulose cores were loaded into a fluid bed apparatus type GPCG2 set up with bottom spray system and lined with the compounded suspension stirred at 300 rpm. The core coating was performed with the following conditions of Table 4.

**TABLE 4**

| | | | |
|---|---|---|---|
| **Nozzle** | 1.2 mm | **Air IN Flow** | 80 mc/h |
| **Pressure Spray** | 1.5 bar | **Air IN Temperature** | 58.0 °C |
| **Cylinder Height** | 30 mm | **Pump Flow** | 5.0 - 10.0 g/min |
| **Bottom Plate** | Type B | **Filter Pressure** | 70 - 1875 psi |
| **Product Filter Mesh** | 50 µm | **Product Pressure** | 750- 1430 psi |
| **Shaking/ Pause Product Filter (sec)** | 10/65 | **Air OUT Temperature** | 39.0°C - 40.0°C |
| **Process Time** | 4 h 30 min | **Product Temperature** | 42.5°C - 44.5°C |

After the spraying step, the granules were dried for 10 minutes at 55° C, then set the air inlet temperature at 36° C and allow the mass to cool the temperature ≤ 36° C, before stopping the process and download the product.

The composition of the omeprazole loaded cores obtained from the first step is reported in Table 5.

**TABLE 5**

| **Components** | **Amount (mg)** |
|---|---|
| Omeprazole | 20 |
| Hydroxypropyl methylcellulose | 0.23 |
| Fumed Silica | 0.04 |
| Talc | 2 |
| Cellulose microcrystalline | 43.73 |

The product obtained highly hygroscopic was immediately packed and stored in sealed containers.

The second step comprised the coating of omeprazole loaded cores in a fluid bed apparatus with hydroxypropyl methylcellulose.

A suspension of 88 g hydroxypropyl methylcellulose in 792 g of water was applied on 800 g of granules obtained from step 1 in a fluid bed apparatus type GPCG2 ,set up with bottom spray system and the suspension stirred at 200 rpm.

The process parameters are reported in Table 6.

**TABLE 6**

| | | | |
|---|---|---|---|
| **Nozzle** | 1.2 mm | **Air IN Flow** | 70 mc/h |
| **Pressure Spray** | 1.5 bar | **Air IN Temperature** | 65 °C |
| **Cylinder Height** | 15mm | **Pump Flow** | 4.6 - 5.5 g/min |
| **Bottom Plate** | Type B | **Filter Pressure** | 30- 105 psi |
| **Product Filter Mesh** | 50 µm | **Product Pressure** | 650- 815 psi |
| **Shaking/ Pause Product Filter (sec)** | 10/65 | **Air OUT Temperature** | 35.0°C - 42.0°C |
| **Process Time** | 3h 10 min | **Product temperature** | 42.7°C - 44.7°C |

The granules were dried for 30 minutes at temperature of 65° C. The unitary granule composition after the second step is reported in Table 7.

**TABLE 7**

| **Component** | **Amount (mg)** |
|---|---|
| Hydroxypropyl methylcellulose | 6.6 |
| Granulate from step 1 | 66.6 |

The third step comprised the coating of omeprazole granules with a gastroresistant film. A suspension of gastroresistant coating was prepared mixing 960 g of Acryl-Eze 93F19255 in 2880 ml of water, filtered on 500µm sieve was sprayed on 800 g of granules of step 2 in a fluid bed apparatus, with the process parameter reported in Table 8.

**TABLE 8**

| | | | |
|---|---|---|---|
| **Nozzle** | 1.2 mm | **Air IN Flow** | 70 m3/h |
| **Pressure Spray** | 1.5 bar | **Air IN Temperature** | 48 °C |
| **Cylinder Height** | 15mm | **Pump Flow** | 4.4 - 7.9 g/min |
| **Bottom Plate** | Type B | **Filter Pressure** | 25 - 100 psi |
| **Product Filter Mesh** | 50 µm | **Product Pressure** | 820- 1050 psi |
| **Shaking/ Pause Product Filter (sec)** | 10/65 | **Air OUT Temperature** | 32.7°C - 34.1 °C |
| **Process Time** | 12h 30 min | **Product Temperature** | 33.8°C - 35.7°C |

After the spraying step, the omeprazole granules were maintained for 10 minutes at 44°C, and then at 36° C to allow the mass to cool to the temperature of about 36° C. The obtained product was maintained at 40 °C for 12 hours in static heater.

The composition of the omeprazole gastroresistant granules containing is reported in Table 9.

**TABLE 9**

| **Component** | **Amount (mg)** | **Amount %** |
|---|---|---|
| Omeprazole | 20 | 17 |
| Hydroxypropyl methylcellulose | 0.23 | 0.2 |
| Fumed Silica | 0.04 | 0.03 |
| Talc | 2 | 1.7 |
| Hydroxypropyl methylcellulose | 6.6 | 5.6 |
| Gastroresistant coating | 87.9 | 75 |
| Total weight | 117.13 | |

The gastroresistant coating comprised methacrylic acid - ethyl acrylate copolymer, talc, macrogol 800, colloidal silica anhydrous, sodium carbonate, sodium lauryl sulfate.

### Example 4: Preparation of Omeprazole in gastroresistant granules (referential)

The process for the preparation of omeprazole gastroresistant granules comprised three steps:
*a*. Preparation of omeprazole loaded cores;
*b.* Preparation of coated omeprazole granules;
c. Coating omeprazole granules with gastroresistant polymer.

An aqueous solution was prepared by adding 14 g of HPMC to 170 ml water pre-heated at 60°C and added to an aqueous suspension prepared by adding at 500 ml water under stirring, 80 g omeprazole, 20 g silica and 60 g talc and the final suspension kept under stirring for 1.5 hours.

The suspension was spried on 1192.64 g microcrystalline cellulose cores in a fluid bed apparatus, type GPCG2 equipped with bottom spray system. The spraying was carried out with air flow of 45 m³/hour, and air temperature in entrance 65°C in entrance. The obtained granules were dried at temperature of 55°C for about 1 hour. The yield of the process was 96% with a loss on drying (LOD) of 2.10%.

The unitary composition of the omeprazole loaded cores is reported in Table 10.

**TABLE 10**

| **Component** | **Qty/Unit (mg)** | **Amount (%)** |
|---|---|---|
| Omeprazole | 20.00 | 5.94 |
| Colloidal anydrous silica | 0.30 | 0.09 |
| Talc | 15.00 | 4.45 |
| Hydroxypropyl methylcellulose (HPMC) | 3.50 | 1.04 |
| Microcrystalline Cellulose (Cellets® 350) | 298.16 | 88.49 |
| Total | 339.96 | |

The second step comprised the coating of omeprazole with hydroxypropyl methylcellulose wherein a suspension prepared by mixing 88 g hydroxypropyl methylcellulose in 792ml of water was sprayed on 800 g of granules obtained from first step in a fluid bed apparatus type GPCG2. The spraying step was carried out with air flow in entrance of 45 m³/hour and air temperature in entrance 65°C and the obtained granules were dried for 30 minutes at temperature of 25° C. The composition of the granules after the second step is reported in Table 11.

**TABLE 11**

| **Component** | **Qty/Unit (mg)** | **Amount (%)** |
|---|---|---|
| Omeprazole granule (step a) | 336.96 | 90.1 |
| Hydroxypropyl methylcellulose (HPMC) | 37.07 | 9.9 |

The third step comprised the coating of omeprazole granules with a gastroresistant film coating. A suspension of 660 g of Acryl-Eze® 93F19255 in 2340 ml of water was sprayed on 550 g of granules of step b) in a fluid bed apparatus with air flow in entrance 50 m³/hour and air temperature in entrance 70°C and the resultant omeprazole granules were dried for 30 minutes with air flow at 25°C.

The unitary composition of omeprazole gastroresistant granules is reported in Table 12.

**TABLE 12**

| **Component** | **Qty/Unit (mg)** | **Amount (%)** |
|---|---|---|
| Granules of omeprazole (step 2) | 374.0 | 45.5 |
| Gastroresistant coating (Acryl-EZE) | 448.00 | 54.5 |
| Total weight | 822.00 | |

The yield of the final process was 89.2%.

### Example 5: Preparation of diclofenac fast release granules (referential)

The fast release diclofenac granules were prepared in a fluid bed apparatus. A solution of 270 g diclofenac in 1750 g water, 126 g povidone and 3478 g sorbitol was sprayed in a fluid bed wherein 10000 g sorbitol were previously loaded. The granules were dried until the loss of drying (LOD) was about 2%.

The granulate composition containing diclofenac is reported in Table 13.

**TABLE 13**

| **Component** | **Amount (mg)** | **% (w/w)** |
|---|---|---|
| Diclofenac | 75 | 19 |
| Povidone | 35 | 0.7 |
| Sorbitol | 3744 | 97.2 |

### Example 6: Preparation of diclofenac fast release granules (referential)

A mixture of 20 g acid diclofenac and 1165 g of sorbitol, previously sieved on a mesh of 1 mm, was loaded in a fluid bed apparatus set up with top spray system. The powder was granulated with 166 ml aqueous solution containing 133 g sorbitol and 25 g PVP, with an air flow in entrance of 60 m³/hour and air temperature in entrance of 45°C.

The obtained granules were dried for 10 minutes at temperature of 45°C.

The diclofenac granulate unitary composition is reported in Table 14.

**TABLE 14**

| **Component** | **Qty/Unit (mg)** | **Amount (%)** |
|---|---|---|
| Acid Diclofenac | 25.00 | 1.5 |
| Polyvinylpyrrolidone (PVP) | 31.25 | 1.9 |
| Sorbitol | 1622.50 | 96.6 |

### Example 7: Preparation of diclofenac granules, (referential)

Diclofenac 75 g was mixed with 16 g microcrystalline cellulose and 7 g of hydroxypropyl methylcellulose in the Hi Shear Mixer; 200 ml water were added to the solid mixture and the mass granulated for 4 minutes. The granules were sieved in an oscillating granulator with 1.14 mm sieve. The granules were dried in a fluid bed apparatus until loss of drying (LOD) about ≤ 1%. The composition of diclofenac granules is reported in Table 15.

**TABLE 15**

| **Components** | **Amount (mg)** | **Amount (%)** |
|---|---|---|
| Diclofenac | 75 | 38.3 |
| Microcrystalline cellulose | 16 | 8.2 |
| Hydroxypropyl methylcellulose | 7 | 3.5 |
| Total weight | 98 | 50 |

### Example 8: Preparation of compositions comprising rifaximin and diclofenac and composition comprising rifaximin. diclofenac and omeprazole in thermo welded bags. (referential)

Table 16 reports the compositions for oral suspension comprising rifaximin, diclofenac and rifaximin, diclofenac and omeprazole in granule in thermo welded bags.

**TABLE 16**

| | **COMPOSITION** | | | | |
|---|---|---|---|---|---|
| **COMPONENTS** | **1 (mg)** | **2 (mg)** | **3 (mg)** | **4 (mg)** | **5 (mg)** |
| Rifaximin | 622.3 | 622.3 | | | 622.3 |
| Omeprazole | 160.6 | 160.6 | 160.6 | 160.6 | 160.6 |
| Colloidal Silica | 10 | 10 | 10 | 10 | 10 |
| Diclofenac | 3900 | | 3900 | | |
| Aspartame | 20 | 20 | 20 | 20 | 20 |
| Cherry-flavour | 100 | 100 | 100 | 100 | 100 |
| Sorbitol | 187.1 | 4012.1 | 409.4 | 4234.4 | 3989.1 |
| Diclofenac powder | | 75 | | 75 | |
| Diclofenac | | | | | 98 |
| Rifaximin powder | | | 400 | 400 | |
| Total weight | 5000 | 5000 | 5000 | 5000 | 5000 |

Thermo welded bags were prepared without omeprazole, wherein proportionally the sorbitol amount was proportionally increased.

### Example 9: Preparation of tablets comprising omeprazole, rifaximin and diclofenac and tablets comprising rifaximin and diclofenac (referential)

Tablet compositions 1-10 were obtained mixing all the components as reported in Table 17 in a V mixer and the mixture compressed in a tabletting machine. The tablets were coated with coating film or gastroresistant coating film and the coated tablets were dried until loss of drying of about 5%. The unitary tablet compositions are reported in Table 17.

**TABLE 17**

| | **Tablet Compositions** | | | | | | | | | |
|---|---|---|---|---|---|---|---|---|---|---|
| **Component** | **1 (mg)** | **2 (mg)** | **3 (mg)** | **4 (mg)** | **5 (mg)** | **6 (mg)** | **7 (mg)** | **8 (mg)** | **9 (mg)** | **10 (mg)** |
| Omeprazole gastroresistant granule (Example 3) | 161 | 161 | 161 | 161 | | | 80 | 80 | 161 | 161 |
| Microcryst. cellulose | 238 | 228 | | | | | 334 | | 105 | |
| Diclofenac | 75 | 75 | | 75 | | 75 | 37.5 | | 75 | |
| Croscarmellose sodium | 25 | 35 | | | | | 35 | | 35 | |
| Glyceryl palmitostearate | 1 | 1 | | | | | 1 | | 1 | |
| Rifaximin | | 400 | 400 | 400 | 400 | 400 | | | | |
| Diclofenac granules (Example 7) | | | 98 | | 98 | | | 49 | | 98 |
| Pregelatinized starch | | | 340 | 363 | 281 | 304 | | 315 | | 275 |
| Colloidal silica | | | 0.8 | 0.8 | 0.6 | 0.6 | | 0.8 | | 0.8 |
| Magnesium stearate | | | 0.8 | 0.8 | 0.6 | 0.6 | | 0.8 | | 0.8 |
| Omeprazole | | | | | 20 | 20 | | | | |
| Rifaximin gastroresistant granules (Example 2) | | | | | | | 311.2 | 311.2 | 622 | 622 |
| Coating film | | | 43 | 43 | | | | 43 | | 43 |
| Gastroresistant Coating film | | | | | 120 | 120 | | | | |
| Total weight (mg) | 500 | 900 | 1000 | 1000 | 800 | 800 | 800 | 800 | 1000 | 1200 |

Tablets composition as compositions 1-4 and 7-10 without omeprazole were prepared wherein proportionally amount of microcrystalline cellulose were increased.

### Example 10: Preparation capsules comprising omeprazole, diclofenac and rifaximin and capsules comprising rifaximin and diclofenac. (referential)

Table 18 reports the compositions of the gelatin capsule comprising omeprazole, diclofenac and rifaximin and capsules comprising rifaximin and diclofenac, wherein compositions 3 and 4 comprise 400 mg of rifaximin in gastroresistant granules an composition 5 and 6 comprise 200 mg rifaximin in gastroresistant granules.

**TABLE 18**

| | **Composition** | | | | | |
|---|---|---|---|---|---|---|
| **Component** | **1 (mg)** | **2 (mg)** | **3 (mg)** | **4 (mg)** | **5 (mg)** | **6 (mg)** |
| Omeprazole gastroresistant granules (Example 3) | 161 | 161 | 161 | 161 | 80.3 | 80.3 |
| Diclofenac Fast release granules (Example 5) | 98 | | 98 | | 49 | |
| Rifaximin powder | 400 | 400 | | | | |
| Talc | 0.5 | 0.5 | 0.5 | 0.5 | 0.5 | 0.5 |
| Titanium dioxide | 0.5 | 0.5 | 0.5 | 0.5 | 0.5 | 0.5 |
| Lactose | 40 | 63.5 | 18 | 41 | 58.7 | 70.2 |
| Diclofenac powder | | 75 | | 75 | | 37.5 |
| Rifaximin gastroresistant granules | | | 622 | 622 | 311 | 311 |
| Total weight | 700 | 700 | 900 | 900 | 500 | 500 |

All the components were mixed in a V mixer for 20 minutes and the mixed powder was introduced into the gelatin capsules.

### Example 11: Preparation of compositions comprising rifaximin. diclofenac and omeprazole in thermo welded bags (referential)

Rifaximin in gastroresistant granules or in powder was mixed a V mixer with diclofenac in granules fast release or diclofenac powder or diclofenac granules, omeprazole gastroresistant granules and the excipients. All the components were previously sieved in sieve mesh of 0.5 mm.

The resulting mixture was divided in thermo welded bags containing 6.5 grams of product, wherein rifaximin is 400 mg. In the following Table 19, the compositions for oral suspension comprising rifaximin, diclofenac and omeprazole are reported.

**TABLE 19**

| **Composition** | | | | | |
|---|---|---|---|---|---|
| **Component** | **6 (mg)** | **7 (mg)** | **8 (mg)** | **9 (mg)** | **10 (mg)** |
| Rifaximin gastroresistant granule (Example 2) | 622.3 | 622.3 | | | 622.3 |
| Rifaximin powder | | | 400 | 400 | |
| Omeprazole gastroresistant granules (Example 4) | 822.5 | 822.5 | 822.5 | 822.5 | 822.5 |
| Diclofenac granules Fast release (Example 5) | 3854 | | 3854 | | |
| Diclofenac powder | | 75 | | 75 | |
| Diclofenac granules (Example 7) | | | | | 98 |
| Passion Fruit Flavor | 200 | 200 | 200 | 200 | 200 |
| Sodium saccharine | 40 | 40 | 40 | 40 | 40 |
| Sodium carboxymethylcellulose | 961.7 | 4740.7 | 1184 | 4963 | 4717.7 |
| Total weight | 6500 | 6500 | 6500 | 6500 | 6500 |

The thermo welded bags were also prepared comprising rifaximin 800 mg or 1244.6 mg rifaximin in gastroresistant granules, with proportional reduction of sodium carboxymethylcellulose. The thermo-welded bags were prepared also without omeprazole increasing proportionally the amount of sodium carboxymethyl cellulose.

### Example 12: Preparation of rifaximin granulates (referential)

A mixture of 2669.5 g of rifaximin, 160.2 g of glyceryl palmitostearate, 8.8 g of talc, 1161.2 g of microcrystalline cellulose, previously sieved on a mesh of 1 mm, was mixed in a V mixer for 10 minutes at 14 rpm.

The mixture was granulated in a dried granulator equipped with a mesh from 3.15 and 1.45 mm.

### Example 13: Preparation of tablets comprising omeprazole, rifaximin and diclofenac (referential)

The tablets were prepared with a process with a first step of preparation of core and a second step wherein the core was coated with a gastroresistant film coating.

### A) Preparation of tablets cores

A mixture of 20 g of omeprazole, 75 g of diclofenac sodium, 80 g of croscarmellose sodium, 20 g of glyceryl palmitostearate, 50 g of copovidone and 556.3 g of microcrystalline cellulose or 556.3 g of calcium dihydrogen phosphate were sieved on a mesh of 0.8 mm and were mixed with 1198.8 g of rifaximin granules, prepared as Example 6 in a V mixer for 20 minutes at 16 rpm, and then compressed with a precompression of 394 mm and a compression of 280 mm.

### B) Coating of tablets with film coating

The tablets obtained were loaded in a pan and the coating was carried out with a 20% (w/v) aqueous solution of Acryl-EZE with an air temperature in entrance of 56°C, an air pressure in entrance of 200 m³/hour and a distance nozzle of 21 cm.

The tablet compositions are reported in Table 20.

**TABLE 20**

| | **Component** | **Qty/unit (mg)** | **Qty/unit (mg)** |
|---|---|---|---|
| | | **Tablet 1** | **Tablet 2** |
| | Granules of Rifaximin obtained as in Example 6 | 599.36 (corresponding to 400 mg of Rifaximin) | 599.36 (correspondin g to 400 mg of Rifaximin) |
| **Core** | Omeprazole | 10.00 | 10.00 |
| | Sodium Diclofenac | 37.50 | 37.50 |
| | Croscarmellose sodium | 40.00 | 40.00 |
| | Glyceryl palmitostearate | 10.00 | 10.00 |
| | Copovidone | 25.00 | 25.00 |
| | Microcrystalline cellulose | 278.14 | |
| | Bibasic Calcium Posphate | | 278.14 |
| **Coating** | Acryl-EZE Clear | 64.96 | 64.96 |
| TOT. | | 1064.96 | 1064.96 |

### Example 14: Preparation of gastoresistant tablets comprising rifaximin and sodium salt diclofenac (referential)

The tablets were prepared in the same condition of Example 8, wherein the omeprazole was omitted.

### A) Preparation of tablet cores

A mixture of 75 g of diclofenac sodium, 80 g croscarmellose sodium, 20 g glyceryl palmitostearate, 50 g copovidone and 576.3 g microcrystalline cellulose or 576.3 g of biphasic calcium phosphate were sieved on a mesh of 0.8 mm and mixed with 1198.8 g of rifaximin granules prepared as Example 6. The mixture was kept in a V mixer for 20 minutes at 16 rpm.

The resultant mixture is compressed with a pre-compression of 394 mm and a compression of 280 mm.

### B) Coating of tablets with film coating

The tablets obtained were loaded in a pan and the coating was carried out with a 20% (w/v) aqueous solution of Acryl-EZE with an air temperature in entrance of 56°C, an air pressure in entrance of 200 m³/h and a distance nozzle of 21 cm. The tablet compositions 3 and 4 are reported in Table 21.

**TABLE 21**

| | **Component** | **Qty/unit (mg)** | **Qty/unit (mg)** |
|---|---|---|---|
| | | **Composition 3** | **Composition 4** |
| | Rifaximin Granules (Example 6) | 600 | 600 |
| | Diclofenac Sodium salt | 75 | 75 |
| **Core** | Croscarmellose sodium | 40 | 40 |
| | Glyceryl palmitostearate | 10 | 10 |
| | Copovidone | 25 | 25 |
| | Microcrystalline cellulose | 250 | |
| | Bibasic Calcium Posphate | | 250 |
| **Gastroresist. Coating** | Acryl-EZE Clear (acrylate copolymer) | 65 | 65 |
| TOT. | | 1065 | 1065 |

Same tablet compositions were prepared with film coating comprising hydroxypropylmethyl cellulose, titanium dioxide, disodium edentate, propylene glycol and red iron oxide E172.

### Example 15: Preparation of capsules comprising rifaximin. omeprazole and diclofenac. (referential)

Gelatin capsule comprising omeprazole, diclofenac and rifaximin and capsules comprising rifaximin and diclofenac, were prepared with a process wherein all the components were mixed in a V blender for 20 minutes and the mixed powder was introduced into the gelatin capsules.

Table 22 reports the unitary capsules compositions.

**TABLE 22**

| | **Capsule Compositions** | | | | | |
|---|---|---|---|---|---|---|
| | **1** | **2** | **3** | **4** | **5** | **6** |
| **Component** | **Amount (mg)** | | | | | |
| Omeprazole gastroresistant granules (Example 4) | 822.5 | 822.5 | 822.5 | 822.5 | 822.5 | 822.5 |
| Rifaximin powder | 400 | 400 | | | | |
| Diclofenac powder | | 75 | | 75 | | 75 |
| Rifaximin gastroresistant granules (Example 2) | | | 622 | 622 | 622 | 622 |

### Example 16: Effects of rifaximin and gastroresistant rifaximin in indomethacin-induced enteropathy in rats (referential)

Efficacy of rifaximin and gastroresistant rifaximin in enteropathy induced by NSAID administration has been evaluated in a preclinical study in animals.

A total of thirty six (36) male Albino Sprague Dawley rats, 500 to 600 g body weight (40-week old) were assessed.

Enteropathy was induced in thirty one (31) male Albino Sprague Dawley rats, using indomethacin. The experimental groups were arranged as follows:
- Group 1: five animals treated with drug vehicle for 14 days (normal controls).
- Group 2: ten animals treated with indomethacin 1.5 mg/kg BID for 14 days (indomethacin ulcerated controls).
- Group 3: seven indomethacin-ulcerated animals co-treated with gastroresistant rifaximin 25 mg/kg BID for 14 days.
- Group 4: seven indomethacin-ulcerated animals co-treated with gastroresistant rifaximin 50 mg/kg BID for 14 days.
- Group 5: seven indomethacin-ulcerated animals co-treated with rifaximin polymorph alpha 50 mg/kg BID for 14 days.

In particular the Groups 1 to 5 were treated as follows:
- Group 1 received 1% methocel (0.25 ml/rat) and 1% methylcellulose (1 ml/rat) for 14 days.
- Group 2 was treated for 14 days with indomethacin 1.5 mg/kg BID by intragastric route, suspended in 1% methocel and administered in a volume of 0.25 ml/rat.
- Group 3 received gastroresistant rifaximin (25 mg/kg; 7 rats/group) suspended in 1% methylcellulose (1 ml/rat), administered twice daily, 1 hour before indomethacin administration.
- Group 4 received gastroresistant rifaximin (50 mg/kg; 7 rats/group) suspended in 1% methylcellulose (1 ml/rat), administered twice daily, 1 hour before indomethacin administration.
- Group 5 received rifaximin polymorph alpha (50 mg/kg, 7 rats/group), suspended in 1% methylcellulose (1 ml/rat), administered twice daily, 1 hour before indomethacin administration.

Twenty four hours after the last dose, the rats were euthanized and samples of blood were collected for the assessment of hemoglobin levels.

The whole intestinal tract was removed and processed for the evaluation of tissue myeloperoxidase (MPO) levels and histological analysis of intestinal damage in jejunum and ileum.

The histological evaluation of intestinal injury was carried out as described by Anthony A, et al., "Early histological features of small intestinal injury induced by indomethacin," Aliment. Pharmacol. Ther. 1993; 7:29-39. Upon removal, the whole intestinal tract was immediately injected with 10% formalin and left in the same fixative solution. After 30 min, it was opened along the anti-mesenteric border, cleaned of fecal content and fixed in 10% formalin for 24 h. Tissue samples were taken as described below and the full length of small intestine was measured.

### In particular the following tissue samples were taken

- Ileum: 1 cm of tissue proximal to the ileo-cecal valve was discarded and two specimens of 1.5 - 2 cm of ileum were taken at this edge as well as 5 cm away from the first ileum sample
- Proximal jejunum: two specimens of 1.5 - 2 cm were taken 5 cm away from both the proximal and distal end of the 37.5% of the small intestine total length.
- Distal jejunum: two specimens of 1.5 - 2 cm were taken 5 cm away from both the proximal and distal end of 81% of small intestine's total length.

After fixation, tissues were embedded into paraffin blocks and were cut into consecutive serial 3-µm sections for staining with hematoxylin and eosin. Histological damage was assessed by two observers, blind to the treatment. Each section was given a score of lesion (Type 1 to 3) (Table 23).

Quantitative assessment of histological damage was expressed as percentage of the total length of lesions over the total length of the histological section.

Exemplary pictures, showing the histological appearance of Type 1, 2 and 3 lesions of jejunum and ileum, are displayed in FIG. 1.

**TABLE 23**

| **Stages of indomethacin** - **induced microscopic intestinal damage** | |
|---|---|
| **Type** | **Histological feature** |
| 1 | • Damages confined to the tunica mucosa |
| | • De-epithelization |
| | • Significant morphologic alterations of villi |
| 2 | • Inflammatory infiltration in the submucosa, with thickening of the tunica muscularis or serosa |
| | • The morphologic architecture of tunica mucosa is preserved |
| 3 | • Damage involves the full thickness of intestinal wall |
| | • The morphologic patterns of tunicae are lost |
| | • Inflammatory reaction widely extended to the tunica serosa with a significant increase in the thickness |

Tissue myeloperoxidase levels (MPO) was assumed as a quantitative index to estimate the degree of mucosal infiltration by polymorphonuclear cells, and thereby the severity of enteropathy elicited by indomethacin. Specimens of intestinal tissues (30 mg) were homogenized on ice with a polytron homogenizer (QIAGEN, Milan, Italy) in 0.6 mL of ice-cold lysis buffer (200 mM NaCl, 5 mM EDTA, 10 mM Tris, 10% glycerine, 1 mM PMSF, 1 µg/ml leupeptin and 28 µg/ml aprotinin (pH 7.4). The homogenate was centrifuged 2 times at 4°C for 15 min at 1.500 g. The supernatant was diluted 1:5 and used for determination of MPO concentration by means of enzyme-linked immunosorbent assay (ELISA) (Hycult Biotech, Uden, Netherlands). All samples were assayed within 2 day after collection. The results were expressed as nanograms of MPO per milligram of intestinal tissue.

Hemoglobin analysis was performed on rat blood samples collected as reported above, by means of Quantichrom Hemoglobin assay kit (Bioassay Systems, Hayward, CA, USA) and expressed as g/dL.

At the end of the treatment period (14 days), 4 rats died in the indomethacin treated group (Group 2), thus displaying a 40 % mortality rate. By contrast, the co-administration of gastroresistant rifaximin 25 mg/kg and 50 mg/kg or rifaximin polymorph alpha 50 mg/kg BID with indomethacin was not associated with deaths. A percentage of 100% of survivors was also recorded in vehicle control group (Group 1).

In FIG. 2 and FIG. 3, the results of the microscopic assessment of intestinal damage are reported. In jejunual specimens obtained from control animals (Group 1), microscopic examination did not reveal any lesion of type 1, 2 and 3 (FIG. 2). Administration of indomethacin (1.5 mg/kg BID) for 14 days was associated with the occurrence of various degrees of type 1, 2 and 3 lesions (FIG. 2).

Gastroresistant rifaximin 25 mg/kg and 50 mg/kg or rifaximin polymorph alpha 50 mg/kg BID reduced the degree of type 1 lesions, although mean values were not significantly different from those estimated in the indomethacin group. By contrast, rifaximin significantly reduced Type 2 lesions, in comparison with indomethacin alone. As regards type 3 lesions, no significant differences were detected when comparing the rifaximin-treated experimental groups with indomethacin alone (FIG. 2).

In the ileum, indomethacin administration was associated with the occurrence of type 1 and 2 lesions, while no significant differences were detected for type 3 lesions, in comparison with control animals (FIG. 3). Treatment with gastroresistant rifaximin 25 mg/kg, gastroresistant rifaximin 50 mg/kg or rifaximin polymorph alpha 50 mg/kg BID elicited a significant reduction of Type 1 lesions, while no significant differences were appreciated on the occurrence of type 2 lesions (FIG. 3). Type 3 lesions did not differ significantly by comparison of controls with the other drug treatment groups (FIG. 3).

The results of tissue myeloperoxidase levels (MPO) levels are reported in FIG. 4. The tissue myeloperoxidase levels (MPO) levels detected in jejuna specimens from rats treated with indomethacin (1.5 mg/kg BID) for 14 days were significantly increased, in comparison with controls (FIG. 4). In the presence of indomethacin, gastroresistant rifaximin 25 mg/kg or 50 mg/kg BID elicited a significant reduction of MPO levels, while rifaximin polymorph alpha 50 mg/kg BID did not exert any relevant effect (FIG. 4). In the ileum, indomethacin caused also a significant increase in tissue MPO levels, which was blunted by gastroresistant rifaximin 25 mg/kg, gastroresistant rifaximin 50 mg/kg or rifaximin polymorph alpha 50 mg/kg BID (FIG. 4).

The blood hemoglobin results are reported in FIG. 5. Animals treated with indomethacin (1.5 mg/kg BID) for 14 days displayed a significant decrease in blood hemoglobin concentration. In animals treated with gastroresistant rifaximin at both doses, hemoglobin levels did not differ significantly from those recorded in control animals, while in rats treated with rifaximin polymorph alpha 50 mg/kg BID, these levels were decreased (FIG. 5).

Table 24 shows the effects of indomethacin, alone or in combination with gastroresistant rifaximin and rifaximin polymorph α on mortality rate, blood hemoglobin and tissue myeloperoxidase levels.

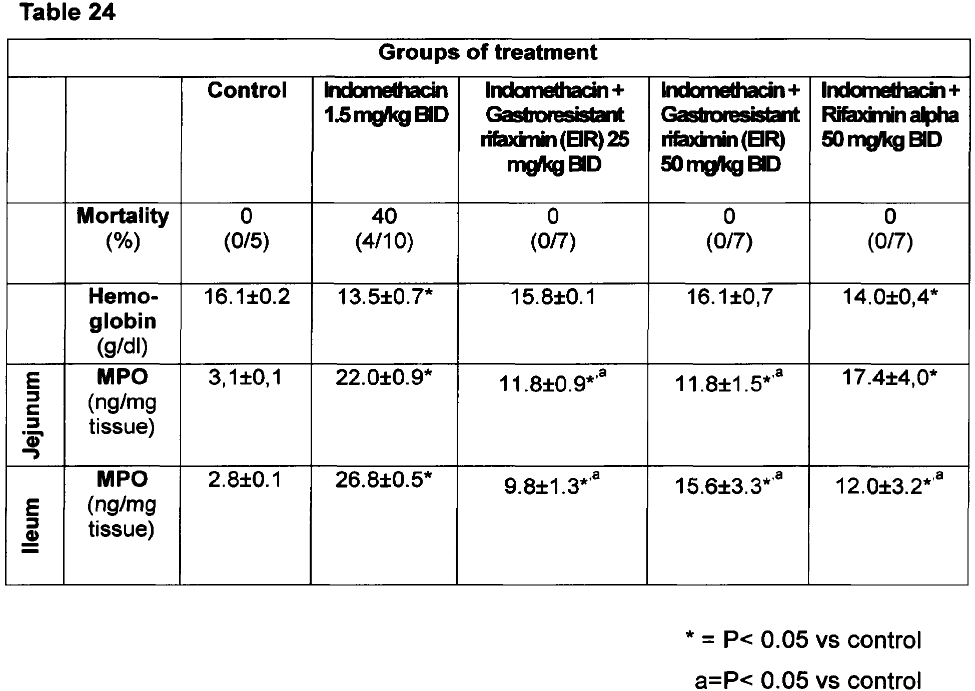

In view of the above results chronic indomethacin administration was associated with occurrence of intestinal damage, tissue polymorphonuclear cell infiltrate and digestive blood loss.

The administration of gastroresistant rifaximin at both 25 and 50 mg/kg BID was associated with a reduced mortality (mortality: 0%) with respect to indomethacin treated rats (mortality: 40%), significantly reduced tissue MPO levels with respect to indomethacin-treated animals and a general improvement of histological damage, although in some cases significant for each Type (1, 2 and 3) of damage described in ileum and jejunum.

The administration of gastroresistant rifaximin (EIR rifaximin) resulted more efficacious than rifaximin not gastroresistant in the treatment or prevention of NSAID intestinal damage.

Rifaximin is more effective than control wherein rifaximin is not present. Rifaximin alpha appears to be less effective than gastroresistant rifaximin in counteracting indomethacin-induced enteropathy.

Gastroresistant rifaximin is effective in the prevention of indomethacin induced damage it's possible to compare or the total daily dose used in animals versus humans or the dose in mg/kg using the human equivalent dose correction factor. In case for example of gastroresistant rifaximin (3 mg/kg daily for 14 days corresponding to a human equivalent dose of 0.5 mg/kg) in a range of human equivalent doses between 8 mg/kg and 16 mg/kg.

The effective rifaximin dosage administered in animal model in terms of human equivalent doses are less than (8 mg/kg) or slightly lower (16 mg/kg) with respect to the dose used in the clinical trial (27 mg/kg) and even if hemoglobin levels, MPO and histological damage were evaluated in humans the same results are expected.

### Example 17: Effects of gastroresistant rifaximin in indomethacin and diclofenac-induced enteropathy in rats

A total of one hundred forty two (142) male Albino Sprague Dawley rats, 500 to 600 g body weight (40-week old) were assessed.

Enteropathy was induced in sixty six (66) male Albino Sprague Dawley rats, using indomethacin and in fifty four (54) male Albino Sprague Dawley rats using diclofenac, in presence or absence of omeprazole.

The enteropathy induced using indomethacin was carried out in the groups 1 to 7 as follows: (referential)
Group 1: ten animals treated with drug vehicle for 14 days (normal controls).
Group 2: fifteen animals treated with indomethacin 1.5 mg/kg BID for 14 days (indomethacin ulcerated controls).
Group 3: fifteen indomethacin-treated animals, 1.5 mg/kg BID, co-treated with omeprazole 0.7 mg/kg once daily for 14 days (indomethacin plus omeprazole ulcerated controls).
Group 4: twelve indomethacin-treated animals, 1.5 mg/kg BID, co-treated with gastroresistant rifaximin (50 mg/kg BID) for 14 days.
Group 5: twelve indomethacin (1.5 mg/kg BID) and omeprazole (0.7 mg/kg/day)-treated animals, co-treated with gastroresistant rifaximin (50 mg/kg BID) for 14 days.
Group 6: twelve omeprazole-treated animals co-treated with gastroresistant rifaximin (50 mg/kg BID) for 14 days (omeprazole controls).
Group 7: twelve animals pretreated with gastroresistant rifaximin, 50 mg/kg BID, for 1 week followed by indomethacin co-treatment with gastroresistant rifaximin for 14 days (50 mg/kg BID).
   The enteropathy induced using diclofenac was carried out in the groups 8 to 11 as follows: (invention)
Group 8: fifteen animals treated with diclofenac 4 mg/kg BID (diclofenac ulcerated control.
Group 9: fifteen animals treated with diclofenac 4 mg/kg BID and omeprazole 0.7 mg/kg once daily (diclofenac and omeprazole ulcerated control).
Group 10: twelve diclofenac-treated animals co-treated with gastroresistant rifaximin (50 mg/kg BID) for 14 days.
Group 11: twelve diclofenac- and omeprazole-treated animals co-treated with gastroresistant rifaximin (50 mg/kg BID) for 14 days.

Groups 1 to 11 were treated as follows:
Group 1 received 1% methylcellulose (0.3 ml/rat) and 1% methylcellulose (1 ml/rat) for 14 days.
Group 2 non-fasted rats were treated for 14 days with indomethacin 1.5 mg/kg BID by intragastric route, suspended in 1% methylcellulose and administered in a volume of 0.3 ml/rat.
Group 3 non-fasted rats were treated for 14 days with indomethacin 1.5 mg/kg BID, plus omeprazole 0.7 mg/kg once daily, by intragastric route, suspended in 1% methylcellulose and administered in a volume of 0.3 ml/rat.
Group 4 received gastroresistant rifaximin (50 mg/kg; 12 rats/group) suspended in 1% methylcellulose (1 ml/rat), twice daily, 1 hour before indomethacin administration.
Group 5 received gastroresistant rifaximin (50 mg/kg; 12 rats/group) suspended in 1% methylcellulose (1 ml/rat), twice daily, 1 hour before indomethacin administration plus omeprazole 0.7 mg/kg once daily.
Group 6 received gastroresistant rifaximin (50 mg/kg; 12 rats/group) suspended in 1% methylcellulose (1 ml/rat), twice daily, 1 hour before omeprazole 0.7 mg/kg once daily.
Group 7 received pretreatment with gastroresistant rifaximin 50 mg/kg BID for 1 week followed by indomethacin co-treatment with gastroresistant rifaximin 1 hour before indomethacin administration for 14 days.
Group 8 non-fasted rats were treated for 14 days with diclofenac 4 mg/kg BID by intragastric route, suspended in 1% methylcellulose and administered in a volume of 0.3 ml/rat.
Group 9 non-fasted rats were treated for 14 days with diclofenac 4 mg/kg BID, plus omeprazole 0.7 mg/kg once daily, by intragastric route, suspended in 1% methylcellulose and administered in a volume of 0.3 ml/rat.
Group 10 received gastroresistant rifaximin (50 mg/kg; 12 rats/group) suspended in 1% methylcellulose (1 ml/rat), twice daily, 1 hour before diclofenac administration.
Group 11 received gastroresistant rifaximin (50 mg/kg; 12 rats/group) suspended in 1% methylcellulose (1 ml/rat), twice daily, 1 hour before diclofenac administration plus omeprazole 0.7 mg/kg once daily.

Twenty four hours after the last dose, the rats were euthanized and samples of blood were collected for the assessment of hemoglobin levels.

The whole intestinal tract was removed and intestinal tissue specimens processed for the evaluation of tissue myeloperoxidase (MPO) levels, an index of tissue neutrophils infiltration, of tissue malondialdehyde (MDA) levels, an index of lipid peroxidation and histological analysis of intestinal damage in jejunum and ileum.

Haemoglobin analysis was performed on blood samples collected as reported above, using a Quantichrom Hemoglobin assay kit (Bioassay Systems, Hayward, CA, USA) and expressed as g/dL.

MPO was assessed as described by Fornai et al., NSAID-Induced Enteropathy: Are the Currently Available Selective COX-2 Inhibitors All the Same, J Pharmacol Exp Ther January 2014 348:86-95 and assumed as a quantitative index to estimate the degree of intestinal wall infiltration by polymorphonuclear cells.

Specimens of 30 mg intestinal tissues were homogenized on ice with a polytron homogenizer (QIAGEN, Milan, Italy) in 0.6 mL of ice-cold lysis buffer consisting of 200 mM NaCl, 5 mM EDTA, 10 mM Tris, 10% glycerine, 1 mM phenylmethylsulfonyl fluoride, 1 µg/ml leupeptin and 28 µg/ml aprotinin at pH 7.4. The homogenate was centrifuged 2 times at 41 mM ph15 min at 1.500 g. The supernatant was diluted 1:5 and used for determination of MPO concentration by means of enzyme-linked immunosorbent assay (ELISA).(Hycult Biotech, Uden, Netherlands). All samples were assayed as described in Example 15. The results were expressed as nanograms of MPO per milligram of intestinal tissue.

MDA concentration in intestinal tissues was determined to obtain quantitative estimates of membrane lipid peroxidation as described by Fornai et al., NSAID-Induced Enteropathy: Are the Currently Available Selective COX-2 Inhibitors All the Same? J Pharmacol Exp Ther January 2014 348:86-95. For this purpose, intestinal tissue was excised, weighed, minced by forceps, homogenized in 2 ml of cold buffer consisting of Tris-HCI 20 mM, at pH 7.4, using a polytron homogenizer (QIAGEN, Milan, Italy), and centrifuged at 1,500 g for 10 min at 4°C. Aliquots of supernatants were then used for subsequent assay procedures. Mucosal MDA concentrations were estimated using a colorimetric assay kit (Cayman Chemical, Ann Arbor, MI, U.S.A.). Results were expressed as nanomoles of MDA per milligram of intestinal tissue.

The histological evaluation of intestinal injury was carried out as previously described by Fornai et al., NSAID-Induced Enteropathy: Are the Currently Available Selective COX-2 Inhibitors All the Same? J Pharmacol Exp Ther January 2014 348:86-95.

Upon removal, the whole intestinal tract was immediately injected with 10% formalin and left in the same fixative solution. After 30 min, it was opened along the anti-mesenteric border, cleaned of fecal content and fixed in 10% formalin for 24 h. Tissue samples were taken as described below in order to rule out any bias:
The full length of the small intestine was measured. In particular:
- Proximal jejunum: two specimens of 1.5-2 cm were taken 5 cm away from both the proximal and distal end of the 37.5% of the small intestine total length.
- Distal jejunum: two specimens of 1.5-2 cm were taken 5 cm away from both the proximal and distal end of the 81% of the small intestine total length.
- Ileum: 1 cm of tissue proximal to the ileo-cecal valve was discarded and two specimens of 1.5-2 cm of ileum were taken at this edge as well as 5 cm away from the first ileum sample.

Sections of jejunum and ileum were embedded into paraffin blocks and cut into 3 consecutive serial 7-8 µm sections. The slices were cut at two different points of the block: two on the surface and three at a deeper level. Each slice was placed on the slide for staining with haematoxylin and eosin. Histological damage was assessed by two observers, blind to the treatment, according to the score system proposed by Fornai et al. NSAID-Induced Enteropathy: Are the Currently Available Selective COX-2 Inhibitors All the Same? J Pharmacol Exp Ther January 2014 348:86-95.

The intestinal damage was classified as reported in Table 27 wherein reference is made with respect to the histological appearance of type 1, 2 and 3 lesions of jejunum and ileum to the illustration of FIG. 1.

The results were presented as mean ± standard error of the mean (S.E.M.). The statistical significance of data was evaluated by one way analysis of variance (ANOVA) followed by post hoc analysis by Student-Newman-Keuls test, and P values lower than 0.05 were considered significant. All statistical procedures were performed using GraphPad Prism 3.0 software (GraphPad Prism, San Diego, CA, USA).

At the end of the treatment period, the group treated with indomethacin displayed a 13.3% mortality rate as reported in Tables 25 and 26. In groups treated with indomethacin plus omeprazole (Group 3), indomethacin and gastroresistant rifaximin, with 1 week pretreatment with gastroresistant rifaximin (Group 7) or indomethacin and gastroresistant rifaximin (Group 4) the mortality rate was lower 6.7, 8.3 and 8.3% respectively, while no deaths were observed in animals treated with indomethacin plus omeprazole and gastroresistant rifaximin (Group 5) or omeprazole and gastroresistant rifaximin (Group 6) as reported in Table 26. The most prominent mortality rate corresponding to 46.7% was observed in animals treated with diclofenac (Group 8) as reported in Tables 25 and 26. In animals treated with diclofenac and omeprazole (Group 9) or diclofenac and gastroresistant rifaximin (Group 10), the mortality rate was significantly lower, 6.7 and 13.3% respectively, as compared with group treated with diclofenac alone, while all animals treated with diclofenac plus omeprazole and gastroresistant rifaximin (Group 11) survived (Table 26).

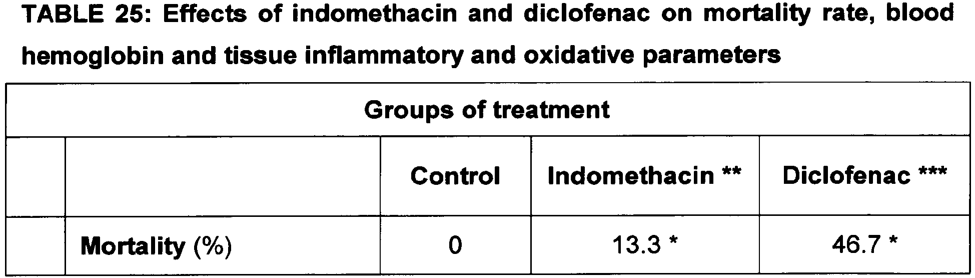

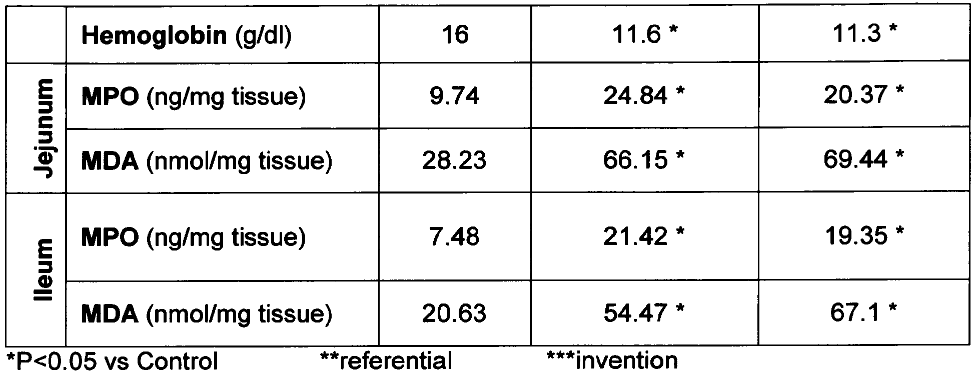

**TABLE 26: Mortality rates in groups of treatment**

| **Treatment** | **Dose (mg/Kg/day)** | **No. of animals** | **Mortality (%)** |
|---|---|---|---|
| Control (group 1) | - | 10 | (0/10) 0 |
| Indomethacin (group 2) * | **3** | 15 | (2/15) 13.3 |
| Indomethacin + omeprazole (Group 3) * | 3+0.7 | 15 | (1/15) 6.7 |
| Indomethacin+ gastroresistant rifaximin (7 day pretreatment) (Group 7) * | 3+100 | 12 | (1/12) 8.3 |
| Indomethacin + gastroresistant rifaximin (Group 4) * | 3+100 | 12 | (1/12) 8.3 |
| Indomethacin + omeprazole + gastroresistant rifaximin (Group 5) * | 3+0.7+100 | 12 | (0/12) 0 |
| Omeprazole + gastroresistant rifaximin (Group 6) * | 0.7+100 | 12 | (0/12) 0 |
| Diclofenac (Group 8) ** | 8 | 15 | (7/15) 46.7 |
| Diclofenac + omeprazole (Group 9) ** | 8+0.7 | 15 | (1/15) 6.7 |
| Diclofenac + gastroresistant rifaximin (Group 10) ** | 8+100 | 12 | (2/12) 13.3 |
| Diclofenac + omeprazole + gastroresistant rifaximin (Group 11) ** | 8+0.7+100 | 12 | (0/12) 0 |

| | | | |
|---|---|---|---|
| *referential **invention | | | |

Animals treated with indomethacin (1.5 mg/kg BID) or diclofenac (4 mg/kg BID) for 14 days displayed a significant decrease in blood haemoglobin concentration (Table 25).

The concomitant administration of omeprazole did not affect the reduced hemoglobin concentration as displayed in FIG 6.

In animals treated with indomethacin and gastroresistant rifaximin, with 1 week pretreatment with gastroresistant rifaximin (Group 7) or indomethacin and gastroresistant rifaximin (Group 4) or indomethacin plus omeprazole and gastroresistant rifaximin (Group 5), hemoglobin levels were significantly increased as compared with indomethacin plus omeprazole (Group 3), while in rats treated with indomethacin and gastroresistant rifaximin, hemoglobin levels were also significantly different from those observed in group treated with indomethacin alone as showed in FIG 6A and FIG 6B. Similarly, in animals treated with diclofenac and gastroresistant rifaximin (Group 10) or diclofenac plus omeprazole and gastroresistant rifaximin (Group 11), the concentration of hemoglobin was higher as compared with diclofenac alone as displayed in FIG 6. In rats treated with omeprazole and gastroresistant rifaximin (Group 6), there was no significant change in blood hemoglobin levels (FIG 6A and FIG 6B).

MPO levels in jejunal specimens excised from control rats accounted for 9.74 ng/mg tissue as reported in Table 25. In animals treated with indomethacin (1.5 mg/kg BID) or diclofenac (4 mg/kg BID) for 14 days, MPO levels were significantly increased up to 24.84 and 20.37 ng/mg tissue respectively, as reported in TABLE 25. In the ileum, the MPO concentration in control animals was 7.48 ng/mg tissue.

The administration of indomethacin 1.5 mg/kg BID or diclofenac 4 mg/kg BID for 14 days was associated with a significant increase in MPO up to 21.42 and 19.35 ng/mg tissue respectively as reported in Table 25.

In the jejunum of rats treated with indomethacin plus omeprazole, MPO levels were lower as compared with indomethacin alone, although they were significantly higher in comparison with control values.

Animals treated with indomethacin and gastroresistant rifaximin, with 1 week pretreatment with gastroresistant rifaximin (Group 7) or indomethacin and gastroresistant rifaximin (Group 4) or indomethacin plus omeprazole and gastroresistant rifaximin (Group 5) or omeprazole plus gastroresistant rifaximin, the jejunal levels of MPO were similar to those observed in control rats as displayed in FIG 7A.

In the ileum of rats treated with indomethacin and gastroresistant rifaximin, with 1 week pretreatment with gastroresistant rifaximin (group 7) or indomethacin and gastroresistant rifaximin (Group 4) or indomethacin plus omeprazole and gastroresistant rifaximin (Group 5) or omeprazole plus gastroresistant rifaximin, there was a significant decrease in MPO concentration, in comparison with animals treated with indomethacin alone as displayed in FIG 7B. In rats treated with diclofenac, the co-administration of omeprazole did not elicit any significant change in jejunal MPO levels, while in animals treated with diclofenac and gastroresistant rifaximin, diclofenac plus omeprazole and gastroresistant rifaximin or omeprazole and gastroresistant rifaximin, MPO concentrations were similar to those detected in control animals as displayed in FIG 7C. Similar patterns of MPO levels were observed in the ileum as displayed in FIG 7D.

In the jejunum of control rats, the MDA concentration was 28.23 nmol/mg of tissue as reported in Table 26. Animals treated with indomethacin (1.5 mg/kg BID) or diclofenac (4 mg/kg BID) for 14 days displayed significant increments of jejunal MDA levels of 66.15 and 69.44 nmol/mg of tissue respectively as reported in Table 26.

In the ileum, the MDA levels in control animals were 20.63 nmol/mg of tissue as showed in Table 26. Treatment with indomethacin or diclofenac resulted in a significant increase in MDA levels up to 54.47 and 67.1 nmol/mg of tissue respectively, reported in Table 26.

In rats treated with indomethacin, the co-administration of omeprazole did not modify MDA levels, both in the jejunum and ileum (FIG 8A and FIG 8B). In animals treated with indomethacin and gastroresistant rifaximin, with 1 week pretreatment with gastroresistant rifaximin (Group 7) or indomethacin and gastroresistant rifaximin (Group 4) or indomethacin plus omeprazole and gastroresistant rifaximin (Group 5) or omeprazole plus gastroresistant rifaximin, a significant reduction of MDA levels was observed, both in jejunum and ileum, reaching values similar to those observed in intestinal tissues from control animals as displayed in FIG 8A and B.

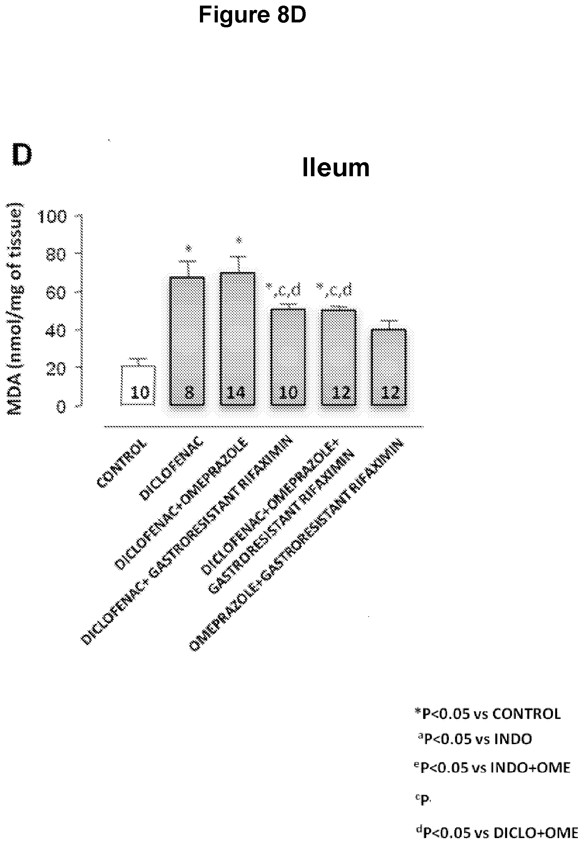

The MDA levels detected in the jejunum and ileum of rats treated with diclofenac plus omeprazole were similar to those observed in animals treated with diclofenac alone as displayed in FIG 8C and FIG 8D. In rats treated with diclofenac and gastroresistant rifaximin, diclofenac plus omeprazole and gastroresistant rifaximin, MDA concentrations in the jejunum and ileum were reduced in comparison with values obtained in group treated with diclofenac, although being significantly higher than those of control rats as displayed FIG 8C and D.

In the jejunum and ileum obtained from control animals, microscopic examination did not reveal any lesion of Type 1, 2 and 3 as reported in Table 27. Administration of 1.5 mg/kg BID of indomethacin or 4 mg/kg BID of diclofenac for 14 days was associated with the occurrence of various degrees of type 1, 2 and 3 lesions in both jejunum and ileum as reported in Table 27.

In the jejunum of rats treated with indomethacin, the co-administration of omeprazole was associated with a significant reduction of both Type 1, 2 and 3 lesions.

In animals treated with indomethacin and gastroresistant rifaximin, with 1 week pretreatment with gastroresistant rifaximin (Group 7) or indomethacin and gastroresistant rifaximin (Group 4) or indomethacin plus omeprazole and gastroresistant rifaximin (Group 5) or omeprazole plus gastroresistant rifaximin (Group 6), the jejunal lesions were significantly reduced or absent, as compared with the group treated with indomethacin alone as displayed in FIG 9.

In the ileum from rats treated with indomethacin plus omeprazole, the rates of type 1, 2 and 3 lesions were similar to those observed in animals treated with indomethacin alone.

In rats treated with indomethacin and gastroresistant rifaximin, with 1 week pretreatment with gastroresistant rifaximin (Group 7) or indomethacin and gastroresistant rifaximin (Group 4) or indomethacin plus omeprazole and gastroresistant rifaximin (Group 5) or omeprazole plus gastroresistant rifaximin (Group 6), the lesions in ileum were significantly reduced or absent, in comparison with group treated with indomethacin alone as displayed in FIG 10.

In rats treated with diclofenac plus omeprazole, jejunal type 2 lesions were significantly higher in comparison with those observed in animals treated with diclofenac alone, while no differences were detected for type 1 and 3 lesions.

In groups treated with diclofenac and gastroresistant rifaximin (Group 10) or diclofenac plus omeprazole and gastroresistant rifaximin (Group 11), all type of lesions were significantly reduced, in comparison with rats treated with diclofenac alone, while no lesions were observed in the jejunum of animals treated with omeprazole plus gastroresistant rifaximin as displayed in FIG 11.

The ileum excised from animals treated with diclofenac plus omeprazole (Group 9) displayed rates of type 1, 2 and 3 lesions similar to those observed in animals treated with diclofenac alone.

In groups treated with diclofenac and gastroresistant rifaximin (Group 10) or diclofenac plus omeprazole and gastroresistant rifaximin (Group 11), all type of lesions were significantly reduced, in comparison with those observed in rats treated with diclofenac alone, while no lesions were observed in the ileum from animals treated with omeprazole plus gastroresistant rifaximin as displayed in FIG 12.

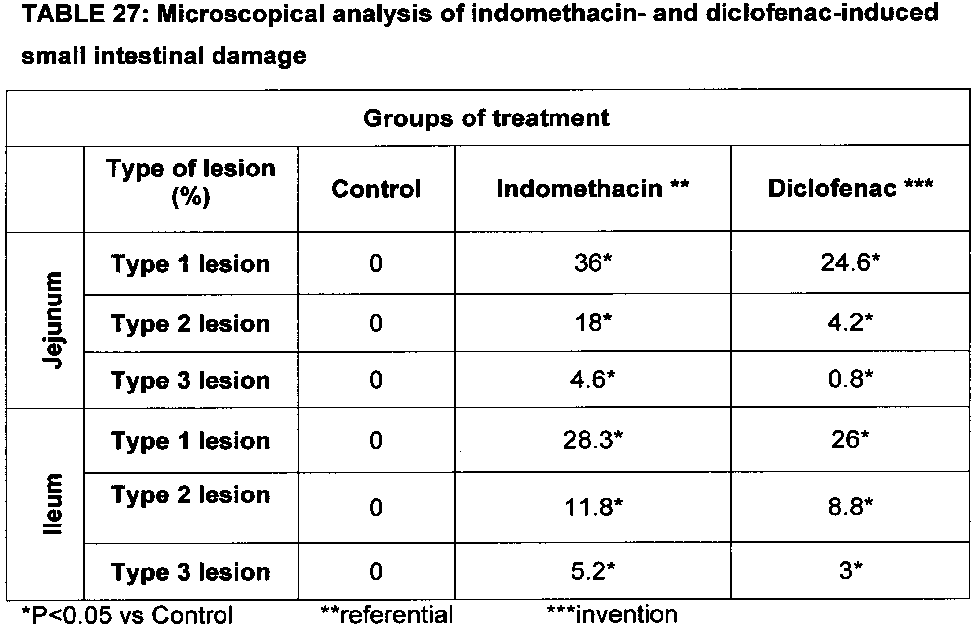

The administration of gastroresistant rifaximin at the dose of 50 mg/kg BID was associated with a significant improvement of tissue inflammatory and oxidative stress parameters, as well as histological injury scores cased by the NSAID alone or in combination with the PPI.

The dose of gastroresistant rifaximin used corresponds to a human equivalent dose of 16 mg/kg daily, that compares to a human dose used in Example 18 of 27 mg/kg.

The doses of diclofenac and omeprazole used in the rat experiment, to induce the damage, correspond to a human equivalent dose of 1.29 mg/kg and 0.11 mg/kg, respectively, which compare to a human dose of 2.5 mg/kg and 0.33 mg/kg.

The results showed that the administration of gastroresistant rifaximin (50 mg/kg, corresponding to a human equivalent dose of 16 mg/kg daily) is associated with a significant improvement of tissue inflammatory and oxidative stress parameters, as well as histological injury scores caused by indomethacin, if administered from 1 week before starting the NSAID administration (in this case in absence of PPI) until 1h before each daily dose of NSAID. In the clinical study rifaximin was co -administered with diclofenac or with diclofenac plus omeprazole.

Since the effective dose of rifaximin used in the Example 18, in terms of human equivalent dose is slightly lower (16 mg/kg) with respect to the dose used in the clinical trial (27 mg/kg), the results of the rat study in terms of histological results in ileum and jejunum and hemoglobin levels and MPO results can be used as measure of damage and inflammation and/or cardiovascular disease, to humans, claiming that if these tests are performed also in the clinical study, similar results are expected to be obtained.

Watanabe et al. (2013) recently reported that PPIs exacerbate small bowel injury in patients with rheumatoid arthritis taking NSAIDs on long term. Moreover, patients with severe damage had significantly lower hemoglobin levels than those with no damage, as also observed in the rat where the use of diclofenac or diclofenac plus PPI reduces the hemoglobin levels with respect to the control group. This is the first time that gastroresistant rifaximin is proved to be effective in the prevention of the mucosal damage induced by a NSAID administered for 14 days in combination with a PPI, that could even exacerbate the intestinal damage. The administration of omeprazole in combination with NSAIDs in Example 17 does not appear to affect the evaluated parameters of intestinal damage, with few exceptions.

### Example 18: Efficacy of rifaximin in preventing small bowel lesions caused by Diclofenac SR 75 mg tablets BID plus Omeprazole 20 mg capsules once a day (OD) in healthy volunteers

60 Healthy volunteers were enrolled within a clinical, randomised, single center study to evaluate the efficacy and safety of rifaximin gastroresistant microgranules 800 mg (2 x 400 mg tablets) twice a day (BID) in preventing small bowel lesions caused by Diclofenac SR 75 mg tablets BID plus Omeprazole 20 mg capsules once a day (OD) in healthy volunteers. Small bowel findings were assessed through video capsule endoscopy (VCE). The demography and other baseline date of the volunteers enrolled are reported in Table 28.

**TABLE 28: Demography and other baseline data - modified Full Analysis Set (mFA), Per Protocol (PP) Analysis Set and Safety Set**

| **Demographic data** | **Gastroresistant Rifaximin N=30** | **Placebo N=30** |
|---|---|---|
| **Sex** | | |
| Male - n (%) | 17 (56.7) | 18 (60.0) |
| Female - n (%) | 13 (43.3) | 12 (40.0) |

| **Race** | | |
|---|---|---|
| White - n (%) | 29 (96.7) | 30 (100.0) |
| Asian - n (%) | 1 (3.3) | 0 (0.0) |

| **Age (years)** | | |
|---|---|---|
| Mean ± SD | 27.7±7.8 | 26.9±4.4 |
| Median (range) | 26.0 (18-50) | 26.0 (19-39) |

| **Body weight (kg)** | | |
|---|---|---|
| Mean ± SD | 71.16±10.78 | 70.48±11.25 |
| Median (range) | 69.75 (51.5-102.4) | 69.85 (52.6-91.3) |

The main inclusion criteria for enrolling volunteers in the study included:
- male or non-pregnant, non-lactating female subjects from 18 years to 64 years old, inclusive;
- normal findings on the physical examination and laboratory evaluations;
- no more than 1 mucosal break at the baseline VCE;
- subjects' signed written informed consent.

The main exclusion criteria included:
- use of oral and systemic steroids, other NSAIDs (including low-dose aspirin and aminosalicylates), biological, sulphasalazine, misoprostol and other mucosal protective compounds, bisphosphonates, within 4 weeks prior to and during screening;
- use of probiotics, prebiotics, rifaximin and other antimicrobials, within 8 weeks prior to and during screening;
- use of prokinetic drugs within 2 weeks prior to and during screening;
- alcohol abuse;
- risk of pregnancy without adequate contraception;
- evidence of active duodenal and/or gastric ulcer, diverticulitis, infectious gastroenteritis, intestinal stricture or obstruction;
- a significant medical condition, which in the opinion of investigator precludes study participation.

Thirty (30) healthy subjects were randomised in each of the two treatment groups:
Group 1: one tablet of diclofenac SR 75 mg every 12 hours plus one capsule of omeprazole 20 mg once a day plus two rifaximin-matching placebo tablets every 12 hours, by oral route, for 14 days.
Group 2: one tablet of diclofenac SR 75 mg every 12 hours plus one capsule omeprazole 20 mg once a day plus two rifaximin gastroresistant microgranules 400 mg tablets every 12 hours, by oral route, for 14 days.

This study had a duration of approximately 5 weeks and consisted of:
Screening period: included a screening visit for potential subjects, to perform clinical investigations and laboratory tests, followed by a baseline VCE at visit 2.
Treatment period: eligible subjects were randomised into two treatment groups at randomisation visit. Each day during the 14 day-treatment period, each subject received diclofenac and omeprazole with rifaximin or rifaximin-matching placebo.
Final visit: performed within 36 hours after the last drug administration and including VCE, clinical assessments and laboratory test.

A telephonic follow-up performed one week after the final visit.

The primary efficacy endpoint criteria was the percentage of subjects developing at least one mucosal break at final visit evaluated by VCE and assessed according to a validated scoring system reported in Table 29.

**TABLE 29**

| **Category score** | **Category description** |
|---|---|
| 0 | Normal |
| 1 | Petechiae/red spot (demarcated, usually circular, area of crimson mucosa with preservation of villi) |
| 2 | Small number of erosions (n=1-4) |
| 3 | Higher number of erosions (>4) |
| 4 | Large erosions and/or ulcers |

The secondary efficacy endpoints were the change from baseline to final visit in the number of mucosal lesions in the small bowel and the change from baseline to final visit in the number of mucosal lesions in the small bowel with/without hemorrhage (visible blood).

The safety parameters considered were adverse events (AEs), clinical laboratory parameters such as hematology, clinical chemistry and urinalysis, and vital signs.

At the end of 2-week treatment with diclofenac plus omeprazole, the rate of subjects who developed at least one mucosal lesion in the small bowel was double in the placebo group, 13 subjects corresponding to 43.3% as compared to 6 subjects of the rifaximin group corresponding to a 20%. In particular, the proportion of subjects developing the lesions as compared to the proportion of subjects not developing the lesions was much lower in the rifaximin group 20% vs 80% than in the placebo group 43.3% vs 56.7%, strongly suggesting a protective action of rifaximin on mucosal damage caused by diclofenac during the 14 -day treatment as reported in Table 30.

**TABLE 30**

| | **Gastroresistant Rifaximin (N=30)** | **Placebo (N=30)** |
|---|---|---|
| Subjects Developing ≥ one mucosal lesion [n (%)] | 6 (20.0) | 13 (43.3) |
| Subjects NOT Developing mucosal lesions [n (%] | 24 (80.0) | 17 (56.7) |

A logistic regression analysis confirmed a trend towards rifaximin protection on diclofenac-induced mucosal lesions, with odd ratios (OR) of 0.3269 [95% Cl 0.1035, 1.0322] (Chi-square p-value=0.0566). In the subgroup analysis including sex as fixed effect and age as covariate, the difference between treatments was statistically significant (OR 0.3074 [95% Cl 0.0950, 0.9948]; p-value=0.0490).

The change from baseline in the total number of lesions was higher in the placebo group than in the rifaximin group (1.2±2.3 vs. 0.3±0.7). All the mucosal lesions detected at final assessment were lesions without haemorrhage. No lesions with haemorrhage were observed (Table 31). Results of the statistical analysis by negative binomial regression clearly indicated a protective effect of rifaximin on mean changes from baseline in total number of lesions (treatment effect OR -1.4137 [95% Cl -2.4935, -0.3339]) and lesions without haemorrhage (Treatment effect OR -1.4404 [95% Cl -2.5266, -0.3541]). The effect was statistically significant (p-value=0.0103 and 0.0094 for total lesions and lesions without haemorrhage, respectively).

**TABLE 31**

| **Mucosal lesions** - **Mean** ± **SD (Min-Max)** | | | | | |
|---|---|---|---|---|---|
| **Total lesions** | | **With haemorrhage** | | **Without haemorrhage** | |
| **Rifaximin N=30** | **Placebo N=30** | **Rifaximin N=30** | **Placebo N=30** | **Rifaximin N=30** | **Placebo N=30** |
| 0.3±0.7 (0-2) | 1.2±2.3 (0-10) | 0.0±0.0 (0-0) | 0.0±0.0 (0-0) | 0.3±0.7 (0-2) | 1.3±2.3 (0-10) |

Notably, at the end of the 2-week treatment at the Final visit, large erosions/ulcers (category 4) were only detected in the placebo group in 9 subjects corresponding to a 30%. No large erosions/lesions were observed in the rifaximin group. All the large erosions/ulcers observed in the placebo group were lesions without haemorrhage.

**TABLE 32**

| **Number of patients-n (%)** | | | | |
|---|---|---|---|---|
| **Category score** | **Gastroresistant Rifaximin N=30** | | **Placebo N=30** | |
| | **Baseline** | **After treatment** | **Baseline** | **After treatment** |
| 0 (Normal) | 11 (37%) | 10 (33%) | 13 (43%) | 6 (20%) |
| 1 (Petechiae/red spot) | 17 (57%) | 14 (47%) | 14 (47%) | 11(37%) |
| 2 (Small number of erosions; n=1-4) | 2 (7%) | 6 (20%) | 3 (10%) | 4 (13%) |
| 3 (Higher number of erosions; n > 4) | 0 (0%) | 0 (0%) | 0 (0%) | 0 (0%) |
| 4 (Large erosions and/or ulcers) | 0 (0%) | 0 (0%) | 0 (0%) | 9 (30%) |

In this study, a protective effect of rifaximin on diclofenac-induced mucosal lesions was clearly observed. Primary efficacy results showed that fewer subjects in the rifaximin group than in the placebo group developed at least one mucosal lesion in the small bowel during the study. Results of the secondary analysis on the changes from baseline, on the other hand, clearly showed a statistically significant difference in total number of lesions and lesions without haemorrhage, thus confirming a protective action of rifaximin on diclofenac-induced gastrointestinal damage.

### Example 19: Safety of rifaximin of Rifaximin gastroresistant microaranules co-administered with Diclofenac SR 75 mg tablets BID plus Omeprazole 20 mg capsules OD in healthy volunteers

The trend towards a protective effect of rifaximin on gastrointestinal damage was also reflected in the number of treatment-emergent adverse events (TEAEs) reported in the rifaximin and placebo groups during the study. In fact, a slightly lower number of TEAEs occurred in the rifaximin group as compared to the placebo group.

Overall, 97 TEAEs were reported: 44 TEAEs were experienced by 21 subjects corresponding to 67.7% in the rifaximin group, and 53 TEAEs by 22 subjects corresponding to 73.3% in the placebo group. Sixty-six of the reported TEAEs, 26 with rifaximin in 14 subjects and 40 with placebo in 18 subjects, were deemed by the investigator as treatment-related and reported in Table 33. In particular, gastrointestinal disorders, the most common related system organ class for TEAEs, were reported by 11 subjects corresponding to 35.5% with rifaximin and 16 subjects corresponding to 53.3% with placebo. The most frequent related TEAEs (>5%) were nausea, upper abdominal pain, diarrhoea and abdominal distension. Headache was reported by 3 of 31 subjects in the rifaximin group only. Abdominal pain, dyspepsia and increased Alanine aminotransferase were only reported in the placebo group, at a frequency > 5%. There were no deaths or SAEs during the study. No clinically significant changes in vital signs or laboratory parameters were observed.

**TABLE 33**

| **System Organ Class** | **Gastroresistant** Rifaximin - N=31 * | | **Placebo** - N=30 | |
|---|---|---|---|---|
| Preferred term | N AEs | N (%) Subjects | N AEs | N (%) Subjects |
| **All related TEAEs** - **all SOCs** | 26 | 14 (45.2) | 40 | 18 (60.0) |
| **Gastrointestinal disorders** | 19 | 11 (35.5) | 28 | 16 (53.3) |
| Nausea | 6 | 6 (19.4) | 8 | 6 (20.0) |
| Abdominal pain upper | 6 | 5 (16.1) | 2 | 2 (6.7) |
| Diarrhoea | 3 | 3 (9.7) | 4 | 3 (10.0) |
| Abdominal distension | 2 | 2 (6.5) | 2 | 2 (6.7) |
| Abdominal pain | 0 | 0 (0.0) | 6 | 4 (13.3) |
| Dyspepsia | 0 | 0 (0.0) | 2 | 2 (6.7) |
| Flatulence | 1 | 1 (3.2) | 1 | 1 (3.3) |
| Gastrointestinal sounds abnormal | 1 | 1 (3.2) | 1 | 1 (3.3) |
| Cheilitis | 0 | 0 (0.0) | 1 | 1 (3.3) |
| Oral disorder | 0 | 0 (0.0) | 1 | 1 (3.3) |
| **Nervous system disorders** | 4 | 3 (9.7) | 1 | 1 (3.3) |
| Headache | 4 | 3 (9.7) | 0 | 0 (0.0) |
| Dizziness | 0 | 0 (0.0) | 1 | 1 (3.3) |
| **General disorders and administration site conditions** | 2 | 2 (6.5) | 1 | 1 (3.3) |
| Fatigue | 2 | 2 (6.5) | 0 | 0 (0.0) |
| Oedema | 0 | 0 (0.0) | 1 | 1 (3.3) |
| **Investigations** | 0 | 0 (0.0) | 4 | 3 (10.0) |
| Alanine aminotransferase increased | 0 | 0 (0.0) | 2 | 2 (6.7) |
| Aspartate aminotransferase increased | 0 | 0 (0.0) | 1 | 1 (3.3) |
| C-reactive protein increased | 0 | 0 (0.0) | 1 | 1 (3.3) |
| **Skin and subcutaneous tissue disorders** | 1 | 1 (3.2) | 6 | 2 (6.7) |
| Eczema | 0 | 0 (0.0) | 3 | 1 (3.3) |
| Night sweats | 1 | 1 (3.2) | 0 | 0 (0.0) |
| Rash | 0 | 0 (0.0) | 3 | 1 (3.3) |

| | | | | |
|---|---|---|---|---|
| * One subject in the placebo group received two tablets of rifaximin (instead of placebo) by mistake, for a total rifaximin dose of 800 mg. This subject was included in the safety analysis of both placebo and rifaximin. | | | | |

The trend towards a protection of rifaximin on gastrointestinal damage was also reflected in the number of treatment-emergent adverse events reported during the study. In particular, gastrointestinal disorders occurred at a higher frequency in the placebo than in the rifaximin group. Overall, safety results confirm a favourable tolerability profile of the investigational product.

## Claims

1. Rifaximin in combination with at least one proton pump inhibitor and optionally a further antibiotic for use in a method for treating or preventing enteropathy in an individual undergoing NSAID administration, wherein the NSAID is diclofenac, ketoprofen, naproxen, ibuprofen, acetylsalicylic acid or mixtures thereof and wherein rifaximin is in gastroresistant form or in form of gastroresistant microgranules and is orally administered in an amount of from 20 mg to 3300 mg per day.

2. Rifaximin in combination with at least one proton pump inhibitor and optionally a further antibiotic as defined in claim 1 for the use according to claim 1, wherein the proton pump inhibitor is selected from omeprazole, ilaprazole, lansoprazole, tenatoprazole, rabeprazole, esomeprazole, pantoprazole, pariprazole, leminoprazole or nepaprazole or a free base, a free acid, a salt, a hydrate, an enantiomer, a tautomer, or a polymorph thereof.

3. Rifaximin in combination with at least one proton pump inhibitor and optionally a further antibiotic as defined in claim 1 for the use according to claim 1 or 2, wherein the NSAID is diclofenac.

4. Rifaximin in combination with at least one gastric acid inhibitor and optionally a further antibiotic as defined in claim 1 for the use of any one of according to claims 1 to 3, wherein the proton pump inhibitor is omeprazole.

5. Rifaximin in combination with at least one proton pump inhibitor and optionally a further antibiotic as defined in claim 1 for the use according to claims 1 to 4, wherein the rifaximin is administered in an amount from 400 mg to 800 mg one time a day, two times a day, three times a day, or four times a day or more often.

6. Rifaximin in combination with at least one proton pump inhibitor and optionally a further antibiotic as defined in claim 1 for the use according to claims 1 to 5, wherein the rifaximin in gastroresistant form or in the form of gastroresistant microgranules is a raw rifaximin, a polymorphic rifaximin or an amorphous rifaximin or a mixture thereof.

7. Rifaximin in combination with at least one proton pump inhibitor and optionally a further antibiotic or as defined in claim 1 for the use according to claims 1 to 6, wherein the rifaximin, the at least one proton pump inhibitor and optionally the further antibiotic are administered simultaneously, sequentially or in combination with the NSAID.

## Patentansprüche

1. Rifaximin in Kombination mit mindestens einem Protonenpumpenhemmer und gegebenenfalls einem weiteren Antibiotikum zur Verwendung in einem Verfahren zur Behandlung oder Verhütung einer Enteropathie in einem Individuum unter NSAID-Verabreichung, wobei das NSAID Diclofenac, Ketoprofen, Naproxen, Ibuprofen, Acetylsalicylsäure oder Gemische davon ist, und worin Rifaximin in einer gastroresisten Form oder in Form von gastroresisten Mikrokügelchen vorliegt und oral in einer Menge von 20 mg bis 3300 mg pro Tag verabreicht wird.

2. Rifaximin in Kombination mit mindestens einem Protonenpumpenhemmer und gegebenenfalls einem weiteren Antibiotikum wie in Anspruch 1 definiert zur Verwendung nach Anspruch 1, worin der Protonenpumpenhemmer ausgewählt ist aus Omeprazol, Ilaprazol, Lansoprazol, Tenatoprazol, Rabeprazol, Esomeprazol, Pantoprazol, Pariprazol, Leminoprazol oder Nepaprazol oder einer freien Base, einer freien Säure, einem Salz, einem Hydrat, einem Enantiomeren, einem Tautomeren oder einem Polymorphen davon.

3. Rifaximin in Kombination mit mindestens einem Protonenpumpenhemmer und gegebenenfalls einem weiteren Antibiotikum wie in Anspruch 1 definiert zur Verwendung nach Anspruch 1 oder 2, worin das NSAID Diclofenac ist.

4. Rifaximin in Kombination mit mindestens einem Magensäurehemmer und gegebenenfalls einem weiteren Antibiotikum wie in Anspruch 1 definiert zur Verwendung nach einem der Ansprüche 1 bis 3, worin der Protonenpumpenhemmer Omeprazol ist.

5. Rifaximin in Kombination mit mindestens einem Protonenpumpenhemmer und gegebenenfalls einem weiteren Antibiotikum wie in Anspruch 1 definiert zur Verwendung nach den Ansprüchen 1 bis 4, worin das Rifaximin in einer Menge von 400 mg bis 800 mg einmal täglich, zweimal täglich, dreimal täglich oder viermal täglich oder öfter verabreicht wird.

6. Rifaximin in Kombination mit mindestens einem Protonenpumpenhemmer und gegebenenfalls einem weiteren Antibiotikum wie in Anspruch 1 definiert zur Verwendung nach den Ansprüchen 1 bis 5, worin das Rifaximin in gastroresister Form oder in Form von gastroresisten Mikrokügelchen rohes Rifaximin, ein polymorphes Rifaximin oder ein amorphes Rifaximin oder ein Gemisch davon ist.

7. Rifaximin in Kombination mit mindestens einem Protonenpumpenhemmer und gegebenenfalls einem weiteren Antibiotikum wie in Anspruch 1 definiert zur Verwendung nach den Ansprüchen 1 bis 6, worin das Rifaximin, der mindestens eine Protonenpumpenhemmer und gegebenenfalls das weitere Antibiotikum gleichzeitig aufeinanderfolgend oder in Kombination mit dem NSAID verabreicht werden.

## Revendications

1. Rifaximine en combinaison avec au moins un inhibiteur de pompe à protons et facultativement un antibiotique supplémentaire à utiliser dans une méthode de traitement ou de prévention de l'entéropathie chez un individu subissant une administration d'AINS, dans laquelle l'AINS est le diclofénac, le kétoprofène, le naproxène, l'ibuprofène, l'acide acétylsalicylique ou des mélanges de ceux-ci et dans laquelle la rifaximine est sous forme gastro-résistante ou sous forme de microgranules gastro-résistants et est administrée par voie orale en une quantité de 20 mg à 3 300 mg par jour.

2. Rifaximine en combinaison avec au moins un inhibiteur de pompe à protons et facultativement un antibiotique supplémentaire telle que définie dans la revendication 1 pour l'utilisation selon la revendication 1, dans laquelle l'inhibiteur de pompe à protons est choisi parmi l'oméprazole, l'ilaprazole, le lansoprazole, le ténatoprazole, le rabéprazole, l'ésoméprazole, le pantoprazole, le pariprazole, le léminoprazole ou le népaprazole ou une base libre, un acide libre, un sel, un hydrate, un énantiomère, un tautomère, ou un polymorphe de ceux-ci.

3. Rifaximine en combinaison avec au moins un inhibiteur de pompe à protons et facultativement un antibiotique supplémentaire telle que définie dans la revendication 1 pour l'utilisation selon la revendication 1 ou 2, dans laquelle l'AINS est le diclofénac.

4. Rifaximine en combinaison avec au moins un inhibiteur d'acide gastrique et facultativement un antibiotique supplémentaire telle que définie dans la revendication 1 pour l'utilisation de l'une quelconque de selon les revendications 1 à 3, dans laquelle l'inhibiteur de la pompe à protons est l'oméprazole.

5. Rifaximine en combinaison avec au moins un inhibiteur de pompe à protons et facultativement un antibiotique supplémentaire telle que définie dans la revendication 1 pour l'utilisation selon les revendications 1 à 4, dans laquelle la rifaximine est administrée en une quantité de 400 mg à 800 mg une fois par jour, deux fois par jour, trois fois par jour, ou quatre fois par jour ou plus souvent.

6. Rifaximine en combinaison avec au moins un inhibiteur de pompe à protons et facultativement un antibiotique supplémentaire telle que définie dans la revendication 1 pour l'utilisation selon les revendications 1 à 5, dans laquelle la rifaximine sous forme gastro-résistante ou sous forme de microgranules gastro-résistants est une rifaximine brute, une rifaximine polymorphe ou une rifaximine amorphe ou un mélange de celles-ci.

7. Rifaximine en combinaison avec au moins un inhibiteur de pompe à protons et facultativement un antibiotique supplémentaire telle que définie dans la revendication 1 pour l'utilisation selon les revendications 1 à 6, dans laquelle la rifaximine, le au moins un inhibiteur de la pompe à protons et facultativement l'antibiotique supplémentaire sont administrés simultanément, séquentiellement ou en combinaison avec l'AINS.
